(19) Europäisches Patentamt
European Patent Office
Office européen des brevets

(11) **EP 3 884 838 A1**

(12) **EUROPEAN PATENT APPLICATION**
published in accordance with Art. 153(4) EPC

(43) Date of publication:
**29.09.2021 Bulletin 2021/39**

(21) Application number: **19887179.0**

(22) Date of filing: **21.11.2019**

(51) Int Cl.:
*A61B 1/00* *(2006.01)*          *A61B 1/045* *(2006.01)*
*A61B 1/267* *(2006.01)*        *A61B 1/273* *(2006.01)*
*G06T 7/00* *(2017.01)*

(86) International application number:
**PCT/JP2019/045580**

(87) International publication number:
**WO 2020/105699 (28.05.2020 Gazette 2020/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **21.11.2018   JP 2018218490
09.08.2019   JP 2019148079
20.09.2019   JP 2019172355
30.10.2019   JP 2019197174**

(71) Applicant: **AI Medical Service Inc.
Tokyo, 1700013 (JP)**

(72) Inventors:
• **SAITO Hiroaki**
**Tokyo 170-0013 (JP)**
• **SHICHIJO, Satoki**
**Tokyo 170-0013 (JP)**

• **ENDO Yuma**
**Tokyo 170-0013 (JP)**
• **AOYAMA Kazuharu**
**Tokyo 170-0013 (JP)**
• **TADA Tomohiro**
**Tokyo 170-0013 (JP)**
• **YAMADA Atsuo**
**Tokyo 170-0013 (JP)**
• **NAKAGAWA Kentaro**
**Tokyo 170-0013 (JP)**
• **ISHIHARA Ryu**
**Tokyo 170-0013 (JP)**
• **AOKI Tomonori**
**Tokyo 170-0013 (JP)**
• **TAMASHIRO Atsuko**
**Tokyo 170-0013 (JP)**

(74) Representative: **Ullrich & Naumann PartG mbB
Schneidmühlstrasse 21
69115 Heidelberg (DE)**

(54) **DISEASE DIAGNOSTIC ASSISTANCE METHOD BASED ON DIGESTIVE ORGAN ENDOSCOPIC IMAGES, DIAGNOSTIC ASSISTANCE SYSTEM, DIAGNOSTIC ASSISTANCE PROGRAM, AND COMPUTER-READABLE RECORDING MEDIUM HAVING DIAGNOSTIC ASSISTANCE PROGRAM STORED THEREON**

(57)    A diagnostic assistance method for a disease based on an endoscopic image of a digestive organ with use of a convolutional neural network (CNN) according to one embodiment of the present invention trains the CNN using a first endoscopic image of the digestive organ and at least one final diagnosis result of the positivity or the negativity for the disease in the digestive organ, or information corresponding to a severity level, the final diagnosis result being corresponding to the first endoscopic image, and the trained CNN outputs at least one of a probability of the positivity and/or the negativity for the disease in the digestive organ, a severity level of the disease, or a probability corresponding to the invasion depth (infiltration depth) of the disease, based on a second endoscopic image of the digestive organ. According to this embodiment, it is possible to acquire the probability of the positivity and/or the negativity for the disease in the digestive organ, the severity level of the disease, and the invasion depth of the disease, of the subject, at an accuracy substantially comparable to that of an endoscopy specialist within a short time period, and it becomes possible to select a subject requiring a separate confirmation diagnosis within a short time period.

EP 3 884 838 A1

# FIG. 15

| TRAINING | |
|---|---|

30,5841 IMAGES OF EROSION/ULCER
(NUMBER OF PATIENTS = 292)

ANNOTATION BY
ENDOSCOPY SPECIALISTS

CNN

| VALIDATION | |
|---|---|

7,507 IMAGES OF PROTRUDING LESION
IN SMALL BOWEL AND 10,000 NORMAL
IMAGES (NUMBER OF PATIENTS = 93)

AUTOMATIC DETECTION
OF PROTRUDING LESION BY
TRAINED CNN

CLASSIFICATION
(POLYPS) AND
PROBABILITY SCORE
(PS = 0.998)

**Description**

Field

The present invention relates to a diagnostic assistance method, a diagnostic assistance system, a diagnostic assistance program, and a computer-readable recording medium storing therein the diagnostic assistance program for a disease based on an endoscopic image of a digestive organ with use of a neural network.

Background

Many endoscopic examinations of digestive organs, such as the larynx, pharynx, esophagus, stomach, duodenum, biliary tract, pancreatic duct, small bowel, and large bowel, are being performed. Endoscopic examinations of upper digestive organs are often performed for screening of stomach cancers, esophageal cancers, peptic ulcer, and reflux gastritis, for example, and endoscopic examinations of the large bowel are often performed for screening of colorectal cancers, colon polyps, and ulcerative colitis, for example. In particular, endoscopic examinations of the upper digestive organs are effective as specific examinations for various symptoms of an upper abdomen, as detailed examinations in response to positive results from barium examinations of stomach diseases, and as detailed examinations in response to abnormal serum pepsinogen levels, which are generally incorporated in regular health checkups in Japan. Furthermore, stomach cancer screening has recently come to be shifted from conventional barium examinations to gastric endoscopic examinations.

Stomach cancers are one of the most common malignant tumors, and a few years ago it was estimated that there were approximately one million cases of stomach cancers worldwide. Of the root causes of the development of stomach cancers, infections with *Helicobacter pylori* (hereinafter, sometimes referred to as *"H. pylori"*) induces atrophic gastritis and intestinal metaplasia, and eventually leads to an onset of a stomach cancer. It is now considered that *H. pylori* contributes to 98% of the cases of noncardia stomach cancers in the world. Patients who have been infected with *H. pylori* have higher risks for stomach cancers. Considering that the incidence of stomach cancers has been reduced by eradicating *H. pylori,* the International Agency for Research on Cancer classifies *H. pylori* as a clear carcinogen. Based on this result, it is useful to eradicate *H. pylori* to reduce the risk of the onset of stomach cancers, and the eradication of *H. pylori* with the use of an antibacterial drug has come to be a treatment covered by the public health insurance system in Japan, and will be a highly recommended treatment in terms of health and hygiene in the future, too. In fact, the Ministry of Health, Labour and Welfare in Japan approved the coverage of eradicating treatment of gastritis caused by a *H. pylori* infection by the public health insurance in February 2013.

Gastric endoscopic examinations provide extremely useful information to differential diagnoses of *H. pylori* infections. Clearly visible capillaries (regular arrangement of collecting venules (RAC)) and fundic gland polyposis are characteristic of *H. pylori* negative gastric mucosa. Atrophy, redness, mucosal swelling, and enlarged gastric folds are typical observations found in gastritis caused by *H. pylori* infections. Red patches are characteristic of gastric mucosa after *H. pylori* eradication. Accurate endoscopic diagnoses of *H. pylori* infections are supported by various examinations such as measurement of anti-*H. pylori* IgG level in the blood or the urine, coproantibody measurement, urea breath tests, and rapid urease tests, and patients with the positive examination result can proceed to the *H. pylori* eradication. While endoscopic examinations are widely used in examining gastric lesions, if it is possible also to identify the presence of *H. pylori* infections during the checkups for gastric lesions without the need of clinical specimen analyses, the burden on patients can be reduced, because the patients are not required to go through standardized blood tests, urinalyses, and the like, and a contribution can also be made from the viewpoint of medical economics.

Esophageal cancers are the eighth most common cancer and have the sixth highest cause of death from cancer. In 2012, it was estimated that there were 456,000 new cases and 400,000 deaths. In Europe and North America, the incidence of esophageal adenocarcinoma has been increasing rapidly, and squamous cell carcinoma (SCC) is the most common type of the esophageal cancer, accounting for 80% of cases worldwide. The overall survival rate of the patients with an advanced esophageal SCC has also remained low. However, if this kind of tumor is detected as a mucosal cancer or submucosal cancer, a good prognosis can be expected.

Furthermore, total colonoscopy (CS) enables detections of colorectal diseases such as colorectal cancers (CRC), colorectal polyps, and inflammatory bowel diseases, at a high sensitivity and a high degree of specificity. Early diagnoses of such diseases enable patients to be treated at an earlier stage for a better prognosis, so that it is important to ensure sufficient CS quality.

Although the endoscopic examinations of the upper digestive organs and the large bowel have come to be widely practiced as described above, endoscopic examinations of the small bowel are not practiced very often because it is difficult to insert a typical endoscope into the small bowel. A typical endoscope has a length of about two meters or so. In order to insert the endoscope into the small bowel, it is necessary to orally insert the endoscope into the small bowel, via the stomach and the duodenum, or through the anus via the large bowel. Furthermore, because the small bowel itself is a long organ with a length of six to seven meters, it is difficult to insert a typical endoscope into the entire small bowel and to make observations. Therefore, for the endoscopic examinations of the small bowel, either double balloon endoscopy (see Patent Literature 1) or wireless capsule endoscopy (hereinafter, sometimes simply referred to as "WCE") (see Patent Literature 2) has been put to use.

Double balloon endoscopy is a method in which a balloon provided at the tip of the endoscope and another balloon provided at the tip of an over-tube covering the endoscope are inflated and deflated alternatingly or simultaneously, and an examination is carried out by reducing the length of, and straightening the long small bowel in a manner hauling the small bowel, but it is difficult to examine the entire length of the small bowel at once, because the length of the small bowel is long. Therefore, examinations of the small bowel by double balloon endoscopy are usually carried out at two separate steps, one through the mouth, and the other through the anus.

WCE endoscopy is carried out by having a patient swallow an orally ingestible capsule that includes a camera, a flash, a battery, a transmitter, and the like, and by causing the capsule to transmit captured images while the capsule is moving through the digestive tract, wirelessly to the external, and by receiving and recording the images externally, so that images of the entire small bowel can be captured all at once.

Moreover, pharyngeal cancers are detected often at an advanced stage prognosis, and are of a poor prognosis. Further, advanced cancer patients need surgical resection and chemoradiotherapy, which cause aesthetic problems and problems of loss of both swallowing and speaking functions and lead to considerable deterioration of quality of life. Previously, it was considered important in esophagogastroduodenoscopic (EGD) examinations that an endoscope quickly passes through the pharynx to reduce discomfort for patients, and observations of the pharynx were also not established. Unlike the case for the esophagus, it is impossible for endoscopy specialists to use iodine staining in the esophagus in view of the risk of airway aspiration. Consequently, superficial pharyngeal cancers were rarely detected.

In recent years, however, due to the development of image enhanced endoscopic examinations, such as narrow-band imaging (NBI), the improvement of consciousness of endoscopy specialists, and the like, pharyngeal cancers have been more and more detected during esophagogastroduodenoscopic examinations. Increased detections of superficial pharyngeal cancers (SPC) accordingly provide occasions to treat superficial pharyngeal cancers using endoscopic resection (ER), endoscopic submucosal dissection (ESD), or endoscopic mucosal resection (EMR), each of which has been established as local resection for superficial pharyngeal cancers. In addition, there have been reported short-term and long-term favorable treatment results of superficial pharyngeal cancers, due to endoscopic submucosal dissection which is a minimally invasive treatment ideal for maintaining functions and quality of life of superficial pharyngeal cancer patients.

Citation List

Patent Literature

Patent Literature 1: Japanese Patent Application Laid-open No. 2002-301019
Patent Literature 2: Japanese Patent Application Laid-open No. 2006-095304
Patent Literature 3: Japanese Patent Application Laid-open No. 2017-045341
Patent Literature 4: Japanese Patent Application Laid-open No. 2017-067489

Non Patent Literature

Non Patent Literature 1: Bibault JE, Giraud P, Burgun A. Big Data and machine learning in radiation oncology: State of the art and future prospects. Cancer Lett. 2016; 382(1):110-117.
Non Patent Literature 2: Esteva A, Kuprel B, Novoa RA, et al. Dermatologist-level classification of skin cancer with deep neural networks. Nature. 2017; 542(7639):115-118.
Non Patent Literature 3: Gulshan V, Peng L, Coram M, et al. Development and Validation of a Deep Learning Algorithm for Detection of Diabetic Retinopathy in Retinal Fundus Photographs. JAMA. 2016; 316(22):2402-2410.
Non Patent Literature 4: Byrne MF, Chapados N, Soudan F, et al. Real-time differentiation of adenomatous and hyperplastic diminutive colorectal polyps during analysis of unaltered videos of standard colonoscopy using a deep learning model. Gut. 2017.
Non Patent Literature 5: Chen PJ, Lin MC, Lai MJ, Lin JC, Lu HH, Tseng VS. Accurate Classification of Diminutive Colorectal Polyps Using Computer-Aided Analysis. Gastroenterology. 2018; 154(3):568-575.
Non Patent Literature 6: Misawa M, Kudo SE, Mori Y, et al. Artificial Intelligence-Assisted Polyp Detection for Colonoscopy: Initial Experience. Gastroenterology. 2018.

Non Patent Literature 7: Takiyama H, Ozawa T, Ishihara S, et al. Automatic anatomical classification of esophagogastroduodenoscopy images using deep convolutional neural networks. Sci Rep. 2018; 8(1):7497.

Non Patent Literature 8: Hirasawa T, Aoyama K, Tanimoto T, et al. Application of artificial intelligence using a convolutional neural network for detecting gastric cancer in endoscopic images. Gastric Cancer. 2018.

Non Patent Literature 9: Shichijo S, Nomura S, Aoyama K, et al. Application of Convolutional Neural Networks in the Diagnosis of Helicobacter pylori Infection Based on Endoscopic Images. EBioMedicine. 2017; 25:106-111.

Non Patent Literature 10: Iakovidis DK, Koulaouzidis A. Automatic lesion detection in capsule endoscopy based on color saliency: closer to an essential adjunct for reviewing software. Gastrointestinal endoscopy 2014 Nov; 80(5) : 877-83.

Non Patent Literature 11: Boal Carvalho P, Magalhaes J, Dias DE Castro F, et al. Suspected blood indicator in capsule endoscopy: a valuable tool for gastrointestinal bleeding diagnosis. Arq Gastroenterol. 2017;54(1):16-20.

Non Patent Literature 12: Muto M, Minashi K, Yano T, et al. Early detection of superficial squamous cell carcinoma in the head and neck region and esophagus by narrow band imaging: a multicenter randomized controlled trial. J Clin Oncol. 2010 Mar 20; 28(9): 1566-1572.

Non Patent Literature 13: Korman LY, Delvaux M, Hagenmuller F, Keuchel M, Friedman S, Weinstein M, Shetzline M, Cave D, de Franchis R: Capsule endoscopy structured terminology (CEST): proposal of a standardized and structured terminology for reporting capsule endoscopy procedures. Endoscopy 2005, 37(10):951-959.

## Summary

Technical Problem

In such endoscopic examinations of digestive organs, a large number of endoscopic images are collected, and endoscopy specialists are obliged to double-check the endoscopic images for the purpose of accuracy management. With some tens of thousands of endoscopic health checkups being carried out every year, the number of images to be interpreted by an endoscopy specialist for the secondary interpretation is enormous, e.g., approximately 2800 images per person per hour, and it has been a heavy burden in practice.

In WCE examinations of the small bowel, in particular, because the WCE is moved by intestinal peristalsis, and does not move based on its own movement, the movement of the WCE cannot be controlled from the external. Therefore, a large number of images are captured in a single examination so as not to miss anything. Furthermore, the number of images captured in a single examination is extremely large because the time for which the WCE moves in the small bowel is about eight hours. For example, because the WCE wirelessly transmits approximately 60,000 images per person, endoscopy specialists check the images by fast-forwarding, while it is possible that an abnormal observation appears only in one or two frames, so that a WCE image analysis with such a number of images requires an intense attention and concentration for a time period of 30 to 120 minutes on the average.

Moreover, diagnoses based on such endoscopic images not only require training of endoscopy specialists and an enormous amount of time for checking the stored images, but also are subjective and the possibility of various false positive and false negative determinations is unavoidable. Furthermore, fatigue of the endoscopy specialists may result in a deterioration in the accuracy of the diagnoses made by the endoscopy specialists. An enormous amount of burden on-site and a deterioration in the accuracy may lead to a restriction imposed on the number of medical examinees, and may result in a lack of sufficient medical services provided based on demands.

Furthermore, almost all the reports on detections and treatments of superficial pharyngeal cancers (SPC) are from Japan, and image enhanced endoscopic examinations, such as a narrow-band imaging (NBI) examination and a magnifying endoscope (IEE) examination, which are approaches adopted therefor are not necessarily used worldwide. The reason is that without sufficient education for endoscopy specialists, or without using narrow-band imaging or a magnifying endoscope, detecting superficial pharyngeal cancers is difficult. Thus, an efficient system for detecting superficial pharyngeal cancers based on esophagogastroduodenoscopic examination images has been strongly demanded.

In order to reduce the burden and to improve the deterioration in accuracy of endoscopy, there is a high expectation in the use of artificial intelligence (AI). There is an expectation that, if the recent AI with an image recognition capability higher than that of humans can be used in assisting endoscopy specialists, it will improve the accuracy and the rate of the secondary interpretations. Recently, the AI using the deep learning has been attracting attention in various medical fields, and it has been reported that the AI can screen medical images, in replacement of the specialists, not only in the fields such as radiation oncology, skin cancer classification, diabetic retinopathy (see Non Patent Literatures 1 to 3), and in the field of gastroenterological endoscopy, particularly in colonoscopy (see Non Patent Literatures 4 to 6), but also in various medical fields. Furthermore, there are some Patent Literatures in which various types of AI are used in making medical image diagnoses (see Patent Literatures 3 and 4). However, not enough validation has been done on whether the AI's capability of making endoscopic image diagnoses can satisfy an accuracy (correctness) and a performance (rate) requirements usable in the actual medical practice. For this reason, diagnoses based on the endoscopic images with the use of the AI have been not yet put into practice.

Deep learning enables a neural network with a plurality of layers stacked to learn high-order features of input data. Deep learning also enables a neural network to update internal parameters that are used in calculating a representation at each layer from the representation at the previous layer, using a back-propagation algorithm, by instructing how the apparatus should make changes.

In establishing associations between medical images, deep learning can train a neural network using medical images accumulated in the past, and has a possibility of being a strong machine-learning technology that allows the clinical features of a patient to be acquired directly from the medical images. A neural network is a mathematical model representing features of a neural circuit of a brain with computational simulations, and the algorithm supporting deep learning takes an approach using a neural network. A convolutional neural network (CNN) is developed by Szegedy and others, and is a network architecture that is most typically used for a purpose of deep learning of images.

In gastrointestinal endoscopy, a big challenge in making determinations using endoscopic images is how the efficiency can be improved while maintaining a high accuracy. In order to apply the AI to such image analyses in this field, improvements in the AI technology have been a big issue. The inventors of the present invention have built a CNN system capable of classifying images of the esophagus, the stomach, and the uodenum based on their anatomical sites, and also capable of reliably finding a stomach cancer in the endoscopic image (see Non Patent Literatures 7 and 8).

Furthermore, the inventors of the present invention have recently reported how a CNN plays a role in making diagnoses of *H. pylori* gastritis based on endoscopic images, indicating that the capability of the CNN came to compare with that of highly experienced endoscopy specialists, and the time required for making diagnoses was reduced significantly (see Non Patent Literature 9). However, because this CNN uses a training/validation data set excluding the cases after the *H. pylori* eradication and including only the cases of *H. pylori* positives and negatives, there is a problem in that it takes time and efforts to build a training/validation data set with the cases after the *H. pylori* eradication excluded, and another problem in that it is impossible to evaluate whether the CNN can correctly identify not only the cases of *H. pylori* positives and negatives, but also the cases after the *H. pylori* eradication.

Furthermore, when the CS is to be carried out, practitioners usually examine the rectum, the colon, and a part of the terminal ileum, because clinical characteristics of a disease differ depending on the anatomical sites of the colon and the rectum. For example, according to some recent researches, it has been pointed out that, with regard to colorectal cancers, there are some differences between the right colon and the left colon in epidemiology, prognosis, and a clinical result of chemotherapy. In the same manner, to treat ulcerative colitis, the anatomical site of the large bowel is important. The reason for that is that the applicability of oral drugs and suppositories for ulcerative colitis is also based on the position where the colitis is located. Therefore, in CS examinations, it is clinically meaningful to correctly identify the anatomical sites of colorectal diseases.

The CS is generally used in screening for cases of fecal occult blood positives or abdominal symptoms. However, sufficient special training is required for the practitioner to be able to handle a colonoscopy as he/she wishes, to recognize abnormal sites, and to make diagnoses of diseases correctly. One of the reasons why it takes such a long time to acquire the skill is in difficulty in making anatomical recognition in the endoscopy. Due to the anatomical differences between the sites of the colon, and similarity between various parts of the colon, not only the beginners of the CS but also the CS specialists cannot recognize the exact position of the tip of the endoscopic scope.

Therefore, in order for a practitioner to perform the CS and to detect abnormality, it is necessary to correctly recognize the anatomical parts of the colon via a CS image. According to some recent evidence, in order to acquire a sufficient skill, at least 200 cases of experiences of completing the entire CS tests are required. In fact, in Japan, certification of endoscopy technique is granted only after endoscope training of 5 years or longer.

Furthermore, the most common symptoms discovered by a WCE in the small bowel is a mucoclasis such as an erosion or an ulcer. Because an erosion and an ulcer are mainly caused by a nonsteroidal anti-inflammatory drug (NSAID), and sometimes caused by Crohn's disease or a malignant tumor in the small bowel, an early diagnosis and an early treatment are mandatory. According to various previous reports, because a part of the small bowel where the mucous membrane is destroyed by an erosion or an ulcer exhibits not much color difference with respect to the normal mucous membrane around such a part, the performance of automatic detection of such parts with the use of software has been lower than that of detections of vasodilatation (see Non Patent Literature 10). Furthermore, no research has been carried out on diagnosing various diseases in the small bowel, bleeding, or a protruding lesion by applying a CNN to the WCE images of the small bowel.

Furthermore, a superficial esophageal squamous cell carcinoma (hereinafter, sometimes referred to as an SCC) that is defined as a mucosal or submucosal cancer accounts for 38% of all esophageal cancers diagnosed in Japan. For the superficial esophageal SCC, it is possible to apply either esophagectomy or endoscopic resection (ER), but these approaches are very different from each other, from the viewpoint of invasiveness. In the selection of an appropriate treatment, the most important factor is an invasion depth (infiltration depth) of cancer, considering the risk of metastasis or the possibility of healing from the ER.

An endoscopic diagnosis of the invasion depth of a cancer requires sufficient expertise in evaluating various endoscopic opinions, such as the postoperative course, the protrusion, and the hardness of the esophageal cancer, and changes in the microvessels. In a diagnose of the invasion depth of a superficial esophageal SCC, a non-magnification endoscopic (non-ME) examination, a magnification endoscopic (ME) examination, and an endoscopic ultrasound (EUS) examination are currently used. Diagnoses using non-magnification endoscopy are subjective, and are based on the protrusion, the depression, and the hardness of a cancer, which can be affected by variability among the observers. A magnification endoscopic examination enables a clear observation of microvessel structures that are closely associated with the invasion depth of an esophageal cancer.

Diagnoses using the endoscopic ultrasound and the magnification endoscopy are more objective than those using the non-magnification endoscopy, but are more complex, and more affected by the expertise of the physicians. Therefore, reported exact accuracies in the invasion depth of cancers in relation to the endoscopic ultrasound and the magnification endoscopy are conflicting and not satisfactory. Therefore, there has been a demand for an innovative approach for facilitating more objective diagnoses of the invasion depths of esophageal cancers.

The present invention is made in consideration of the challenges in the conventional technologies described above. In other words, a first object of the present invention is to provide a diagnostic assistance method, a diagnostic assistance system, a diagnostic assistance program, and a computer-readable recording medium storing therein the diagnostic assistance program for a disease based on an endoscopic image of a digestive organ, being capable of correctly identifying, for example, not only the cases of *H. pylori* positives and negatives but also cases after *H. pylori* eradication, using an endoscopic image of the digestive organ with use of a CNN system.

Furthermore, a second object of the present invention is to provide a diagnostic assistance method, a diagnostic assistance system, a diagnostic assistance program, and a computer-readable recording medium storing therein the diagnostic assistance program for a disease based on an endoscopic image of a digestive organ, being capable of identifying, for example, the anatomical site of a colorectal disease, using an endoscopic image of a digestive organ with use of a CNN system.

Moreover, a third object of the present invention is to provide a diagnostic assistance method, a diagnostic assistance system, a diagnostic assistance program, and a computer-readable recording medium storing therein the diagnostic assistance program for a disease in the small bowel based on an endoscopic image of the small bowel by a WCE, being capable of correctly identifying an erosion/ulcer, the presence/absence of bleeding, or a protruding lesion in the small bowel with use of a CNN system.

Further, a fourth object of the present invention is to provide a diagnostic assistance method, a diagnostic assistance system, a diagnostic assistance program, and a computer-readable recording medium storing therein the diagnostic assistance program for a superficial esophageal SCC based on an endoscopic image of the esophagus, using non-magnification endoscopy and magnification endoscopy, being capable of detecting the invasion depth of and classifying the superficial esophageal SCC.

Furthermore, a fifth object of the present invention is to provide a diagnostic assistance method, a diagnostic assistance system, a diagnostic assistance program, and a computer-readable recording medium storing therein the diagnostic assistance program for a disease based on an endoscopic image of a digestive organ, based on an esophagogastroduodenoscopic (EGD) examination image, being capable of correctly identifying the presence/absence of a superficial pharyngeal cancer, with use of a CNN system.

Solution to Problem

A diagnostic assistance method for a disease based on an endoscopic image of a digestive organ with use of a convolutional neural network system (hereinafter, sometimes referred to as a "CNN system") according to a first aspect of the present invention is a diagnostic assistance method for a disease based on an endoscopic image of a digestive organ with use of a network system, the diagnostic assistance method is characterized by including:

training the CNN system using

a first endoscopic image of the digestive organ, and
at least one final diagnosis result of the positivity or the negativity for the disease in the digestive organ, a severity level, or an invasion depth of the disease, the final diagnosis result being corresponding to the first endoscopic image, in which

the trained CNN system outputs at least one of a probability of the positivity and/or the negativity for the disease in the digestive organ, a probability of the past disease, the severity level of the disease, the invasion depth of the disease, and a probability corresponding to the site where the image is captured, based on a second endoscopic image of the digestive organ.

With the diagnostic assistance method for a disease based on an endoscopic image of a digestive organ with use of a CNN system according to the present aspect, because the CNN system is trained based on the first endoscopic image including a plurality of the endoscopic images of the digestive organ that are acquired for each of a plurality of subjects in advance, and on the at least one final diagnosis result of the positivity or the negativity for the disease, the past disease, the severity level, or the invasion depth of the disease, the final diagnosis result having been acquired in advance for each of the subjects, it is possible to acquire one or more of the probability of the positivity and/or the negativity for the disease in the digestive organ, the probability of the past disease, the severity level of the disease, the invasion depth of the disease, and the probability corresponding to the site where the image is captured, of the subject, at an accuracy substantially comparable to that of an endoscopy specialist within a short time period. Therefore, it becomes possible to select a subject requiring a separate confirmation diagnosis within a short time period. Moreover, because it becomes possible to achieve an automatic diagnosis of at least one of the probability of the positivity and/or the negativity for the disease, the probability of the past disease, the severity level of the disease, the invasion depth of the disease, and the probability corresponding to the site where the image is captured, for test data including the plurality of the endoscopic images of the digestive organ of a large number of subjects, not only an endoscopy specialist is enabled to perform check and make corrections easily, but also it becomes possible to simplify the tasks of creating a collection of images that are associated with a disease.

Furthermore, a diagnostic assistance method for a disease based on an endoscopic image of a digestive organ with use of a CNN system according to a second aspect of the present invention is, in the diagnostic assistance method for a disease based on an endoscopic image of a digestive organ with use of a CNN according to the first aspect, characterized in that the second endoscopic image is associated with a site of a digestive organ where the image is captured.

An untrained CNN system sometimes has difficulty in identifying the site where an endoscopic image of a specific digestive organ is captured. With the diagnostic assistance method for a disease in a digestive organ with use of a CNN system according to the second aspect, because the CNN system is trained with the endoscopic images classified into the respective sites, it becomes possible to train the CNN system finely correspondingly to the sites, so that it becomes possible to improve the detection accuracy of the probability of the negativity or the positivity for the disease, the probability of the past disease, the severity level of the disease, the probability corresponding to the site where the image is captured, and the like, for the second endoscopic image.

Furthermore, a diagnostic assistance method for a disease based on an endoscopic image of a digestive organ with use of a CNN system according to a third aspect of the present invention is, in the diagnostic assistance method for a disease based on an endoscopic image a digestive organ with use of a CNN according to the second aspect, characterized in that the site of the digestive organ includes at least one of the pharynx, the esophagus, the stomach, the duodenum, the small bowel, and the large bowel.

With the diagnostic assistance method for a disease based on an endoscopic image of a digestive organ with use of a CNN system according to the third aspect, because the sites can be correctly classified into the pharynx, the esophagus, the stomach, the duodenum, and the large bowel, it is possible to improve the detection accuracy of the probability of the positivity and the negativity for the disease, the probability of the past disease, the severity level of the disease, the probability corresponding to the site where the image is captured, and the like, for each of the sites.

Furthermore, a diagnostic assistance method for a disease based on an endoscopic image of a digestive organ with use of a CNN system according to a fourth aspect of the present invention is, in the diagnostic assistance method for a disease based on an endoscopic image of a digestive organ with use of a CNN according to the third aspect, characterized in that the site of the digestive organ is sectioned into a plurality of sections in at least one of the pharynx, the esophagus, the stomach, the duodenum, the small bowel, and the large bowel.

Because every digestive organ has a complex shape, if only a small number of sites to be classified are available, it is sometimes difficult to recognize to which site of the digestive organ a specific endoscopic image corresponds. In the diagnostic assistance method for a disease based on an endoscopic image of a digestive organ with use of a CNN system according to the fourth aspect, because each of the plurality of digestive organs is sectioned into the plurality of sections, it becomes possible to obtain a highly accurate diagnosis result within a short time period.

Furthermore, a diagnostic assistance method for a disease based on an endoscopic image of a digestive organ with use of a CNN system according to a fifth aspect of the present invention is, in the diagnostic assistance method for a disease based on an endoscopic image of a digestive organ with use of a CNN system according to the third or the fourth aspect, characterized in that the site of the digestive organ is the stomach, the at least one final diagnosis result includes at least one of positive *H. pylori* infection, negative *H. pylori* infection, and *H. pylori* eradicated, and the CNN outputs at least one of a probability of the positive *H. pylori* infection, a probability of the negative *H. pylori* infection, and a probability of the *H. pylori* eradicated.

With the diagnostic assistance method for a disease based on an endoscopic image of a digestive organ with use of a CNN system according to the fifth aspect of the present invention, it is possible to output not only the probabilities of the positive *H. pylori* infection or negative *H. pylori* infection of the subject, but also a probability of the subject having gone through the *H. pylori* eradication, within an extremely short time period, at the accuracy equivalent to that of a specialist in the Japan Gastroenterological Endoscopy Society. Therefore, it becomes possible to select a subject requiring a separate confirmation diagnosis correctly within a short time period. Note that it is possible to make the confirmation diagnosis by subjecting the selected subject to a measurement of an anti-H. *pylori* IgG level in the blood or urine, coproantibody test, or a urea breath test.

Furthermore, a diagnostic assistance method for a disease based on an endoscopic image a digestive organ with use of a CNN system according to a sixth aspect of the present invention is, in the diagnostic assistance method for a disease based on an endoscopic image of a digestive organ with use of a CNN system according to the fourth aspect, characterized in that the site of the digestive organ is the large bowel; the section is at least one of the terminal ileum, the cecum, the ascending colon, the transverse colon, the descending colon, the sigmoid colon, the rectum, and the anus; and the CNN system outputs, as the section where the second endoscopic image is captured, a probability corresponding to at least one of the terminal ileum, the cecum, the ascending colon, the transverse colon, the descending colon, the sigmoid colon, the rectum, and the anus.

Furthermore, a diagnostic assistance method for a disease based on an endoscopic image of a digestive organ with use of a CNN system according to a seventh aspect of the present invention is, in the diagnostic assistance method for a disease based on an endoscopic image of a digestive organ with use of a CNN system according to the fourth aspect, characterized in that the site of the digestive organ is the large bowel; and the sections are the terminal ileum, the cecum, the ascending colon and transverse colon, the descending colon and sigmoid colon, the rectum, and the anus; and the CNN outputs, as the section where the second endoscopic image is captured, a probability corresponding to at least one of the terminal ileum, the cecum, the ascending colon and transverse colon, the descending colon and sigmoid colon, the rectum, and the anus.

Furthermore, a diagnostic assistance method for a disease based on an endoscopic image of a digestive organ with use of a CNN system according to an eighth aspect of the present invention is, in the diagnostic assistance method for a disease based on an endoscopic image of a digestive organ with use of a CNN system according to the fourth aspect, characterized in that the site of the digestive organ is the large bowel; the sections are the terminal ileum, the right colon including the cecum-ascending colon-transverse colon, and the left colon including the descending colon-sigmoid colon-rectum, and the anus; and the CNN system outputs, as the section where the second endoscopic image is captured, a probability corresponding to at least one of the terminal ileum, the right colon including the cecum-ascending colon-transverse colon, the left colon including the descending colon-sigmoid colon-rectum, and the anus.

With the diagnostic assistance method for a disease based on an endoscopic image of a digestive organ with use of a CNN system according to any one of the sixth to the eighth aspects of the present invention, because the sections of the large bowel can be classified correctly, it becomes easy to understand the section requiring a detailed examination. Note that the sections of the large bowel may be selected, as appropriate, considering the appearance tendency, appearance frequency, and the like of large bowel diseases, and considering sensitivities of the CNN system and degrees of specificity corresponding to the respective sections.

Furthermore, a diagnostic assistance method for a disease based on an endoscopic image of a digestive organ with use of a CNN system according to a ninth aspect of the present invention is, in the diagnostic assistance method for a disease based on an endoscopic image of a digestive organ with use of a CNN system according to the third aspect, characterized in that the site of the digestive organ is the small bowel; the endoscopic image is a wireless-capsule endoscopic (WCE) image; and the disease is at least one of erosion and ulcer, or a protruding lesion.

With the diagnostic assistance method for a disease based on an endoscopic image of a digestive organ with use of a CNN system according to the ninth aspect of the present invention, for WCE endoscopic images of the small bowel acquired from a large number of subjects, it becomes possible to acquire a region and a probability of the positivity and/or the negativity for at least one of erosion and ulcer, or a protruding lesion in the small bowel of the subjects, at an accuracy substantially comparable to that of an endoscopy specialist, within a short time period. Therefore, it becomes possible to select a subject requiring a separate confirmation diagnosis within a short time period, and an endoscopy specialist is enabled to perform check and make corrections easily. Note that with the diagnostic assistance method for a disease based on an endoscopic image of a digestive organ with use of a CNN system according to the present aspect, although the erosion and the ulcer are not clearly distinguishable in a WCE endoscopic image of the small bowel, it becomes possible to select at least one of the erosion and the ulcer automatically and correctly.

A diagnostic assistance method for a disease based on an endoscopic image of a digestive organ with use of a CNN system according to a tenth aspect of the present invention is, in the diagnostic assistance method for a disease based on an endoscopic image of a digestive organ with use of a CNN system according to the ninth aspect, characterized in that the final diagnosis result of the positivity or the negativity for the disease in the small bowel is displayed as a disease-positive region in the second endoscopic image; and the CNN system displays the detected disease-positive region in the second endoscopic image, and also displays the probability score in the second image.

With the diagnostic assistance method for a disease based on an endoscopic image of a digestive organ with use of a CNN system according to the tenth aspect of the present invention, the region from which an endoscopy specialist has acquired the final diagnosis result can be compared correctly with the disease-positive region detected by the trained CNN system in the second endoscopic image, which allows the sensitivity and the degree of specificity of the CNN to be further favorable.

Furthermore, a diagnostic assistance method for a disease based on an endoscopic image of a digestive organ with use of a CNN system according to an eleventh aspect of the present invention is, in the diagnostic assistance method for a disease based on an endoscopic image of a digestive organ with use of a CNN system according to the tenth aspect, characterized in that a determination as to whether a result diagnosed by the CNN system is correct is made based on an overlap between the disease-positive region displayed in the second endoscopic image, being displayed as the final diagnosis result of the positivity or the negativity for the disease in the small bowel, and the disease-positive region displayed by the trained CNN system in the second endoscopic image.

With the diagnostic assistance method for a disease based on an endoscopic image of a digestive organ with use of a CNN system according to the eleventh aspect of the present invention, because the region from which an endoscopy specialist has acquired the final diagnosis result and the disease-positive region detected by the trained CNN system are both displayed in the second endoscopic image, comparisons with the diagnosis result of the trained CNN can be performed immediately, based on the overlap of such regions.

Furthermore, a diagnostic assistance method for a disease based on an endoscopic image of a digestive organ with use of a CNN system according to a twelfth aspect of the present invention is, in the diagnostic assistance method for a disease based on an endoscopic image of a digestive organ with use of a CNN system according to the tenth aspect, characterized in that,

(1) when the overlap occupies 80% or more of the disease-positive region displayed in the second endoscopic image, as the final diagnosis result of the positivity or the negativity for the disease in the small bowel, or

(2) when a plurality of the disease-positive regions are displayed by the CNN system in the second endoscopic image, and any one of the regions overlaps with the disease-positive region displayed in the second endoscopic image, as the final diagnosis result of the positivity or the negativity for the disease,

the diagnosis made by the CNN system is determined to be correct.

With the diagnostic assistance method for a disease based on an endoscopic image of a digestive organ with use of a CNN according to the twelfth aspect of the present invention, the correctness of the diagnosis made by the CNN system can be determined easily, so that the accuracy of the diagnosis made by the trained CNN system is improved.

Furthermore, a diagnostic assistance method for a disease based on an endoscopic image of a digestive organ with use of a CNN system according to a thirteenth aspect of the present invention is, in the diagnostic assistance method for a disease based on an endoscopic image of a digestive organ with use of a CNN system according to any one of the ninth to the twelfth aspects, characterized in that the trained CNN system displays a probability score as well as the detected disease-positive region in the second image.

With the diagnostic assistance method for a disease based on an endoscopic image of a digestive organ with use of a CNN system according to the thirteenth aspect of the present invention, an endoscopy specialist is enabled to, for a large number of subjects, get grasp of the region of the positivity and/or the negativity for the disease in the small bowel for an endoscopic image of the small bowel by a WCE, and the probability score correctly, within a short time period, so that an endoscopy specialist is enabled to perform check and make a correction easily.

A diagnostic assistance method for a disease based on an endoscopic image of a digestive organ with use of a CNN system according to a fourteenth aspect of the present invention is, in the diagnostic assistance method for a disease based on an endoscopic image of a digestive organ with use of a CNN system according to the third aspect, characterized in that the site of the digestive organ is the small bowel, the endoscopic image is a wireless-capsule endoscopic image, and the disease is the presence/absence of bleeding.

With the diagnostic assistance method for a disease based on an endoscopic image of a digestive organ with use of a CNN system according to the fourteenth aspect of the present invention, an image containing a blood component of the small bowel and a normal mucosal image can be distinguished from each other correctly and at a high rate, so that an endoscopy specialist is enabled to perform check and make a correction easily.

Furthermore, a diagnostic assistance method for a disease based on an endoscopic image of a digestive organ with use of a CNN system according to a fifteenth aspect of the present invention is, in the diagnostic assistance method for a disease based on an endoscopic image of a digestive organ with use of a CNN system according to the third aspect, characterized in that the site of the digestive organ is the esophagus; the endoscopic image is a non-magnification endoscopic image or a magnification endoscopic image; and the disease is an invasion depth of a squamous cell carcinoma (SCC). Furthermore, a diagnostic assistance method for a disease based on an endoscopic image of a digestive organ with use of a CNN system according to a sixteenth aspect of the present invention is, in the diagnostic assistance method for a disease based on an endoscopic image of a digestive organ with use of a CNN system according to the fifteenth aspect, characterized in that the final diagnosis result of the positivity or the negativity for the disease in the small bowel determines that the invasion depth of the squamous cell carcinoma is one of a mucosal epithelium-lamina propria mucosa (EP-LPM), a muscularis mucosa (MM), a section near a surface of a submucosal layer (SM1), and a level deeper than an intermediary portion of the submucosal layer (SM2-).

With the diagnostic assistance method for a disease based on an endoscopic image of a digestive organ with use of a CNN system according to the fifteenth or the sixteenth aspect of the present invention, because it is possible to get grasp of the invasion depth of the superficial esophageal SCC in the esophagus correctly within a short time period, the determination of the applicability of endoscopic resection (ER) to the superficial esophageal SCC can be made correctly.

Furthermore, a diagnostic assistance method for a disease based on an endoscopic image of a digestive organ with use of a CNN system according to a seventeenth aspect of the present invention is characterized in that the site of the digestive organ is the pharynx, the endoscopic image is an esophagogastroduodenoscopic examination image, and the disease is a pharyngeal cancer.

With the diagnostic assistance method for a disease based on an endoscopic image of a digestive organ with use of a CNN system according to the seventeenth aspect of the present invention, it becomes possible to detect the presence of a pharyngeal cancer at a high sensitivity and a high accuracy during a normal esophagogastroduodenoscopic examination.

Furthermore, a diagnostic assistance method for a disease based on an endoscopic image of a digestive organ with use of a CNN system according to an eighteenth aspect of the present invention is, in the diagnostic assistance method for a disease based on an endoscopic image of a digestive organ with use of a CNN system according to the seventeenth aspect, characterized in that the endoscopic image is a white light endoscopic image.

With the diagnostic assistance method for a disease based on an endoscopic image of a digestive organ with use of a CNN system according to the eighteenth aspect of the present invention, the presence of a pharyngeal cancer can be detected based on an image obtained by a white light endoscope widely used worldwide, so that it becomes possible even for a less skilled physician to detect the presence of a pharyngeal cancer less erroneously and correctly.

Furthermore, a diagnostic assistance method for a disease based on an endoscopic image of a digestive organ with use of a CNN system according to a nineteenth aspect of the present invention is, in the diagnostic assistance method for a disease based on an endoscopic image of a digestive organ with use of a CNN system according to any one of the first to the eighteenth aspects, characterized in that the CNN is further combined with three dimensional information from an X-ray computer tomographic imaging apparatus, an ultrasound computer tomographic imaging apparatus, or a magnetic resonance imaging diagnosis apparatus.

Because an X-ray computer tomographic imaging apparatus, an ultrasound computer tomographic imaging apparatus, and a magnetic resonance imaging diagnosis apparatus are capable of representing the structure of each of the digestive organs three dimensionally, it becomes possible to get grasp of the site where the endoscopic image is captured, more correctly, by combining the three dimensional information with the output of the CNN system according to any one of the first to the eighteenth aspects.

Furthermore, a diagnostic assistance method for a disease based on an endoscopic image of a digestive organ with use of a CNN system according to an twentieth aspect of the present invention is, in the diagnostic assistance method for a disease based on an endoscopic image of a digestive organ with use of a CNN system according to any one of the first to the nineteenth aspects, characterized in that the second endoscopic image is at least one of an image captured by an endoscope, an image transmitted via a communication network, an image provided by a remote control system or a cloud system, an image recorded in a computer-readable recording medium, and a video.

With the diagnostic assistance method for a disease based on an endoscopic image of a digestive organ with use of a CNN system according to the twentieth aspect, because it is possible to output the probability of each of the positivity and the negativity for the disease in the digestive organ, or the severity of the disease within a short time period, for the input second endoscopic image, regardless of the way in which the second endoscopic image is inputted, for example, even an image transmitted from a remote location, or even a video can be used. Note that as the communication network, the Internet, an intranet, an extranet, a local area network (LAN), an integrated services digital network (ISDN), a value-added network (VAN), a cable television (CATV) communication network, a virtual private network, a telephone network, a mobile communication network, a satellite communication network, and the like, which are known, may be used. Furthermore, as the transmission medium of the communication network, a known wired transmission such as an IEEE1394 serial bus, a USB, a powerline transmission, a cable TV circuit, a telephone network, and an ADSL line, a known wireless transmission such as infrared rays, Bluetooth (registered trademark), and IEEE802.11, a known wireless transmission such as a mobile telephone network, a satellite circuit, and a terrestrial digital network, and the like, may also be used. In this manner, this method may be used in a configuration as what is called a cloud service or a remote assistance service.

Furthermore, as the computer-readable recording medium, it is also possible to use known tapes such as a magnetic tape and a cassette tape, known disks including magnetic disks such as a floppy (registered trademark) disk and a hard disk, and optical discs such as a compact disc read-only memory (CD-ROM)/a magneto-optical (MO) disc/a MiniDisc (MD: registered trademark)/a digital video disc/a compact disc recordable (CD-R), cards such as an integrated circuit (IC) card, a memory card, and an optical card, semiconductor memories such as a mask ROM/an erasable programmable read-only memory (EPROM)/an electrically erasable programmable read-only memory (EEPROM)/a flash ROM, or the like. Thereby, it is possible to provide a configuration for enabling the system to be easily transferred or installed in what is called a medical care organization or a health check organization.

Furthermore, a diagnostic assistance system for a disease based on an endoscopic image of a digestive organ according to a twenty first aspect of the present invention is a diagnostic assistance system for a disease based on an endoscopic image of an endoscopic image, the diagnostic assistance system including an endoscopic image input unit, an output unit, and a computer in which a CNN program is incorporated, characterized in that

the computer includes:

a first storage area that stores therein a first endoscopic image of the digestive organ;
a second storage area that stores therein at least one of a final diagnosis result of the positivity or the negativity for the disease in the digestive organ, a past disease, a severity level, or an invasion depth of the disease, the final diagnosis result being corresponding to the first endoscopic image; and

a third storage area that stores therein the CNN program, and
the CNN program

is trained based on the first endoscopic image stored in the first storage unit, and the final diagnosis result stored in the second storage area, and
outputs to the output unit, based on a second endoscopic image of the digestive organ, the second endoscopic image being inputted from the endoscopic image input unit, at least one of a probability of the positivity and/or the negativity for the disease in the digestive organ, a probability of the past disease, a severity level of the disease, an invasion depth of the disease, and a probability corresponding to the site where the image is captured, for the second endoscopic image.

Furthermore, a diagnostic assistance system for a disease based on an endoscopic image of a digestive organ according to a twenty second aspect of the present invention is, in the diagnostic assistance system for a disease based on an endoscopic image of a digestive organ according to the twenty first aspect of the present invention, characterized in that the first endoscopic images are associated with respective sites where the images are captured.

Furthermore, a diagnostic assistance system for a disease based on an endoscopic image of a digestive organ according to a twenty third aspect of the present invention is, in the diagnostic assistance system for a disease based on an endoscopic image of a digestive organ according to the twenty second aspect of the present invention, characterized in that the site of the digestive organ includes at least one of the pharynx, the esophagus, the stomach, the duodenum, the small bowel, and the large bowel.

Furthermore, a diagnostic assistance system for a diagnosis based on an endoscopic image of a digestive organ according to a twenty fourth aspect of the present invention is, in the diagnostic assistance system for a diagnosis based on an endoscopic image of a disease in a digestive organ according to the twenty third aspect of the present invention, characterized in that the site of the digestive organ is sectioned into a plurality of sections in at least one of the pharynx, the esophagus, the stomach, the duodenum, the small bowel, and the large bowel.

Furthermore, a diagnostic assistance system for a disease based on an endoscopic image of a digestive organ according to a twenty fifth aspect of the present invention is, in the diagnostic assistance system for a disease based on an endoscopic image of a digestive organ according to the twenty third or the twenty fourth aspect of the present invention, in which the site of the digestive organ is the stomach; and the CNN program outputs at least one of a probability of positive *H. pylori* infection, a probability of negative *H. pylori* infection, and a probability of *H. pylori* eradicated.

Furthermore, a diagnostic assistance system for a disease based on an endoscopic image of a digestive organ according to a twenty sixth aspect of the present invention is, in the diagnostic assistance system for a disease based on an endoscopic image of a digestive organ according to the twenty third or the twenty fourth aspect of the present invention, characterized in that the site of the digestive organ is the large bowel; the section is at least one of the terminal ileum, the cecum, the ascending colon, the transverse colon, the descending colon, the sigmoid colon, the rectum, and the anus; the CNN program outputs, as the section where the second endoscopic image is captured, a probability corresponding to at least one of the terminal ileum, the cecum, the ascending colon, the transverse colon, the descending colon, the sigmoid colon, the rectum, and the anus.

Furthermore, a diagnostic assistance system for a disease based on an endoscopic image of a digestive organ according to a twenty seventh aspect of the present invention is, in the diagnostic assistance system for a disease based on an endoscopic image of a digestive organ according to the twenty third or the twenty fourth aspect of the present invention, characterized in that the site of the digestive organ is the large bowel; the section is at least one of the terminal ileum, the cecum, the ascending colon, the transverse colon, the descending colon, the sigmoid colon, the rectum, and the anus; and the CNN program outputs, as the site where the second endoscopic image is captured, a probability corresponding to at least one of the terminal ileum, the cecum, the ascending colon and transverse colon, the descending colon and sigmoid colon, the rectum, and the anus.

Furthermore, a diagnostic assistance system for a disease based on an endoscopic image of a digestive organ according to a twenty eighth aspect of the present invention is, in the diagnostic assistance system for a disease based on an endoscopic image of a digestive organ according to the twenty third or the twenty fourth aspect of the present invention, characterized in that the site of the digestive organ is the large bowel; the section is the terminal ileum, the right colon including the cecum-ascending colon-transverse colon, the left colon including the descending colon-sigmoid colon-rectum, and the anus; and the trained CNN program outputs, as the site where the second endoscopic image is captured, a probability corresponding to at least one of the sections of the terminal ileum, the right colon including the cecum-ascending colon-transverse colon, the left colon including the descending colon-sigmoid colon-rectum, and the anus.

Furthermore, a diagnostic assistance system for a disease based on an endoscopic image of a digestive organ according to a twenty ninth aspect of the present invention is, in the diagnostic assistance system for a disease based on an endoscopic image of a digestive organ according to the twenty third aspect of the present invention, characterized in that the site of the digestive organ is the small bowel, the endoscopic image is a wireless capsule endoscopic image, and the trained CNN program outputs a probability score of at least one of erosion and ulcer, or a protruding lesion in the wireless capsule endoscopic image inputted from the endoscopic image input unit.

Furthermore, a diagnostic assistance system for a disease based on an endoscopic image of a digestive organ according to a thirtieth aspect of the present invention is, in the diagnostic assistance system for a disease based on an endoscopic image of a digestive organ according to the twenty ninth aspect, characterized in that the trained CNN program displays a probability score of at least one of the detected erosion and the detected ulcer, or the detected protruding lesion in the second endoscopic image.

Furthermore, a diagnostic assistance system for a disease based on an endoscopic image of a digestive organ according to a thirty first aspect of the present invention is, in the diagnostic assistance system for a disease based on an endoscopic image of a digestive organ according to the twenty ninth aspect, characterized in that a region of the protruding lesion is displayed in the second endoscopic image, based on a final diagnosis result of the positivity or the negativity for the disease in the small bowel, and the trained CNN program determines whether a result diagnosed by the trained CNN program is correct, based on an overlap between the disease-positive region displayed in the second endoscopic image and the disease-positive region displayed by the trained CNN program in the second endoscopic image.

Furthermore, a diagnostic assistance system for a disease based on an endoscopic image of a digestive organ according to a thirty second aspect of the present invention is, in the diagnostic assistance system for a disease based on an endoscopic image of a digestive organ according to the thirty first aspect, characterized in that

(1) when the overlap occupies 80% or more of the disease-positive region displayed in the second endoscopic image, as the final diagnosis result of the positivity or the negativity for the disease in the small bowel, or
(2) when a plurality of the disease-positive regions are displayed by the trained CNN program in the second endoscopic image, and any one of the regions overlaps with the disease-positive region displayed in the second endoscopic image, as the final diagnosis result of the positivity or the negativity for the disease,

the diagnosis made by the trained CNN program is determined to be correct.

Furthermore, a diagnostic assistance system for a disease based on an endoscopic image of a digestive organ according to a thirty third aspect of the present invention is, in the diagnostic assistance system for a disease based on an endoscopic image of a digestive organ according to any one of the twenty ninth to thirty second aspects, characterized in that the trained convolutional neural network program displays in the second endoscopic image the detected disease-positive region and the probability score.

Furthermore, a diagnostic assistance system for a disease based on an endoscopic image of a digestive organ according to a thirty fourth aspect of the present invention is, in the diagnostic assistance system for a disease based on an endoscopic image of a digestive organ according to the twenty third aspect, characterized in that the site of the digestive organ is the small bowel, the endoscopic image is a wireless capsule endoscopic image, and the trained CNN program displays in the second endoscopic image a probability of the presence/absence of bleeding as the disease.

Furthermore, a diagnostic assistance system for a disease based on an endoscopic image of a digestive organ according to a thirty fifth aspect of the present invention is, in the diagnostic assistance system for a disease based on an endoscopic image of a digestive organ according to the twenty third aspect, characterized in that the site of the digestive organ is the esophagus, the endoscopic image is a non-magnification endoscopic image or a magnification endoscopic image, and the trained CNN program displays in the second image an invasion depth of a squamous cell carcinoma as the disease.

Furthermore, a diagnostic assistance system for a disease based on an endoscopic image of a digestive organ according to a thirty sixth aspect of the present invention is, in the diagnostic assistance system for a disease based on an endoscopic image of a digestive organ according to the thirty fifth aspect, characterized in that the trained CNN program displays in the second image that the invasion depth of the squamous cell carcinoma is one of a mucosal epithelium-lamina propria mucosa, a muscularis mucosa, a section near a surface of a submucosal layer, and a level deeper than an intermediary portion of the submucosal layer.

Furthermore, a diagnostic assistance system for a disease based on an endoscopic image of a digestive organ according to a thirty seventh aspect of the present invention is, in the diagnostic assistance system for a disease based on an endoscopic image of a digestive organ according to the twenty third aspect, characterized in that the site of the digestive organ is the pharynx, the endoscopic image is an esophagogastroduodenoscopic image, and the disease is a pharyngeal cancer.

Furthermore, a diagnostic assistance system for a disease based on an endoscopic image of a digestive organ according to a thirty eighth aspect of the present invention is, in the diagnostic assistance system for a disease based on an endoscopic image of a digestive organ according to the thirty seventh aspect, characterized in that the endoscopic image is a white light endoscopic image.

Furthermore, a diagnostic assistance system for a disease based on an endoscopic image of a digestive organ according to a thirty ninth aspect of the present invention is, in the diagnostic assistance system for a disease based on an endoscopic image of a digestive organ according to any one of the twenty first to the thirty eighth aspects of the present invention, characterized in that the CNN program is further combined with three dimensional information from an X-ray computer tomographic imaging apparatus, an ultrasound computer tomographic imaging apparatus, or a magnetic resonance imaging diagnosis apparatus.

Furthermore, a diagnostic assistance system for a disease based on an endoscopic image of a digestive organ according to a fortieth aspect of the present invention is, in the diagnostic assistance system for a disease based on an endoscopic image of a digestive organ according to any one of the twenty first to the thirty ninth aspects of the present invention, characterized in that the second endoscopic image is at least one of an image captured by an endoscope, an image transmitted via a communication network, an image provided by a remote control system or a cloud system, an image recorded in a computer-readable recording medium, and a video.

With the diagnostic assistance system for a disease based on an endoscopic image of a digestive organ according to any one of the twenty first to the fortieth aspects of the present invention, it is possible to achieve the same effects as those achieved by the diagnostic assistance method for a disease based on an endoscopic image of a digestive organ with use of a CNN according to any one of the first to the twentieth aspects.

Furthermore, a diagnostic assistance program based on an endoscopic image of a digestive organ according to a forty first aspect of the present invention is characterized by being configured to cause a computer to operate as units included in the diagnostic assistance system for a disease based on an endoscopic image of a digestive organ according to any one of the twenty first to the fortieth aspects.

With the diagnostic assistance program based on an endoscopic image of a digestive organ according to the forty first aspect of the present invention, it is possible to provide a diagnostic assistance program based on an endoscopic image of a digestive organ, the diagnostic assistance program being configured to cause a computer to operate as units included in the diagnostic assistance system based on an endoscopic image of a digestive organ according to any one of the twenty first to the fortieth aspects.

Furthermore, a computer-readable recording medium according to a forty second aspect of the present invention is characterized by storing therein the diagnostic assistance program based on an endoscopic image of a digestive organ according to the forty first aspect.

With the computer-readable recording medium based on an endoscopic image of a digestive organ according to the forty second aspect of the present invention, it is possible to provide a computer-readable recording medium storing therein the diagnostic assistance program based on an endoscopic image of a digestive organ according to the forty first aspect.

Advantageous Effects of Invention

As described above, according to the present invention, because a computer program in which a CNN is incorporated is trained based on a plurality of endoscopic images of a digestive organ, acquired for each of a plurality of subjects in advance, and a final diagnosis result of the positivity or the negativity for the disease, acquired for each of the subjects in advance, it is possible to acquire the probability of the positivity and/or the negativity for the disease in the digestive organ of the subject, the severity level of the disease, the invasion depth of the disease, and the like, within a short time period, at an accuracy substantially comparable to that of an endoscopy specialist. Therefore, it becomes possible to select a subject requiring a separate confirmation diagnosis within a short time period.

Brief Description of Drawings

FIG. 1A is an example of a gastroscopic image with positive *H. pylori* infection; FIG. 1B is an example of a gastroscopic image with negative *H. pylori* infection; and FIG. 1C is an example of a gastroscopic image after *H. pylori* eradication.

FIG. 2 is a schematic view illustrating main anatomical sites of the stomach.

FIG. 3 is a conceptual schematic view illustrating an operation of GoogLeNet.

FIG. 4 is a view illustrating a selection of patients for a validation data set for building a CNN according to a first embodiment.

FIG. 5 is a view illustrating main anatomical sites of the large bowel.

FIG. 6 is a schematic view of a flowchart for building a CNN system according to a second embodiment.

FIG. 7 is a view illustrating a typical colonoscopic image, and a probability score of each of sites recognized by a CNN according to the second embodiment.

FIGS. 8A to 8F are views illustrating receiver operating characteristic (ROC) curves of the terminal ileum, the cecum, the ascending colon, the descending colon, the sigmoid colon, and the rectum, and the anus, respectively.

FIG. 9A is a view illustrating an image correctly recognized as the anus and a probability score of each of sites; and FIG. 9B is a view illustrating an image of the terminal ileum, erroneously recognized as the anus, and a probability score of each of sites.

FIG. 10A is a view illustrating an image correctly recognized as the cecum, and a probability score of each of sites; and FIG. 10B is a view illustrating an image of the cecum, erroneously recognized as the terminal ileum, and a probability score of each of sites.

FIG. 11 is a schematic view illustrating a flowchart for building a CNN system according to a third embodiment.

FIG. 12 is a view illustrating one example of a ROC curve achieved by a CNN according to the third embodiment.

FIGS. 13A to 13D are views illustrating typical enteroscopic images, diagnosed correctly by the CNN according to the third embodiment, and probability scores of specific sites recognized by the CNN.

FIGS. 14A to 14E are examples of images diagnosed as a false positive by the CNN according to the third embodiment, based on the darkness, the laterality, bubbles, fragments, and the vasodilatation, respectively; and FIGS. 14F to 14H are examples of images of true erosion but diagnosed as a false positive.

FIG. 15 is a schematic view illustrating a flowchart for building a CNN system according to a fourth embodiment.

FIG. 16 is a view illustrating one example of a ROC curve achieved by a CNN according to the fourth embodiment.

FIGS. 17A to 17E are views illustrating representative regions correctly detected and classified by the CNN according to the fourth embodiment into five categories as polyps, nodules, epithelium tumors, submucosal tumors, and venous structures, respectively.

FIGS. 18A to 18C are each an example of an image of one patient in which a detection could not be correctly made by the CNN according to the fourth embodiment.

FIG. 19 is an example image diagnosed by endoscopy specialists to exhibit a true protruding lesion.

FIG. 20 is a schematic view illustrating a flowchart for building a CNN system according to a fifth embodiment.

FIG. 21 is a view illustrating one example of a ROC curve achieved by a CNN according to the fifth embodiment.

FIG. 22A shows images of representative blood correctly classified by the CNN system according to the fifth embodiment, and FIG. 22B shows images similarly showing normal mucosa images.

FIG. 23A shows images correctly classified as blood contents by a SBI, and FIG. 23B shows images incorrectly classified as normal mucosa by the red region estimation indication function (SBI).

FIG. 24 is a schematic cross-sectional diagram illustrating a relation between an invasion depth of an esophageal squamous cell carcinoma (SCC) and its classification to which a CNN according to a sixth embodiment is applied.

FIGS. 25A to 25D are each an example of a representative image correctly classified as a pharyngeal cancer by the CNN system according to a seventh embodiment.

FIGS. 26A to 26F are each an example of a representative image classified as false-positive by the CNN system according to the seventh embodiment.

FIG. 27 is a graph illustrating a sensitivity and a positive prediction value in a WLI and an NBI by the CNN system according to the seventh embodiment.

FIGS. 28A to 28D are each an example of an image classified as false-negative by the CNN system according to the seventh embodiment.

FIG. 29 is a block diagram of a diagnostic assistance method for a disease based on an endoscopic image of a digestive organ with use of a neural network according to an eighth embodiment.

FIG. 30 is a block diagram related to a diagnostic assistance system for a disease based on an endoscopic image of a digestive organ, a diagnostic assistance program based on an endoscopic image of a digestive organ, and a computer-readable recording medium, according to a ninth embodiment.

Description of Embodiments

A diagnostic assistance method, a diagnostic assistance system, a diagnostic assistance program, and a computer-readable recording medium storing therein the diagnostic assistance program for a disease based on an endoscopic image of a digestive organ, according to the present invention will now be explained in detail, using an example of gastritis induced by a *H. pylori* infection, and an example of recognizing large bowel sites. Note that the embodiments described below are merely examples for embodying the technical idea according to the present invention, and the scope of the present invention is not to be limited to these examples. In other words, the present invention is also equally applicable to other embodiments that fall within the scope defined in the appended claims. Furthermore, in the present invention, the term "image" includes video as well as still images.

[First Embodiment]

In a first embodiment, a diagnostic assistance method, a diagnostic assistance system, a diagnostic assistance program, and a computer-readable recording medium storing therein the diagnostic assistance program for a disease based on an endoscopic image of a digestive organ, according to the present invention will be explained, as an example of application to gastritis caused by a *H. pylori* infection. In the clinic to which one of the inventors of the present invention belongs, 33 endoscopy specialists in total carried out esophagogastroduodenoscopic (hereinafter, sometimes referred to as "EGD") examinations using an endoscope with an ordinary magnification with white light. Indications for the applications of EGD included various symptoms in the upper abdomen, a positive result in barium examinations for stomach diseases, abnormal serum pepsinogen levels, past diseases in the stomach or duodenum, or referrals concerning screening from primary care physicians.

The EGD was carried out by capturing images using a standard EGD endoscope (EVIS GIF-XP290N, GIF-XP260, GIF-XP260NS, GIF-N260; Olympus Medical Systems Corp., Tokyo) with white light. The acquired images were those captured at an ordinary magnification, and no enlarged image was used.

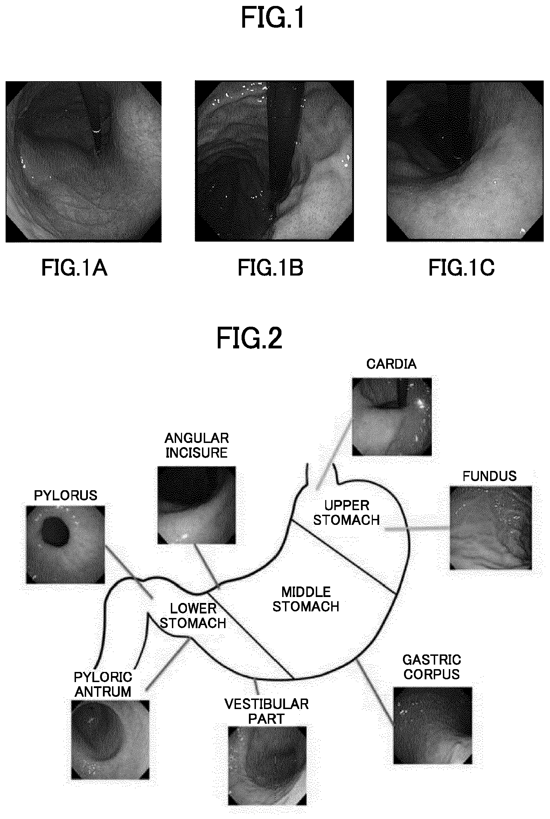

All of the patients received an examination to find out whether there was an *H. pylori* infection. The examination included at least one of a measurement of the anti-*H. pylori* IgG level in the blood or the urine, coproantibody measurement, and a urea breath test. Then, the patients who exhibited a positive reaction in any of these examinations were classified as *H. pylori* positive. The patients who were not diagnosed as *H. pylori* positive and who had no experience of receiving *H. pylori* eradication treatment were classified as *H. pylori* negative. Further, the patients who had received the *H. pylori* eradication treatment in the past and whose *H. pylori* was successfully eradicated were classified as *H. pylori* eradicated. FIG. 1 illustrates acquired typical gastroscopic images endoscopic images. FIG. 1A is an example of an image diagnosed as *H. pylori* positive. FIG. 1B is an example of an image diagnosed as *H. pylori* negative, and FIG. 1C is an example of an image after the *H. pylori* eradication.

[About Data Sets]

By retroactively reviewing images of 5,236 patients who received an EGD during the period between December 2015 and April 2017, data sets to be used for training and validating a CNN-based diagnostic system were prepared (these data sets will be hereinafter respectively referred to as a "training data set" and a "validation data set", and collectively referred to as a "training/validation data set". The training and validation are sometimes collectively referred to as "training/validation"). The data of patients who had a stomach cancer, an ulcer, or a submucosal tumor, and those who had a history of these diseases were excluded from the training/validation data set. The images of the stomach diagnosed as *H. pylori* positive, *H. pylori* negative, or *H. pylori* eradicated were further screened by endoscopy specialists to exclude unclear images due to food residue or bleeding in the stomach, or halation. Further, an endoscopic image data set to be evaluated (hereinafter, referred to as a "test data set") was also prepared. Note that this "training/validation data" corresponds to a "first endoscopic image" according to the present invention, and the "test data" corresponds to a "second endoscopic image" according to the present invention.

As indicated in Table 1, 98,564 images acquired from 742 patients who were determined as *H. pylori* positive, 3,469 patients who were determined as *H. pylori* negative, and 845 patients who were determined as *H. pylori* eradicated were prepared for the training data set. The number of images was then increased by rotating the 98,564 endoscopic images randomly, at an angle between 0 and 359°, by trimming and deleting the black frame portions around the images, and decreasing or increasing the scale of the resultant images within a range of 0.9 times to 1.1 times, as appropriate.

The number of images can be thus increased by at least one of rotation, increasing or decreasing the scale, changing the number of pixels, extracting bright or dark portions, and extracting the sites with a color tone change, and can be increased automatically using some tool. Note that it is also possible to exclude emphasized images such as narrow-band images, so that the training data set includes only ordinary white light images at an ordinary magnification. A CNN system was then built using images classified into seven sites of the stomach (cardia, fundus, gastric body, angular incisure, vestibular part, pyloric antrum, and pylorus; see FIG. 2).

[Preparation of Validation Data Set]

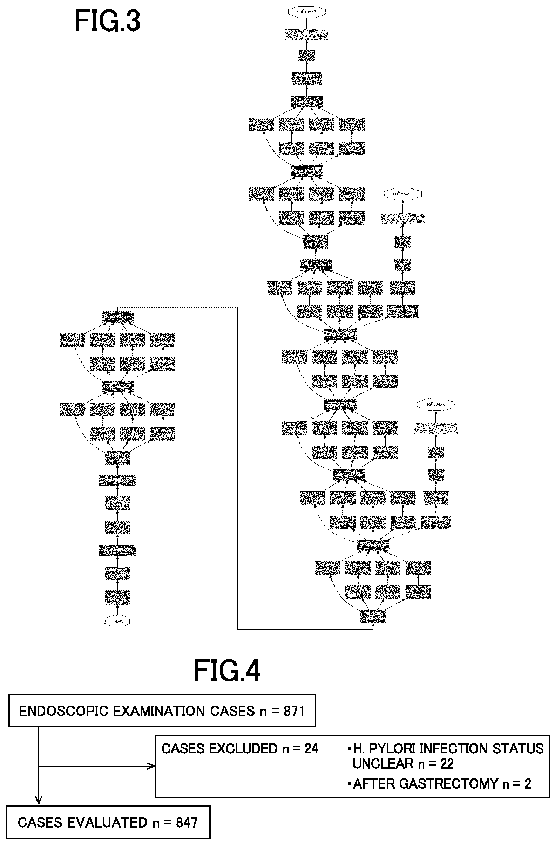

A validation data set was prepared in order to evaluate the accuracies of diagnoses made by the CNN system according to the first embodiment, having been built using the training data set described above, and diagnoses made by endoscopy specialists. From the image data of 871 patients who received endoscopic examinations in the clinic to which one of the inventors of the present invention belongs, within a period between May and June in 2017, the image data of 22 patients whose infection status of *H. pylori* was unclear and of 2 patients who had received gastrectomy were excluded. The final validation data set included 23,699 images collected from 847 patients in total (70 patients who were *H. pylori* positive, 493 patients who were *H. pylori* negative, and 284 patients who were *H. pylori* eradicated) (see FIG. 3).
The demographic features of these patients, and the features of the images are indicated in Table 1.

[Table 1]

| Characteristics | | Training data set | Validation data set |
|---|---|---|---|
| Number of images | | 98,564 | 23,699 |
| Number of endoscopy specialists | | 33 | 13 |
| Number of patients | | 5236 | 847 |
| Age of patients (SD (years old)) | | 52.7 (13.2) | 50.4 (11.2) |
| Sex of patients | Male (%) | 480 (45) | 168 (43) |
| | Female (%) | 598 (55) | 226 (57) |
| *H. pylori* clinical diagnosis Results(%) | Positive | 742 (14) | 70 (8) |
| | Negative | 3,649 (70) | 493 (58) |
| | Eradicated | 845 (16) | 284 (34) |
| SD: Standard Deviation 23,699 | | | |

The clinical diagnoses were made using the coproantibody test for 264 patients (31%), and using the anti-*H. pylori* IgG level in the urine for 126 patients (15%). In the cases of 63 patients (7%), a plurality of diagnosis tests were used. There was no redundancy between the training data set and the validation data set.

[Training/Validation Algorithm]

To build an CNN-based diagnostic system, a convolutional neural network (CNN) architecture with 22 layers was built with GoogLeNet (https://arxiv.org/abs/1409.4842), using Caffe framework first developed by the Berkeley Vision and Learning Center (BVLC), as the infrastructure for the development of a leading-edge deep learning neural network developed by Szegedy and others.
As illustrated in FIG. 4, the CNN system used in this first embodiment was trained with backpropagation. Each layer in the CNN was probabilistically optimized with AdaDelta (https://arxiv.org/abs/1212.5701), at a global learning rate of 0.005. In order to ensure all of the images the compatibility with GoogLeNet, each image was resized to $244 \times 244$ pixels. A trained model trained with the features of natural images in ImageNet was used as initial values at the time of the start of the training. ImageNet (http://www.image-net.org/) is a database having a collection of over 14 million images at the beginning of 2017. This training technique is referred to as transfer learning, and has been recognized to be effective even when the supervisor data is small in number. In the CNN system according to the first embodiment, INTEL's Core i7-7700K was used as the CPU, and NVIDEA's GeForce GTX 1070 was used as the graphics processing unit (GPU).

[Evaluation Algorithm]

The trained/validated CNN system according to the first embodiment outputs a probability score (PS) within a range between 0 and 1, as diagnosis results for *H. pylori* positive, *H. pylori* negative, and *H. pylori* eradicated, for images inputted. Denoting the probability score for *H. pylori* positive as Pp, denoting the probability score for the *H. pylori* negative as Pn, and denoting the probability score for the *H. pylori* eradicated as Pe, then Pp + Pn + Pe = 1. A value with the maximum value among these three probability scores was selected as the seemingly most reliable "diagnosis made by the CNN".

To ensure the anonymity of the patients, the entire patient information was deleted before the data analysis. This research was approved by the Japan Medical Association Ethical Review Board (ID JMA-IIA00283), and was implemented under the Declaration of Helsinki.

Relations between the diagnosis results measured by the CNN system according to the first embodiment, and the diagnosis results achieved by the clinical examinations are summarized in Table 2.

[Table 2]

| | | Symptoms confirmed in clinical examinations | | | |
|---|---|---|---|---|---|
| | | Negative | Positive | Eradicated | Total |
| CNN diagnoses | All images negative | 466 (71%) | 22 (3%) | 167 (25%) | 655 |
| | At least one image positive or eradicated | 27 (14%) | 48 (25%) | 117 (61%) | 192 |
| | At least one image eradicated | 16 (13%) | 20 (17%) | 83 (70%) | 119 |

Among 23, 699 images in total, the CNN system made a diagnosis of 418 images as *H. pylori* positive, 23, 034 images as *H. pylori* negative, and further 247 images as *H. pylori* eradicated. Among the 655 patients for which all of the images were diagnosed as *H. pylori* negative by the CNN system, 466 patients (71%) were diagnosed in the clinical examinations as *H. pylori* negative in the same manner. 22 patients (3%) were diagnosed as *H. pylori* positive, and 167 patients (25%) were diagnosed as *H. pylori* eradicated.

Furthermore, among 192 patients having at least one of their images diagnosed by the CNN system as *"H. pylori* positive or eradicated"*, in the clinical examinations, 48 patients (25%) were diagnosed as *H. pylori* positive, and 117 patients (61%) were diagnosed as *H. pylori* eradicated. Total of 165 patients (86%) were diagnosed as *"H. pylori* positive or eradicated"* in the same manner, while 27 patients (14%) were diagnosed as *H. pylori* negative. Further, among 119 patients having at least one of their images diagnosed by the CNN system as *H. pylori* eradicated, 83 patients (70%) were diagnosed in the clinical examinations as *H. pylori* eradicated, in the same manner, 16 patients (13%) were diagnosed as *H. pylori* negative, and 20 patients (17%) were diagnosed as *H. pylori* positive. Note that the time required for the CNN system to diagnose the 23,669 images was 261 seconds.

The following is clear from the results indicated in Table 2. In other words, when a CNN is used in making a diagnosis of a H. *pylori* infection status based on a gastroscopic image, it is clear that it is useful to extract the cases of *"H. pylori* positive or eradicated"* in a shorter time period by building a training/validation data set for building the CNN, by including not only the images diagnosed as *H. pylori* positive and as negative, but also those diagnosed as *H. pylori* eradicated in the clinical examinations. Furthermore, the screening system based on this CNN has a sufficient sensitivity and the degree of specificity to be deployed for clinical practice, and it is suggested that this system can greatly reduce the work load for endoscopy specialists in performing screening of images (test data) captured during endoscopic examinations.

With the CNN according to this first embodiment, it is possible to greatly reduce the time required for screening *H. pylori* infections without fatigue, and it becomes possible to acquire report results immediately after endoscopic examinations. In this manner, it is possible to reduce burdens on endoscopy specialists in diagnosing *H. pylori* infections and medical care expenditures, both of which are big issues to be addressed worldwide. Furthermore, with diagnoses of *H. pylori* using the CNN according to this first embodiment, because a result can be immediately obtained if an endoscopic image in an endoscopic examination is inputted, it is possible to provide completely "online" assistance to the diagnoses of *H. pylori,* and therefore, it becomes possible to solve the problem of heterogeneity of the distribution of medical doctors across the regions, by providing what is called "remote medical cares".

In Japan, there are many cases of *H. pylori* infection, particularly among the elderly. *H. pylori* eradication therapies for patients with gastritis caused by a *H. pylori* infection have come be covered by the Japanese health insurance since February 2013, and actually, the *H. pylori* eradication therapies have come to be widely adopted for patients with a *H. pylori* infection. Further, in the mass screening for stomach cancers using endoscopic images started in 2016, an enormous number of endoscopic images are processed, and there has been a demand for a more efficient image screening method. The results acquired in the first embodiment suggest possibilities that, by using this CNN with an enormous number of images in storage, screening of a *H. pylori* infection can be assisted greatly without evaluations of endoscopic examiners, further tests will lead to more confirmed cases of a *H. pylori* infection, and such cases will be eventually treated by *H. pylori* eradication. Furthermore, the CNN's capability to diagnose a *H. pylori* infection status is improved by adding classification of the stomach sites, and it is also possible to improve the capability to make a diagnosis of a stomach cancer by adding *H. pylori* infection status information.

Note that in the first embodiment an example using GoogLeNet as the CNN architecture is described, while CNN architectures has been developing on a daily basis, and sometimes better results may be obtained by adopting the latest architecture. Furthermore, while Caffe, which is also an open source, was used as a deep learning framework, CNTK, TensorFlow, Theano, Torch, MXNet, and the like may also be used. Furthermore, although Adam is used as an optimization technique, it is also possible to selectively use other known methods, such as Stochastic Gradient Descent (SGD), a MomentumSGV method in which momentum is added to SGD, an AdaGrad method, an AdaDelta method, a NesterovAG method, an RMSpropGraves method, and the like, as appropriate.

As described above, the accuracy of *H. pylori* infection diagnoses made by the CNN system according to the first embodiment with the use of the endoscopic images of the stomach was comparable to that achieved by endoscopy specialists. Therefore, the CNN system according to the first embodiment is useful in selecting patients with *H. pylori* infection based on acquired endoscopic images, for reasons such as screening. Furthermore, because the CNN system has been trained with images after the *H. pylori* eradication, it is possible to use the CNN system to determine whether the *H. pylori* has been successfully eradicated.

[Diagnostic Assistance System]

A CNN-incorporated computer as a diagnostic assistance system according to the first embodiment basically includes an endoscopic image input unit, a storage unit (a hard disk or a semiconductor memory), an image analyzing device, a determination display device, and a determination output device. The computer may also be directly provided with an endoscopic image capturing device. Further, this computer system may also be installed remotely away from an endoscopic examination facility, and operated as a centralized diagnostic assistance system by receiving image information from remote locations, or as a cloud computer system via the Internet.

The storage unit in this computer is provided with a first storage area storing therein a plurality of endoscopic images of a digestive organ acquired in advance from each of a plurality of subjects, a second storage area storing therein final diagnosis results representing the positivity or the negativity for the disease acquired for each of the subjects in advance, and a third storage area storing therein a CNN program. In such a case, because the number of the endoscopic images of the digestive organ acquired in advance from each of the subjects is large, and the data volume is large, and because an enormous number of data processing is performed when the CNN program is run, it is preferable to run the processes in parallel, and to have a large-capacity storage unit.

The recent improvement in CPU or GPU performance has been prominent, and by using a somewhat high-performance commercially available personal computer as the CNN program-incorporated computer serving as the diagnostic assistance system used in the first embodiment, it is possible to process 3000 cases or more per hour, as a diagnostic system for gastritis caused by a *H. pylori* infection, and to process a single image in approximately 0.2 seconds. Therefore, by providing image data captured by an endoscope to the CNN program-incorporated computer used in the first embodiment, it becomes also possible to make a determination of a *H. pylori* infection in real time, and to make remote diagnoses using not only gastroscopic images received from global or remote locations but also using videos. In particular, because GPUs of recent computers exhibit extremely high performance, by incorporating the CNN program according to the first embodiment, highly accurate image processing can be achieved at a high rate.

Furthermore, the endoscopic image of a digestive organ of a subject, inputted to the input unit of the CNN program-incorporated computer serving as the diagnostic assistance system according to the first embodiment, may be an image captured by an endoscope, an image transmitted via a communication network, or an image recorded in a computer-readable recording medium. In other words, because the CNN program-incorporated computer serving as the diagnostic assistance system according to the first embodiment can output a probability of each of the positivity and the negativity for the disease in the digestive organ within a short time period, for an inputted endoscopic image of a digestive organ of a subject, such images can be used regardless of the way in which the endoscopic image of the digestive organ of the subject is inputted.

Note that as the communication network, the Internet, an intranet, an extranet, a LAN, an ISDN, a VAN, a CATV communication network, a virtual private network, a telephone network, a mobile communication network, a satellite communication network, and the like, which are known, may be used. Furthermore, as the transmission medium of the communication network, a known wired transmission such as an IEEE1394 serial bus, an USB, a powerline transmission, a cable TV circuit, a telephone network, and an ADSL line, wireless transmission such as via infrared, Bluetooth (registered trademark), and IEEE802.11, or a wireless transmission such as a mobile telephone network, a satellite circuit, and a terrestrial digital network may also be used. Furthermore, as the computer-readable recording medium, it is also possible to use known tapes such as a magnetic tape or a cassette tape, disks including magnetic disks such as a floppy (registered trademark) disk or a hard disk, discs including optical discs such as a compact ROM/an MO/an MD/a digital video disc/a compact disc-R, cards such as an IC card, a memory card, and an optical card, semiconductor memories such as a mask ROM/an EPROM/an EEPROM/a flash ROM, or the like.

[Second Embodiment]

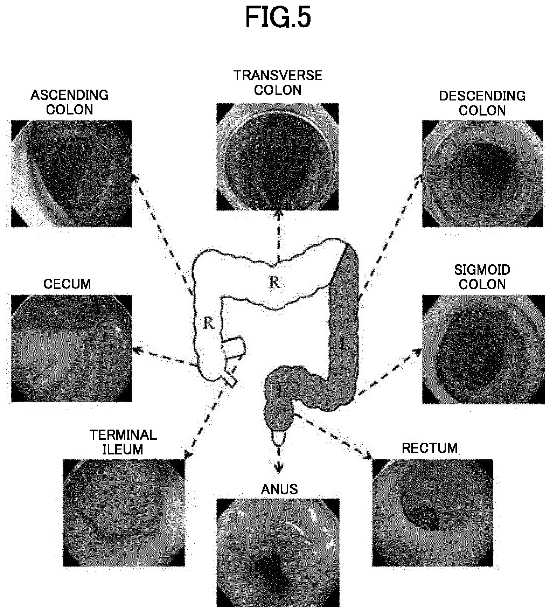

In a second embodiment, there will be explained an example in which the diagnostic assistance method and the diagnostic assistance system for a disease based on an endoscopic image of a digestive organ, the diagnostic assistance program, and the computer-readable recording medium storing therein the diagnostic assistance program according to the present invention are applied to classification of the large bowel sites. The sites of the large bowel include the terminal ileum, the cecum, the ascending colon, the transverse colon, the descending colon, the sigmoid colon, the rectum, and the anus. Note that this main anatomical classification of the large bowel is illustrated in FIG. 5. In the second embodiment, the CNN system was trained and validated so as to make the CNN system capable of automatically distinguishing images for each of these sites.

Clinical data of patients who received total colonoscopy (CS) within a period between January 2017 and November 2017 in the clinic to which one of the inventors of the present invention belongs, was reviewed retrospectively. The reasons for performing the CS included abdominal pains, diarrhea, positive fecal immunochemical tests, follow-ups of the past CS in the same clinic, mere screening, and the like. In order to correctly identify the anatomical sites of the colon and the rectum, only the images of the normal colon and the normal rectum filled with a sufficient amount of air, with the sites having been identified were used. A major portion of excluded images included those with a colorectal polyp, a cancer, and a biopsy scar, for example, and those with severe inflammation or bleeding were also excluded. Further, only white light images or emphasized images at an ordinary magnification were included.

Images captured in this CS method were captured using a standard colonoscope (EVIS LUCERA ELITE, CF TYPE H260AL/I, PCF TYPE Q260AI, Q260AZI, H290I, and H290ZI, Olympus Medical Systems Corp., Tokyo, Japan). Images of the ileum, the cecum, the ascending colon, the transverse colon, the descending colon, the sigmoid colon, the rectum, and the anus were captured during the course of CS, and 24 images were acquired for each part on the average, during the CS.

Note that in order to train/validate the CNN system, all pieces of the patient information associated with the images were anonymized, before the algorithm was developed. It was ensured that none of the endoscopy specialists involved with the CNN according to the second embodiment had any access to any identifiable patient information. Because this training/validation of the CNN system was a retrospective study using the anonymized data, an opt-out approach was used for the consent from the patients. This study was approved by the Japan Medical Association Ethical Review Board (ID: JMA-IIA00283).

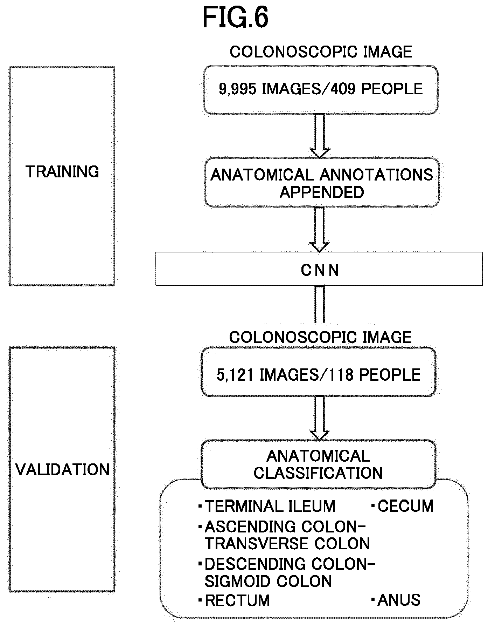

An overview of a flowchart for the CNN system according to the second embodiment is illustrated in FIG. 6. In this flowchart, the images were classified by endoscopy specialists in order to train/validate the CNN system in seven categories as the terminal ileum, the cecum, the ascending and transverse colons, the descending and sigmoid colons, the rectums, the anus, and unclassifiable. Classification of all of the training/validation images was checked by at least two endoscopy specialists, before the CNN system was trained/validated. The training/validation data set was classified into six categories as the terminal ileum, the cecum, the ascending and transverse colons, the descending and sigmoid colons, the rectum, and the anus. The training/validation data set did not include any unclassifiable images.

Conventionally, 5,000 or less images of the image data required to build an AI system for colorectal polyps were used in the training. Therefore, in order to ensure a sufficient amount of data, an aim was set to build the CNN system according to the second embodiment, using about 10,000 images. As training images, 9995 images of 409 patients collected within a period between January 2017 and March 2017 were prepared, and 5121 images of 118 patients acquired in November 2017 were used as a validation image set (see Table 3). The number of images of each of the anatomical sites in each of these image sets is indicated in Table 4.

[Table 3]

|  | Training data set | Validation data set |
|---|---|---|
| Age | 49.7 ± 10.8 | 51.0 ± 11.0 |
| Number of Males (%) | 180 (44.0) | 39 (33.0) |
| Number of Patients (Number of Images) | 409 (9,995) | 118 (5,121) |

[Table 4]

| Classification | Sub-classification | Number of images in training/validation data set (%) | Number of images in test data set (%) |
|---|---|---|---|
| Terminal ileum |  | 652 (6.5) | 209 (4.1) |
| Cecum | Right colon | 1,048 (10.5) | 423 (8.3) |
| Ascending-transverse colon |  | 2,376 (23.8) | 1,742 (34.0) |
| Descending-sigmoid colon | Left colon | 3,535 (35.4) | 2,081 (40.6) |
| Rectum |  | 1,037 (10.4) | 467 (9.1) |
| Anus |  | 970 (9.7) | 199 (3.9) |
| Unclear |  | 377 (3.8) | 0 (0) |
| Total |  | 9,995 (100) | 5,121 (100) |

All of the images in the training/validation data set acquired in the manner described above in the second embodiment were resized to 244×244 pixels to ensure the compatibility with GoogLeNet. Then, the CNN system used in the second embodiment was trained using the same approach as that used for the CNN system according to the first embodiment.

The CNN system according to the second embodiment outputs, for the training/validation images, a probability score (PS) for each of the sites in each of the images. The probability score takes a value within a range of 0 to 1 (0 to 100%), and represents a probability at which the image belongs to a corresponding site of the large bowel. The CNN system calculates, for each of the images, a probability score for each of the seven sites (the terminal ileum, the cecum, the ascending and transverse colons, the descending and sigmoid colons, the rectum, the anus, and the unclassifiable). An anatomical site with the highest probability score is assigned to the site of the image. Note that the sites of the large bowel may be classified into four sites of the terminal ileum, the right colon, the left colon, and the anus, by putting the cecum, the ascending colon, and the transverse colon into the right colon, and putting the descending colon, the sigmoid colon, and the rectum into the left colon, based on the similarity of their tissues.

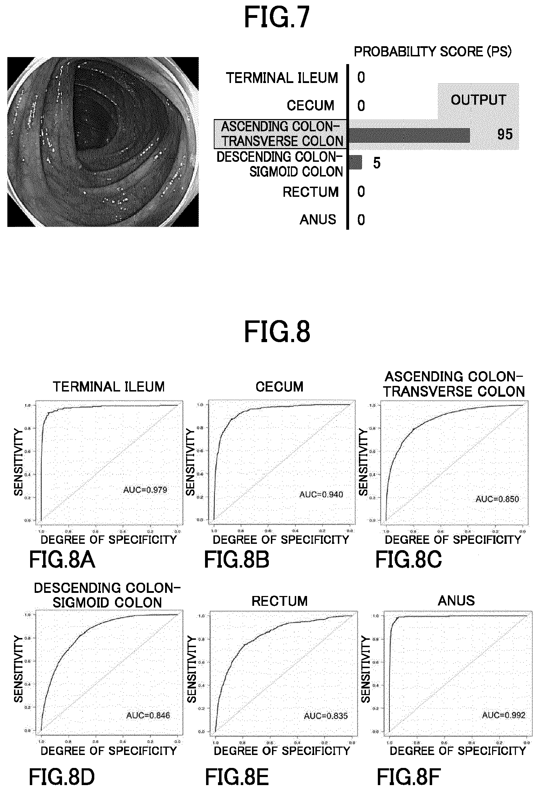

For example, the colonoscopic image on the left side of FIG. 7 is an example of an image of the ascending-transverse colon, in which the CNN system determined a probability score of 95% for the ascending-transverse colon, while also determining a probability score of 5% for the descending-sigmoid colon. As a result, the CNN system assigns the colonoscopic image on the left in FIG. 7, to the ascending-transverse colon.

The main objective of the CNN system according to the second embodiment is to acquire the sensitivity and the degree of specificity of the anatomical classification of colonoscopic images, by the CNN system. A receiver operating characteristic (ROC) curve is plotted for each of the sites, and the area under the curve (AUC), under the ROC curve, was calculated using GraphPad Prism 7 (GraphPad software Inc., California, U.S.A.). An ROC curve for each of the sites of the large bowel, created by the CNN system according to the second embodiment, is illustrated in FIG. 8. Note that FIGS. 8A to 8F are views illustrating receiver operating characteristic (ROC) curves of the terminal ileum, the cecum, the ascending colon, the descending colon, the sigmoid colon, and the rectum, and the anus, respectively.

The CNN system built in the second embodiment correctly recognized 66.6% of the images (3,410/5,121 images) of the validation data set. Table 5 indicates correct recognition ratios based on probability scores assigned to the images by the CNN system.

[Table 5]

| Probability score | Number of correct determinations | Total number of images | Correctness |
|---|---|---|---|
| > 99% | 465 (14) | 507 (10) | 91.7% |
| > 90% | 1,039 (30) | 1,296 (25) | 80.2% |
| > 70% | 1,009 (30) | 1,549 (30) | 65.1% |
| > 50% | 761 (22) | 1,397 (27) | 54.5% |
| ≤ 50% | 136 (4) | 372 (7) | 36.6% |
| Total | 3,410 (100) | 5,121 (100) | 66.6%* |
| *: mean value | | | |

The CNN system assigned the probability scores higher than 99% to 10% (507 images) of the entire images (5,121 images), in which 465 images (14% of those correctly classified) were those correctly classified by clinical diagnoses. Therefore, the accuracy was 91.7%.

In the same manner, the CNN system assigned the probability scores higher than 90% and equal to or less than 99% to 25% (1,296 images) of the entire images, in which 1,039 images (30% of those correctly classified) were those correctly classified by clinical diagnoses. Therefore, the accuracy was 80.2%. In the same manner, the CNN system assigned the probability scores higher than 70% and equal to or less than 90% to 30% (1,549) of the entire images, in which 1,009 images (30% of those correctly classified) were those correctly classified by clinical diagnoses. Therefore, the accuracy was 65.1%.

In the same manner, the CNN system assigned the probability scores higher than 50% and equal to or less than 70% to 27% (1,397 images) of the entire images, in which 761 images (22% of those correctly classified) were those correctly classified by clinical diagnoses. Therefore, the accuracy was 54.5%. Furthermore, the CNN system assigned the probability scores equal to or lower than 50% to 7% (372 images) of the entire images, in which 136 images (4% of those correctly classified) were those correctly classified by clinical diagnoses. Therefore, the accuracy was 36.6%.

Table 6 indicates the CNN system output distribution for each of the anatomical sites classified by clinical diagnoses. In this table, there is no image classified as "unclassifiable".

[Table 6]

| CNN output | Terminal ileum n = 209 (%) | Cecum (n = 423) (%) | Ascending-transverse colon (n = 1,742) (%) | Descending -sigmoid colon (n = 2,081) (%) | Rectum (n = 467) (%) | Anus (n = 199) (%) |
|---|---|---|---|---|---|---|
| Terminal ileum | 145 (69) | 13 (3) | 4 (0) | 11 (1) | 6 (1) | 0 (0) |
| Cecum | 9 (4) | 211 (50) | 64 (4) | 7 (0) | 4 (1) | 0 (0) |
| Ascending-transverse colon | 6 (3) | 89 (21) | 891 (51) | 108 (5) | 6 (1) | 1 (1) |
| Descending-sigmoid colon | 40 (19) | 97 (23) | 775 (44) | 1,872 (90) | 265 (57) | 13 (7) |
| Rectum | 1 (0) | 4 (1) | 1 (0) | 78 (4) | 109 (23) | 3 (2) |
| Anus | 8 (4) | 9 (2) | 7 (0) | 5 (0) | 77 (16) | 182 (91) |
| Unclear | 0 (0) | 0 (0) | 0 (0) | 0 (0) | 0 (0) | 0 (0) |
| Sensitivity | 69.4 | 49.8 | 51.1 | 90.0 | 23.3 | 91.4 |
| Degree of specificity | 99.3 | 98.2 | 93.8 | 69.9 | 98.1 | 97.8 |

For the CNN system built in the second embodiment, the sensitivity at which the images were recognized was the highest for the anus at 91.4%, the sensitivity for the descending colon and the sigmoid colon was the second highest at 90.0%, the sensitivity for the terminal ileum was 69.4%, the sensitivity for the ascending colon and the transverse colon was 51.1%, and further the sensitivity for the cecum was 49.8%, while the sensitivity for the rectum was the lowest at 23.3%. Further, the degree of specificity for each of the anatomical sites was 90% or higher except for that of the sites of the descending colon and the sigmoid colon (60.9%). Note that the CNN system built in the second embodiment recognized images having an AUC exceeding 0.8, for each of the anatomical sites.

Table 7 indicates an output distribution of the CNN system built in the second embodiment, for the terminal ileum, the right colon, the left colon, and the anus, by representing the cecum, the ascending colon, and the transverse colon as the "right colon", and representing the descending colon, the sigmoid colon, and the rectum as the "left colon". For the left colon, the CNN system exhibited a high sensitivity of 91.2% and a relatively low specificity of 63.%, while exhibiting reversed results for the terminal ileum, the right colon, and the anus.

[Table 7]

| CNN output | Terminal ileum n = 209 (%) | Right colon (n = 2,165) (%) | Left colon (n = 2,548) (%) | Anus (n = 199) (%) |
|---|---|---|---|---|
| Terminal ileum | 145 (69) | 17 (1) | 17 (1) | 0 (0) |
| Right colon | 15 (7) | 1,255 (58) | 125 (5) | 1 (1) |
| Left colon | 41 (20) | 877 (41) | 2,324 (91) | 16 (8) |
| Anus | 8 (4) | 16 (1) | 82 (3) | 182 (91) |
| Sensitivity | 69.4 | 58.0 | 91.2 | 91.5 |
| Degree of specificity | 99.3 | 95.2 | 63.7 | 97.8 |

Next, the sensitivity and the degree of specificity were calculated for each of the anatomical sites, for each of specific probability scores, that is, in accordance with four sections of 70% ≥ PS > 60%, 80% ≥ PS > 70%, 90% ≥ PS > 80%, and PS > 90%. Calculation results are indicated in Table 8.

[Table 8]

| Probability Score (PS) | | Terminal ileum | Cecum | Ascending-transverse colon | Descending-sigmoid colon | Rectum | Anus |
|---|---|---|---|---|---|---|---|
| PS > 60 | Sensitivity | 80.1 | 62.7 | 52.5 | 94.7 | 18.1 | 94.1 |
| | Degree of specificity | 99.6 | 98.9 | 97.0 | 61.6 | 89.9 | 98.0 |
| PS > 70 | Sensitivity | 81.8 | 67.6 | 53.6 | 96.2 | 15.1 | 95.1 |
| | Degree of specificity | 99.7 | 99.0 | 98.0 | 63.0 | 99.1 | 97.9 |
| PS > 80 | Sensitivity | 88.2 | 77.0 | 55.6 | 97.6 | 12.4 | 96.6 |
| | Degree of specificity | 99.8 | 99.2 | 99.0 | 66.8 | 99.5 | 97.9 |
| PS > 90 | Sensitivity | 92.2 | 82.7 | 56.5 | 99.1 | 8.2 | 97.0 |
| | Degree of specificity | 99.8 | 99.3 | 99.5 | 72.9 | 99.9 | 97.5 |

According to the results indicated in Table 8, for all of the probability scores, when the probability score was higher, the sensitivity and the degree of specificity were higher, for all of the sites excluding the rectum. However, in the rectum, although the degree of specificity was higher when the probability score was higher, the sensitivity did not match the tendency of the probability scores.

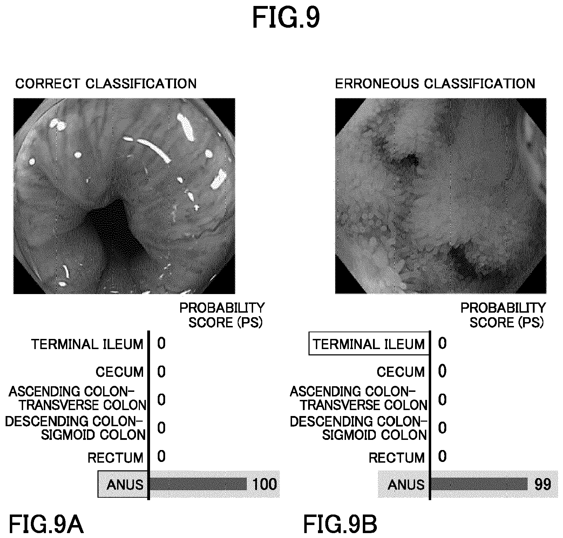

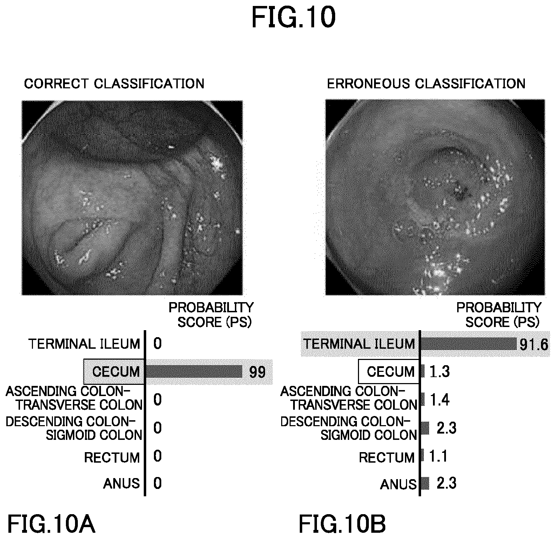

Review was then conducted on 1,711 images (subtracting the number of correctly determined images from the total number of images = 5,121 -3,410 = 1,711, see Table 5) erroneously recognized by the CNN system according to the second embodiment. The CNN system according to the second embodiment erroneously recognized 17.5% of the entire images (299/1,711), and the probability scores were 0.9 or higher. FIGS. 9 and 10 illustrate typical examples of images erroneously recognized by the CNN according to the second embodiment. FIG. 9A is an example of an endoscopic image recognized correctly as the anus, and FIG. 9B illustrates an image of the terminal ileum erroneously recognized as the anus. The contour of the lumen in FIG. 9B was similar to the contour of the anus. FIG. 10A is an example of an endoscopic image recognized correctly as the cecum, and FIG. 10B is an example of an image of the cecum erroneously recognized as the terminal ileum. In FIG. 10A, the appendix cavity is visible as one of the features of the cecum, whereas in FIG. 10B, the cecum was erroneously recognized as the terminal ileum.

As described above, in the second embodiment, the CNN system was build based on the 9995 colonoscopic images of the 409 patients. This CNN system was caused to identify the anatomical sites using an independent large-scale validation data set, and this CNN system exhibited clinically useful performance. This CNN system succeeded in recognizing images of the colon at an accuracy of 60% or higher. Therefore, it is expected that this CNN system will serve as a foundation for the development of AI systems for colonoscopy in the near future.

In order to develop an AI system for colon diseases, the first important factor is the capability for efficiently recognizing anatomical sites in an image. Conventionally, AI systems for recognizing colon polyps have been known, and the sensitivity was within a range of 79% and 98.7%, and the degree of specificity was within a range of 74.1% and 98.5%. However, the conventional systems do not have the capability for recognizing an anatomical site of the polyp. It is well known that the frequency of polyp or colorectal cancer occurrence differs depending on anatomical sites of the colon. If the CNN system according to the second embodiment can change the sensitivity at which a colon lesion is detected based on its anatomical sites, it is possible to develop a more effective AI system.

In the CNN system built in the second embodiment, the accuracy varied depending on values of probability scores. Generally, because images with higher probability scores are recognized at a higher accuracy, the CNN system is enabled to function better by limiting images only to those with higher probability scores. To realize a clinically useful application, appropriate probability scores are required to achieve more reliable recognition results.

The results achieved by the CNN system built in the second embodiment was not better than previous reports made by the inventors of the present invention who built a CNN system capable of classifying gastrointestinal images. The conventional sensitivity and the conventional degree of specificity for recognizing anatomical sites of the gastrointestinal tract were 93.9% and 100% for the larynx, 95.8% and 99.7% for the esophagus, 98.9% and 93.0% for the stomach, and 87.0% and 99.2% for the duodenum, respectively.

However, even for clinicians, it is more difficult to recognize anatomical sites correctly in a colonoscopic image, at the same level as for anatomical sites in a gastrointestinal endoscopic image. For example, clinicians are sometimes not able to distinguish an image of the ascending-transverse colon from an image of the descending-sigmoid colon. In particular, images with a margin between each of sites are difficult to recognize. Furthermore, clinicians can usually recognize which site a colonoscopic image represents by considering a successive order of images, or a relation of an image with a previous or a subsequent image in the clinical setting. Thus, the accuracy of 66% achieved by the CNN system based on a single image can be improved by integrating a relation of the image with a previous or a subsequent image in the manner described above, which cannot be underestimated.

The sensitivity and the degree of specificity of the CNN system built in the second embodiment vary depending on anatomical sites. For the descending colon-sigmoid colon site, the CNN system exhibited a high sensitivity of 90% or higher, but exhibited the lowest degree of specificity of 69.9%. By contrast, for the terminal ileum, the cecum, the ascending colon-transverse colon, and the rectum, the CNN system exhibited high degrees of specificity but exhibited low sensitivities of 23.3% to 69.4%. Furthermore, the CNN system according to the second embodiment recognized the anus at a high sensitivity and a high degree of specificity of 90% or higher. Interestingly, the recognition sensitivity for the rectum decreased when the sensitivity was calculated from an image with a high probability score.

The CNN system according to the second embodiment failed to make a correct output reliably for rectum images, and recognized rectum images as the descending-sigmoid colon. It is assumed that the reason why the rectum was recognized at a low sensitivity is that the rectum has no characteristic portion. However, with the CNN system according to the second embodiment, although the terminal ileum and the cecum had characteristic portions such as an ileocecal valve and an appendix orifice, respectively, recognition sensitivities remained relatively low. The reason why such results were acquired can be explained by the fact that the CNN system according to the second embodiment was not able to recognize such a characteristic portion belonging to each site. The reason is that the CNN system according to the second embodiment can recognize an image based only on the entire structure in an image, and merely classifies all the images into corresponding sites without being trained with characteristic portions based on each of the sites in the images. If the CNN system according to the second embodiment can be trained with typical portions in images, the recognition accuracy for sites therein are improved.

In other words, it becomes difficult to capture the shape of a lumen when the endoscope is moved closer to the surface of a site, or when the lumen is not sufficiently filled with the air. Because the epithelia of the esophagus, the stomach, and the duodenum in images of the esophagus, the stomach, and the duodenum are different from one another, it is necessary to recognize images based on the microstructure of the surface. For example, in the stomach, the epithelium is classified differently depending on anatomical sites. For example, pyloric glands are distributed across the gastric pylorus, and gastric fundic glands exist in another area.

By contrast, the cecum, the ascending colon, the transverse colon, the descending colon, the sigmoid colon, and the rectum have microstructures the patterns of which are almost the same. Therefore, it is inefficient to train the CNN system with surface microstructures so as to enable the CNN system to distinguish colorectal images. However, it is useful to train the CNN system with surface microstructures in order to enable the CNN system according to the second embodiment to recognize the terminal ileum or the anus.

Furthermore, in the CNN system according to the second embodiment, in order to improve the capability for correct position identification for images, the colonoscopy may be combined with another modality for capturing medical images, such as an X-ray computed tomography (CT) device, an ultrasonic computer tomography (USCT) device, and a magnetic resonance imaging (MRI) device capable of displaying three-dimensional information such as a computer tomographic image or a fluoroscopic image. When images with such modalities can be used for the training data set, the CNN system can recognize the position for colonoscopic images more correctly.

The capability for automatically recognizing anatomical sites of the colon has a great impact on diagnoses as well as on treatments. Firstly, the position where a colon disease is located is recognized. For example, in the treatment of ulcerative colitis, treatment can be provided or an appropriate type of drug can be administered, based on a site where the colitis is present. Furthermore, for a colorectal cancer, an anatomical site where the cancer is located is important information for surgery.

Secondly, information on anatomical sites of the colon is useful for a correct examination in a process between insertion and discharge of a colonoscope. In particular, in order for a medical intern who is in the process of training or a physician at the first contact to complete the insertion of an endoscope scope, one of the most difficult tasks is to recognize where the endoscope scope is inserted. The CNN system enables objective recognition of the position where the endoscope scope is located, which is useful for a medical intern who is in the process of training or a physician at first contact to insert the colonoscope. If a function of recognizing anatomical sites is provided in video images, the time and the difficulty for completing the insertion of the colonoscope are reduced.

[Third Embodiment]

In a third embodiment, a diagnostic assistance method, a diagnostic assistance system, a diagnostic assistance program, and a computer-readable recording medium storing therein the diagnostic assistance program for a disease of an erosion/ulcer in the small bowel based on a wireless capsule endoscope (WCE) image will now be explained. Note that in the third embodiment, because the distinction between the erosion and the ulcer was difficult, the erosion and the ulcer are collectively described as "erosion/ulcer". In other words, the term "erosion/ulcer" herein is used to mean not only "erosion", "ulcer", and "erosion and ulcer", but also something which is "not clear whether it is erosion or ulcer, but at least not a normal mucosa".

[About Data Set]

As the training data set, 5360 images of an erosion/ulcer in the small bowel were collected from 115 patients who received a WCE within a period between October 2009 and December 2014 in the clinic to which one of the inventors of the present invention belongs. Furthermore, for the validation of the CNN system according to the third embodiment, 10,440 independent images were prepared from 65 patients within a period between January 2015 and January 2018, and such images were used for a validation data set. In this validation data set, 440 images from 45 patients included an erosion/ulcer in the small bowel, while 10,000 image from 20 patients were diagnosed to exhibit a normal mucosa of the small bowel by three endoscopy specialists. The WCE was performed using a Pillcam (registered trademark) SB2 or SB3WCE (Given Imaging, Yoqneam, Israel).

Note that in order to train/validate the CNN system according to the third embodiment, all pieces of the patient information associated with the images were anonymized, before the algorithm was developed. It was also ensured that none of the endoscopy specialists involved with the CNN system according to the third embodiment had any access to any identifiable patient information. Because this training/validation of the CNN system was a retrospective study using the anonymized data, an opt-out approach was used for the consent from the patients. This study was approved by the Ethics Committee of the University of Tokyo (No. 11931) and the Japan Medical Association Ethical Review Board (ID JMA-IIA00283). An overview of a flowchart for the CNN system according to the third embodiment is illustrated in FIG. 10.

Indications for the WCE were mainly obscure gastrointestinal bleeding (OGIB), and others included cases in which abnormality in the small bowel was observed in an image using another medical device, abdominal pains, follow-ups of a past small bowel case, or a referral concerning screening for diarrhea from a primary care physician, and the like. The most frequent cause was nonsteroidal anti-inflammatory, and an inflammatory bowel disease came to the second. Other main causes were a malignant tumor in the small bowel, and an anastomotic ulcer. However, there were many cases the cause of which could not be identified. The patient characteristics of the data set used for the training and the validation of the CNN system are indicated in Table 9.

[Table 9]

| Characteristics, number (%) | Training Data Set (n = 115) | Validation Data Set | |
|---|---|---|---|
| | | Erosion, ulcer (n = 45) | Normal (n = 20) |
| Number of Images | 5360 | 440 | 10,000 |
| Average Age ($\pm$SD) | 63$\pm$16 | 59$\pm$21 | 52$\pm$12 |
| Sex (male) | 62 (54) | 28 (62) | 12 (60) |
| Reason for receiving WCE | | | |
| Obscure gastrointestinal bleeding | 78 (69) | 29 (64) | 12 (60) |
| Abnormality in small bowel image using another medical device | 12 (10) | 2 (4) | 2 (10) |
| Abdominal pains | 8 (7) | 1 (2) | 3 (15) |
| Follow-ups | 6 (5) | 3 (7) | 0 (0) |
| Diarrhea | 4 (3) | 4 (9) | 1 (5) |
| Screening | 3 (3) | 1 (2) | 1 (5) |
| Crohn's disease | 2 (2) | 3 (7) | 0 (0) |
| Lymphoma | 2 (2) | 2 (4) | 1 (5) |
| Number of lesions | | | |
| One | 40 (35) | 12 (27) | - |
| More than one | 75 (65) | 33 (73) | - |
| Site of lesion | | | |
| Jejunum | 32 (28) | 13 (29) | - |
| Ileum | 47 (41) | 13 (29) | - |
| Diffuse lesion | 36 (31) | 19 (42) | - |
| Cause of disease | | | |
| Nonsteroidal anti-inflammatory drug | 30 (26) | - | - |
| Inflammatory bowel disease | 10 (9) | 5 (11) | - |
| Malignant tumor in small bowel* | 8 (7) | 2 (4) | - |
| Anastomotic ulcer | 7 (6) | 2 (4) | - |
| Ischemic enteritis | 2 (2) | 2 (4) | - |
| Meckel's diverticulum | 2 (2) | 0 (0) | - |
| Radiation enteritis | 1 (1) | 0 (0) | - |
| Infectious enteritis | 0 (0) | 1 (2) | - |
| Other cases | 3 (3)** | 3 (7)*** | - |
| Unknown | 52 (45) | 14 (31) | - |
| * Including small intestine cancer and malignant lymphoma<br>** Scar (n = 1) and damages caused by double-balloon endoscope (n = 2)<br>*** graft-versus-host disease (GVHD) (n = 3) | | | |

[Training/Validation Algorithm]

In order to build the CNN system according to the third embodiment, a deep neural network architecture referred to as Single Shot MultiBox Detector (SSD, https://arxiv.org/abs/1512.02325) was used without changing the algorithm. To begin with, two endoscopy specialists manually appended annotations having a rectangular boundary box to all regions of an erosion/ulcer in images of the training data set. These images were then incorporated into the SSD architecture via the Caffe framework developed first in the Berkeley Vision and Learning Center. The Caffe framework is one of the frameworks that was developed first and is most common and widely used.

The CNN system according to the third embodiment was trained such that regions inside the boundary box are an erosion/ulcer region, and the other regions are the background. Then, the CNN system according to the third embodiment extracted specific features of the boundary box region by itself, and "learned" features of an erosion/ulcer through the training data set. All of the layers in the CNN was applied with a probabilistic optimization at a global learning rate of 0.0001. Each of images was resized to 300×300 pixels. Accordingly, the size of the boundary box was also changed. These values were set through trial and error to ensure that every piece of data has the compatibility with SSD. As the CPU, INTEL's Core i7-7700K was used, and as the graphics processing unit (GPU), NVIDEA's GeForce GTX 1070 was used.

[Measurement Results and Statistics]

To begin with, a rectangular boundary box (hereinafter, referred to as a "true box") was manually provided to all of erosions/ulcers in images included in the validation data set, using a thick line. Further, the trained CNN system according to the third embodiment was caused to provide a rectangular boundary box (hereinafter, referred to as a "CNN box") with a thin line to a region corresponding to an erosion/ulcer detected in the images in the validation data set, and to output a probability score (within the range of 0 and 1) for the erosion/ulcer. When the probability score is higher, it means that the trained CNN system according to the third embodiment determines that the region is more likely to include an erosion/ulcer.

The inventors of the present invention evaluated the capability with which the CNN system according to the third embodiment determines whether each of images includes an erosion/ulcer. To make this evaluation, the following definitions were used.

1) It was determined to be correct when the CNN box overlapped with the true box by 80% or higher.
2) When there were a plurality of CNN boxes within one image, and at least one of the boxes correctly detected an erosion/ulcer, it was concluded that the image was identified correctly.

Note that a WCE endoscopic image determined to be correct in the manner described above is used as a diagnostic assistance in double-checking captured images in the practice, by assigning the information to the image, or used as a diagnostic assistance by displaying the information in real time as a video during a WCE endoscopic examination.

Further, a receiver operating characteristic (ROC) curve was plotted by changing a cutoff value of a probability score, and the area under the curve (AUC) was calculated to evaluate erosion/ulcer identification performed by the trained CNN system according to the third embodiment. By using various cutoff values of a probability score including a score in accordance with the Youden index, the sensitivity, the degree of specificity, and the accuracy representing the capability with which the CNN system according to the third embodiment detects an erosion/ulcer were calculated. Note that the Youden index is one of the standard methods for determining an optimal cutoff value, calculated using the sensitivity and the degree of specificity, and is used to obtain such a cutoff value that a value of "the sensitivity + the degree of specificity - 1" is maximized. In this example, the data were statistically analyzed using STATA software (version 13; StataCorp, College Station, TX, USA).

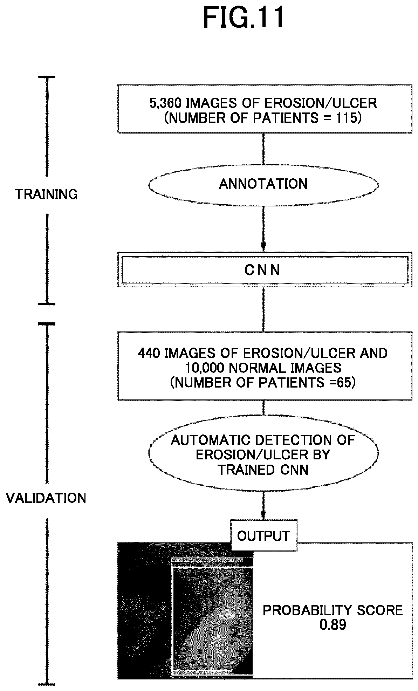

The validation data set contained 10,440 images of 65 patients (male = 62%, average age = 57, standard deviation (SD) age = 19). The trained CNN system according to the third embodiment required 233 seconds to evaluate these images. This is equivalent to a rate of 44.8 images per second. The AUC of the trained CNN system according to the third embodiment which detected an erosion/ulcer was 0.960 (with a 95% confidence interval [CI], 0.950 to 0.969; see FIG. 11).

According to the Youden index, the optimal cutoff value for a probability score was 0.481, and a region with a probability score of 0.481 was recognized as an erosion/ulcer by the CNN. At the cutoff value, the sensitivity, the degree of specificity, and the accuracy of the CNN were 88.2% (95% CI (confidence interval), 84.8% to 91.0%), 90.9% (95% CI, 90.3% to 91.4%), and 90.8% (95% CI, 90.2% to 91.3%), respectively (see Table 10). Note that table 10 indicates the sensitivity, the degree of specificity, and the accuracy as the cutoff value of a probability score was increased from 0.2 to 0.9 at an increment of 0.1.

[Table 10]

| Cutoff value (probability score) | Sensitivity (%) | Degree of specificity (%) | Accuracy (%) |
|---|---|---|---|
| 0.2 | 98.9 | 56.0 | 57.8 |
| 0.3 | 95.9 | 76.7 | 77.5 |
| 0.4 | 91.6 | 86.0 | 86.3 |
| 0.481* | 88.2 | 90.9 | 90.8 |
| 0.5 | 86.8 | 91.7 | 91.5 |
| 0.6 | 81.4 | 94.9 | 94.4 |
| 0.7 | 74.6 | 97.2 | 96.2 |
| 0.8 | 63.6 | 98.6 | 97.1 |
| 0.9 | 45.2 | 99.3 | 97.1 |

*Calculation with Youden index

Relations between results of erosion/ulcer classification classified in the manner described above by the trained CNN system according to the third embodiment, with a cutoff value of a probability score = 0.481, and results of erosion/ulcer classification classified by endoscopy specialists are summarized in Table 11.

[Table 11]

| | | Classification by specialists | | |
|---|---|---|---|---|
| | | Erosion/ulcer | Normal | Total |
| Classification by CNN | Erosion/ulcer | 388 | 913 | 1,301 |
| | Normal | 52 | 9,087 | 9,139 |
| | Total | 440 | 10,000 | 10,440 |

Sensitivity = 88.2%   Degree of specificity = 90.9%

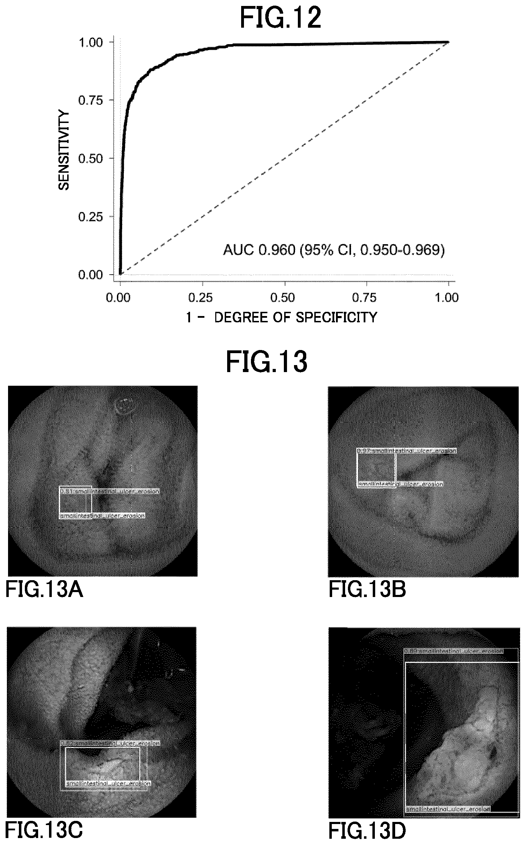

Furthermore, FIGS. 12A to 12D illustrate typical regions correctly detected by the CNN system, and FIGS. 13A to 13H illustrate typical regions erroneously classified by the CNN system. As indicated in Table 12, causes of false negative images were classified into the following four classes: the boundary was unclear (see FIG. 13A); the color was similar to that of the normal mucosa therearound; the size was too small; and the entire picture was not observable (due to the laterality (an affected area is hard to see because the area is located on the side) or the partiality (only partially visible)) (see FIG. 13B).

[Table 12]

| Cause of false negative (n = 52) | n (%) |
|---|---|
| Unclear boundary* | 33 (64) |

(continued)

| Cause of false negative (n = 52) | n (%) |
|---|---|
| Same color as that of surrounding mucosa | 11 (21) |
| Too small | 6 (12) |
| Not entirely observable | 2 (4) |
| *: Due to darkness, debris, or out of focus | |

Meanwhile, causes of false positive images were classified into five classes of normal mucosa, bubbles (FIG. 13C), fragments (FIG. 13D), vasodilatation (FIG. 13E), and true erosion (FIGS. 13F to 13H), as indicated in Table 13.

[Table 13]

| Cause of false positive (n = 913) | n (%) |
|---|---|
| Normal mucosa | 347 (38) |
| Bubbles | 228 (25) |
| Debris | 216 (24) |
| Vasodilatation | 119 (13) |
| True erosion** | 3 (0.3) |
| **Those recognized as erosion by endoscopy specialists after CNN pointed out lesions | |

As described above, with the trained CNN system according to the third embodiment, a CNN-based program for automatically detecting an erosion and an ulcer in WCE images of the small bowel was built, and it became clear that detection of an erosion/ulcer succeeds in independent test images at a high accuracy of 90.8% (AUC, 0.960).

[Fourth Embodiment]

In a fourth embodiment, a diagnostic assistance method, a diagnostic assistance system, a diagnostic assistance program, and a computer-readable recording medium storing therein the diagnostic assistance program for a disease of a protruding lesion in the small bowel based on a wireless capsule endoscope (WCE) image will now be explained. Note that morphological characteristics of the protruding lesion are various and include polyps, nodules, epithelium tumors, submucosal tumors, and venous structures, and etilologies of these lesions include neuroendocrine tumor, adenocarcinoma, familial adenomatous polyposis, Peutz-Jeghers syndrome, follicular lymphoma, and gastrointestinal stromal tumor. Because these lesions require early diagnosis and treatment, oversights during WCE examinations should be avoided.

[About Data Set]

As the training data set, 30,584 images of a protruding lesion were collected from 292 patients who received a WCE within a period between October 2009 and May 2018 in the clinics to which the inventors of the present invention belongs. Further, independent of images used for training the CNN, a total of 17,507 images, including 10,000 images without a lesion and 7,507 images with a protruding lesion, from 93 patients were used for validation. The protruding lesion was morphologically classified into five categories as polyps, nodules, epithelium tumors, submucosal tumors, and venous structures based on the definition of the CEST classification (seeNon Patent Literature 13). Note that mass/tumor lesions on the CEST definition were classified into epithelium tumors and submucosal tumors.

[Training/Validation Algorithm]

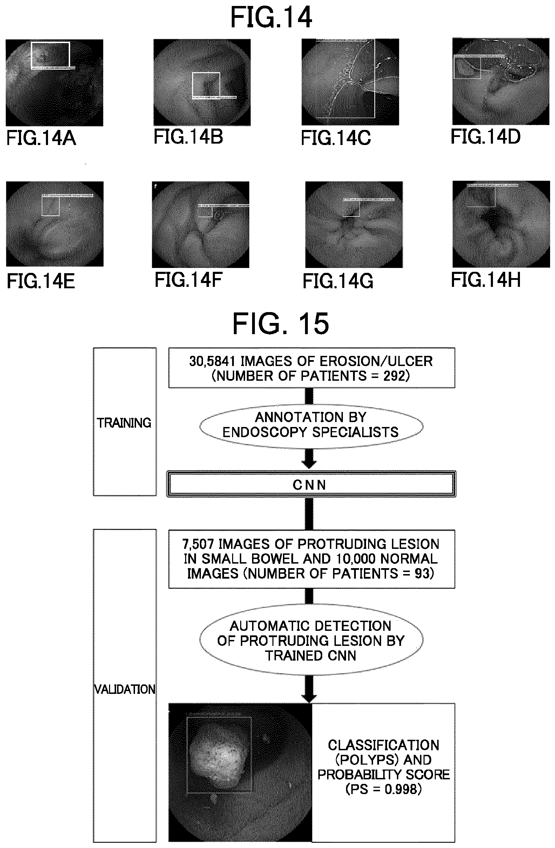

An overview of a flowchart for the CNN system according to the fourth embodiment is illustrated in FIG. 15. For the CNN system according to the fourth embodiment, the SSD deep neural network architecture and the Caffe framework which are similar to those in the third embodiment were used. First, six endoscopy specialists manually appended annotations having a rectangular boundary box to the areas of all protruding lesions in images of the training data set. The annotation was performed separately by each endoscopy specialist, and consensus was later determined.

These images were fed into the SSD architecture through the Caffe deep learning framework.

The CNN system according to the fourth embodiment was trained such that regions inside the boundary box are a protruding lesion region, and the other regions are the background. Then, the CNN system according to the fourth embodiment extracted specific features of the boundary box region by itself, and "learned" features of the protruding lesion through the training data set. All of the layers in the CNN was applied with a probabilistic optimization at a global learning rate of 0.0001. Each of images was resized to 300x300 pixels. Accordingly, the size of the boundary box was also changed. These values were set through trial and error to ensure that every piece of data has the compatibility with SSD. As the CPU, INTEL's Core i7-7700K was used, and as the graphics processing unit (GPU), NVIDEA's GeForce GTX 1070 was used. As the WCE, Pillcam SB2 or SB3WCE similar to that in the third embodiment was used. The data were analyzed using STATA software (version 13; StataCorp, College Station, TX, USA).

Note that in order to train/validate the CNN system according to the fourth embodiment, all pieces of the patient information associated with the images were anonymized, before the algorithm was developed. It was also ensured that none of the endoscopy specialists involved with the CNN system according to the fourth embodiment had any access to any identifiable patient information. Because this training/validation of the CNN system was a retrospective study using the anonymized data, an opt-out approach was used for the consent from the patients. This study was approved by the Japan Medical Association Ethical Review Board (ID JMA-IIA00283), Sendai Kosei Hospital (No.30-5), the University of Tokyo Hospital (No. 11931), and Hiroshima University Hospital (No. E-1246) .

[Measurement Results and Statistics]

To begin with, a rectangular boundary box (hereinafter, referred to as a "true box") was manually provided to all of protruding lesion regions in images included in the validation data set, using a thick line. Further, the trained CNN system according to the fourth embodiment was caused to provide a rectangular boundary box (hereinafter, referred to as a "CNN box") with a thin line to a region corresponding to the protruding lesion detected in the images in the validation data set, and to output a probability score (within the range of 0 and 1) for the protruding lesion region. When the probability score is higher, it means that the trained CNN system according to the fourth embodiment determines that the region is more likely to include the protruding lesion.

The inventors of the present invention evaluated the CNN box in descending order of a possibility score for each of images in terms of the capability with which the CNN system according to the fourth embodiment determines whether each of images includes the protruding lesion. The CNN box, the possibility score of the protruding lesion, and the category of the protruding lesion were determined as the outcome of the CNN, when a CNN box clearly framed the protruding lesion.

When a CNN box was difficult to be visually determined due to many drawn boxes and when the overlap of a CNN box and the true box was greater than or equal to 0.05 Intersection over Unions (IoU), determination as the outcome of the CNN was made. Note that the IoU is an evaluation method to measure the accuracy of an object detector and is calculated by dividing the area of overlap of the two boxes by the area of union of two boxes.

$$\mathtt{IoU = (area\ of\ overlap)/(area\ of\ union)}$$

If a CNN box was not applied to the above rules, the CNN box with the next lower possibility score was evaluated in order.

When a plurality of true boxes were presented in one image, if one of the CNN boxes overlaps the truth box, the CNN box was determined as the outcome of the CNN. For images without the protruding lesion, the CNN box with the maximum probability score was determined as the outcome of the CNN. These tasks were carried out for all images by three endoscopy specialists.

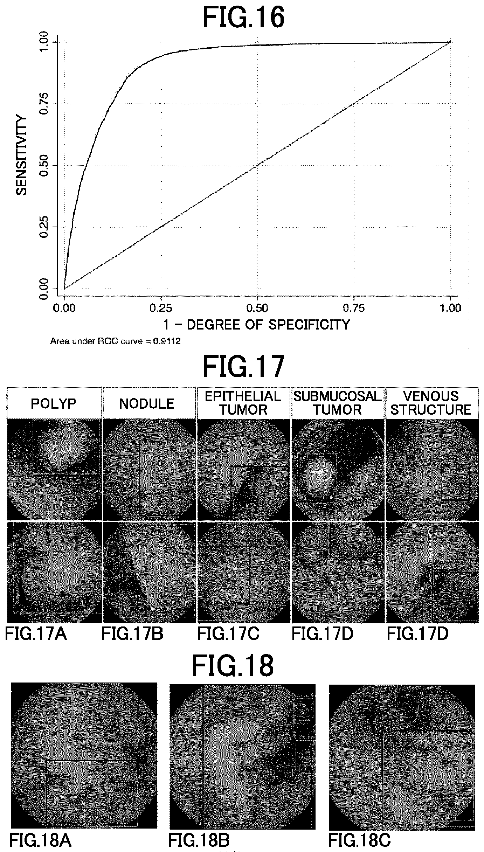

A receiver operating characteristic (ROC) curve was plotted by changing a cutoff value of a probability score, and the area under the curve (AUC) was calculated to evaluate the degree of identification of the protruding lesion by the trained CNN system according to the fourth embodiment (see FIG. 16). Then, similarly to the third embodiment, by using various cutoff values of a probability score including a score in accordance with the Youden index, the sensitivity, the degree of specificity, and the accuracy representing the capability with which the CNN system according to the fourth embodiment detects the protruding lesion were calculated.

Secondary outcome of the fourth embodiment was the classification of the protruding lesion into five categories by the CNN, and the detection of the protruding lesion in the individual patient analysis. For the classification accuracy, the concordance rate of classification between the CNN and endoscopy specialists was examined. In the individual patient analysis for the detection rate of the protruding lesion, the detection by the CNN was defined as correct when the CNN detected at least one protruding lesion image from multiple images of the same patient.

Further, we reevaluated 10,000 clinically normal images in the validation test set after the CNN process was performed. CNN boxes in normal images that appeared to be some true lesions were extracted. It is possible that such lesions were overlooked by physicians. This was based on the consensus of three endoscopy specialists.

The patient characteristics of the data set used for the training and the validation of the CNN system according to the fourth embodiment and details of the training data set and the validation data set are indicated in Table 14. The validation test set consisted of 7,507 images with the protruding lesion from 73 patients (males, 65.8%; mean age, 60.1 years; standard deviation, 18.7 years) and 10,000 images without the lesion from 20 patients (males, 60.0%; mean age, 51.9 years; standard deviation, 11.4 years).

[Table 14]

| Characteristics, number (%) | Training data set | Test data set Protruding lesion | Normal |
|---|---|---|---|
| No. of images | 30,584 | 7,507 | 10,000 |
| Polyps | 10,704 (35.0) | 3,522 (46.9) | - |
| Nodules | 11,907 (38.9) | 1,932 (25.7) | - |
| Epithelial tumors | 6,514 (21.3) | 1,462 (19.5) | - |
| Submucosal tumors | 1,875 (6.1) | 339 (4.5) | - |
| Venous structures | 393 (1.3) | 252 (3.4) | - |
| No. of patients | 292 | 73 | 20 |
| Mean age ± SD | 61.1 ± 17.0 | 60.1 ± 18.7 | 51.9 ± 11.4 |
| Sex (male) | 184 (63.0) | 48 (65.8) | 12 (60.0) |
| Protruding lesion | | | |
| Polyps | 78 (26.7) | 30 (41.1) | - |
| Nodules | 78 (26.7) | 14 (19.2) | - |
| Epithelial tumors | 36 (12.3) | 14 (19.2) | - |
| Submucosal tumors | 66 (22.6) | 11 (15.1) | - |
| Venous structures | 34 (11.6) | 4 (5.5) | - |
| No. of lesions per patient | | | |
| Single | 133 (45.6) | 37 (50.7) | - |
| Multiple Diagnosis | 159 (54.4) | 36 (49.3) | - |
| Hamartomatous polyp | 31 (10.6) | 11 (15.1) | - |
| Adenomatous polyp | 23 (7.9) | 5 (6.8) | - |
| Inflammatory polyp | 3 (1.0) | 4 (5.5) | - |
| Malignant lymphoma | 95 (32.5) | 18 (24.7) | - |
| Cancer | 12 (4.1) | 6 (8.2) | - |
| Lipomatosis | 15 (5.1) | 3 (4.1) | - |
| GIST | 9 (3.1) | 0 (0) | - |
| Carcinoid | 3 (1.0) | 1 (1.4) | - |
| Hemagioma | 15 (5.1) | 2 (2.7) | - |
| Varices | 8 (2.7) | 1 (1.4) | - |
| Others | 33 (11.3) | 10 (13.7) | - |
| Unknown | 45 (15.4) | 12 (16.4) | - |

The CNN constructed in the fourth embodiment analyzed all the images in 530.462 seconds, with an average speed of 0.0303 seconds per image. The AUC of the CNN according to the fourth embodiment that is used to detect the protruding lesion was 0.911 (95% confidence interval (CI), 0.9069 to 0.9155) (see FIG. 16).

According to the Youden index, an optimal cutoff value for a probability score was 0.317. Thus, regions with a probability score of 0.317 or more were recognized as the protruding lesion detected by the CNN. Using this cutoff value, the sensitivity and the degree of specificity of the CNN were 90.7% (95% CI, 90.0% to 91.4%) and 79.8% (95% CI, 79.0% to 80.6%), respectively (Table 15).

[Table 15]

|  |  | Diagnosis by specialists |  |  |
|---|---|---|---|---|
|  |  | Protruding lesion | Normal mucosa | Total |
| CNN diagnosis | Protruding lesion | 6,810 (90.7) | 2,019 (20.2) | 8,678 |
|  | Normal mucosa | 697 (9.3) | 7,981 (79.8) | 8,829 |
|  | Total | 7,507 | 10,000 | 17,507 |
|  |  | Sensitivity 90.7% | Degree of specificity 79.8% |  |

In the subgroup analysis of the category of the protruding lesion, the sensitivity of the CNN was 86.5%, 92.0%, 95.8%, 77.0%, and 94.4% for the detection of polyps, nodules, epithelial tumors, submucosal tumors, and venous structures, respectively. FIGS. 17A to 17F illustrate representative regions correctly detected and classified by the CNN into five categories as polyps, nodules, epithelial tumors, submucosal tumors, and venous structures.

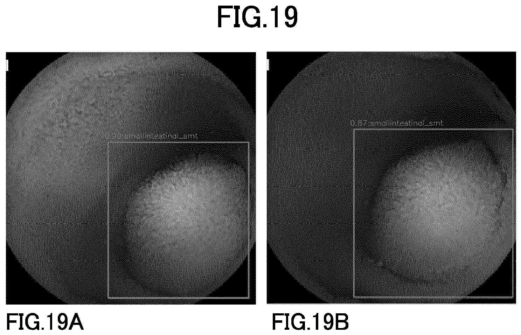

In the individual patient analysis, the detection rate of the protruding lesion was 98.6% (72/73). Based on the categories of the protruding lesion, the detection rate per patient of polyps, nodules, epithelial tumors, submucosal tumors, and venous structures was 96.7% (29/30), 100% (14/14), 100% (14/14), 100% (11/11), and 100% (4/4), respectively. However, all three images of polyps of one patient illustrated in FIGS. 18A-18C could not be detected by the CNN according to the fourth embodiment. For these images, all the CNN boxes exhibited a possibility score below 0.317, and consequently no protruding lesion was detected by the CNN. Further, from the false-positive images (n = 2,019) provided with CNN boxes having a possibility score of 0.317 or more by the CNN that seemed, however, to have no protruding lesion, it was suggested that two of them exhibited the true protruding lesion by endoscopy specialists (FIG. 19).

The labeling of the protruding lesion by the CNN and expert endoscopy specialists is indicated in Table 16. The concordance rate of the labelling by the CNN and the endoscopy specialists for polyps, nodules, epithelial tumors, submucosal tumors, and venous structures was 42.0%, 83.0%, 82.2%, 44.5%, and 48.0% respectively.

[Table 16]

| | | The expert's classification (n = 7,507) | | | | | |
|---|---|---|---|---|---|---|---|
| | | Polyps | Nodules | Epithelial tumors | Submucosal tumors | Venous structures | Total |
| The CNN classification | Polyps | 1,478 (42.0) | 52 (2.7) | 121 (8.3) | 48 (14.2) | 2 (0.8) | 1,701 |
| | Nodules | 432 (12.3) | 1,604 (83.0) | 39 (2.7) | 2 (0.6) | 0 (0) | 2,077 |
| | Epithelial tumors | 1,298 (36.9) | 15 (0.8) | 1,202 (82.2) | 53 (15.6) | 86 (34.1) | 2,654 |
| | Submucosal tumors | 31 (0.9) | 0 (0) | 39 (2.7) | 151 (44.5) | 29 (11.5) | 250 |
| | Venous structures | 0 (0) | 0 (0) | 0 (0) | 7 (2.1) | 121 (48.0) | 128 |
| | No lesion | 283 (8.0) | 261 (13.5) | 61 (4.2) | 78 (23.0) | 14 (5.6) | 697 |
| | Total | 3,522 | 1,932 | 1,462 | 339 | 252 | 7,507 |

As described above, the categories based on the CEST were applied, and although there were differences in sensitivity among the categories ; polyps, nodules, epithelial tumors, submucosal tumors, and venous structures, it was revealed that the CNN according to the fourth embodiment enables detection and classification at a high sensitivity and a favorable detection rate.

[Fifth Embodiment]

Wireless capsule endoscopy (hereinafter, simply referred to as "WCE") has become an essential tool for investigating small bowel diseases, and the major indication for performing the WCE is mainly obscure gastrointestinal bleeding (OGIB) of unknown cause from a source that cannot be identified. In screening WCE images, physicians are required to spend 30-120 minutes reading more than 10,000 images per patient. Thus, it is important in a WCE image analysis whether to be capable of automatically detecting blood contents. As a means for automatically detecting blood contents in such WCE images, for example, the "red region estimation indication function" (suspected blood indicator: hereinafter simply referred to as "SBI") is known (see Non Patent Literature 11). The SBI is an image selection tool that is mounted on the RAPID CE reading software (Medtronic, Minneapolis, MN, USA) and tags possible regions of bleeding with red pixels.

In a fifth embodiment, a diagnostic assistance method, a diagnostic assistance system, a diagnostic assistance program, and a computer-readable recording medium storing therein the diagnostic assistance program for bleeding in the small bowel based on a WCE image will be explained in comparison with the above SBI. Note that in detecting blood contents in the small bowel, the blood can be quantitatively estimated and, in such a case, the blood quantity can be also estimated from a blood distribution range or the like. In the following, by way of example, cases of detecting the presence/absence of blood contents, i.e., the presence/absence of bleeding will be explained.

[About Data Sets]

WCE images between November 2009 and August 2015 were retrospectively obtained from a single institute (The University of Tokyo Hospital, Japan) to which one of the inventors of the present invention belongs. In that period, the WCE was performed using a Pillcam SB2 or SB3 WCE device similar to that in the third embodiment. Two endoscopy specialists obtained images of luminal blood contents and images of normal small bowel mucosa, without consideration for the SBI. The luminal blood contents were defined as active bleeding or blood clots.

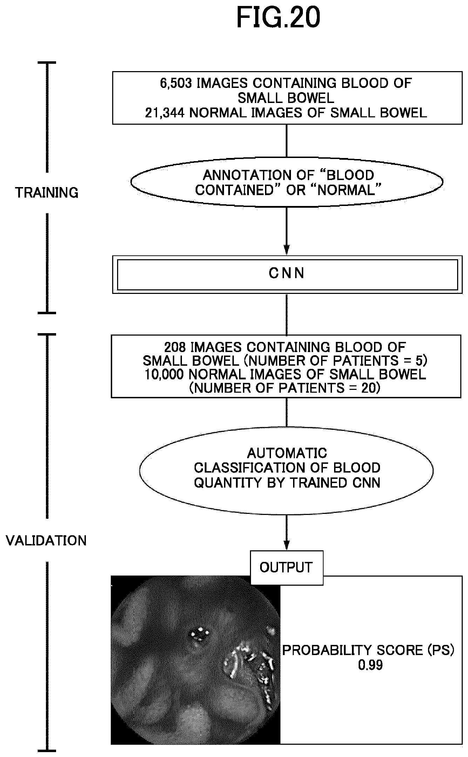

As the training data set for the CNN system according to the fifth embodiment, we collected 27,847 images (6,503 images of blood contents from 29 patients and 21,344 images of normal small bowel mucosa from 12 patients). Similarly, as the validation data set for the CNN system, 10,208 images were prepared independently of the training data set. From among these images, 208 images from 5 patients indicated blood contents in the small bowel, and 10,000 images from 20 patients were of normal small bowel mucosa. An overview of a flowchart for the CNN system according to the fifth embodiment is illustrated in FIG. 20.

Note that in order to train/validate the CNN system according to the fifth embodiment, all pieces of the patient information associated with the images were anonymized, before the algorithm was developed. It was also ensured that none of the endoscopy specialists involved with the CNN system according to the fifth embodiment had any access to any identifiable patient information. Because this training/validation of the CNN system was a retrospective study using the anonymized data, an opt-out approach was used for the consent from the patients. This study was approved by the Ethics Committee of the University of Tokyo (No. 11931) and the Japan Medical Association Ethical Review Board (ID JMA-IIA00283).

[Training/Validation Algorithm]

The algorithm for the CNN system used in the fifth embodiment was developed using ResNet50 (https://arxiv.org/abs/1512.03385) which is a deep neural network architecture with 50 layers. Then, the Cafe framework originally developed at the Berkeley Vision and Learning Center was used to train and validate a newly-developed CNN system. Stochastic optimization of all layers of the network was carried out using stochastic gradient descent (SGD) with a global learning rate of 0.0001. To ensure that all images were compatible with ResNet50, each image was resized to $224 \times 224$ pixels.

[Measurement Results and Statistics]

Primary outcome of the CNN system according to the fifth embodiment included the area under the receiver operating characteristic curve (ROC-AUC), the sensitivity, the degree of specificity, and the accuracy of the discrimination capability by the CNN system between images of blood contents and those of normal mucosa. The trained CNN system according to the fifth embodiment outputted a continuous number between 0 and 1 as a probability score for blood contents per image. The higher the probability score, the more the CNN system had confidence that the image included blood contents. The validation test of the CNN system according to the fifth embodiment was performed using a single still image, the ROC curve was plotted by varying a threshold of a probability score, and the AUC was calculated to assess the degree of discrimination.

In the fifth embodiment, for the final classification by the CNN system, the threshold of a probability score was simply set at 0.5, and the sensitivity, the degree of specificity, and the accuracy of discrimination capability by the CNN system between images of blood contents and those of normal mucosa were calculated. In addition, the sensitivity, the degree of specificity, and the accuracy of discrimination capability by the SBI between images of blood contents and those of normal mucosa were evaluated by reviewing 10,208 images in a validation set. The difference in the ability of the CNN system according to the fifth embodiment and the SBI was compared with each other using the McNemar's test. Obtained data were statistically analyzed using STATA software (version 13; StataCorp, College Station, TX, USA).

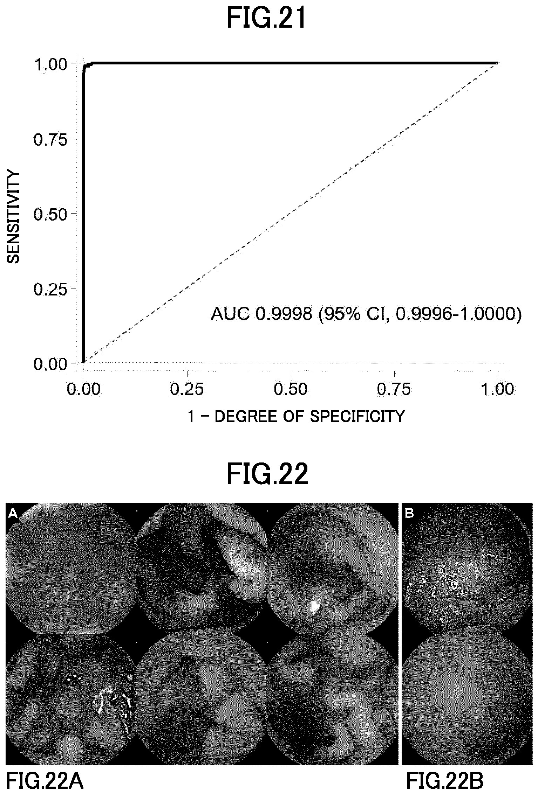

The validation set consisted of 10,208 images from 25 patients (males, 56%; mean age, 53.4 years; standard deviation, 12.4 years). The trained CNN system according to the fifth embodiment required 250 seconds to evaluate the images. This corresponds to a rate of 40.8 images per second. The AUC of the CNN system according to the fifth embodiment for discriminating images of blood contents was 0.9998 (95% confidence interval (CI), 0.9996-1.0000; see FIG. 21). Further, Table 15 indicates the sensitivity, the degree of specificity, and the accuracy each calculated by increasing a cutoff value for a probability score by 0.1 from 0.1 to 0.9.

[Table 17]

| | | Diagnosis by specialists | | |
|---|---|---|---|---|
| | | Protruding lesion | Normal mucosa | Total |
| CNN diagnosis | Protruding lesion | 6,810 (90.7) | 2,019 (20.2) | 8,678 |
| | Normal mucosa | 697 (9.3) | 7,981 (79.8) | 8,829 |
| | Total | 7,507 | 10,000 | 17,507 |
| | | Sensitivity 90.7% | Degree of specificity 79.8% | |

At the cutoff value of a probability score of 0.5, the sensitivity, the degree of specificity, and the accuracy of the CNN system according to the fifth embodiment were 96.63% (95% CI, 93.19-98.64%), 99.96% (95% CI, 99.90-99.99%), and 99.89% (95% CI, 99.81-99.95%), respectively. Note that although the cutoff value of a probability score of 0.21 was an optimal cutoff value calculated according to the Youden index, the accuracy at the cutoff value according to the Youden index was lower than the accuracy at the simple cutoff value of 0.5 in this validation data set. Further, FIG. 22 shows images of representative blood correctly classified by the CNN system according to the fifth embodiment (FIG. 22A) and images similarly showing normal mucosa images (FIG. 22B). Note that possibility scores obtained by the CNN system according to the fifth embodiment for each of FIGS. 22A and 22B are indicated in Table 18 as below.

[Table 18]
Probability scores of CNN

| A | | | B |
|---|---|---|---|
| 1.00 | 0.99 | 0.84 | 0.46 |
| 0.99 | 1.00 | 0.67 | 0.00 |

Table 18A relates to small bowel images of blood and Table 18B relates to images of normal small bowel mucosa.
Meanwhile, the sensitivity, the degree of specificity, and the accuracy of the SBI were 76.92% (95% CI, 70.59-82.47%), 99.82% (95% CI, 99.72-99.89%), and 99.35% (95% CI, 99.18-99.50%), respectively. All of these were significantly lower than those by the CNN system (p<0.01). Table 19 indicates category differences between the CNN system according to the fifth embodiment and the SBI.

[Table 19]
Probability scored of CNN

| 0.43 | 0.35 | 0.40 | 0.23 |
|---|---|---|---|
| 0.06 | 0.21 | 0.09 | |

The left table relates to images correctly classified by the SBI as blood contents (n = 4) and the right table relates to images incorrectly classified by the SBI as normal mucosa (n = 3).

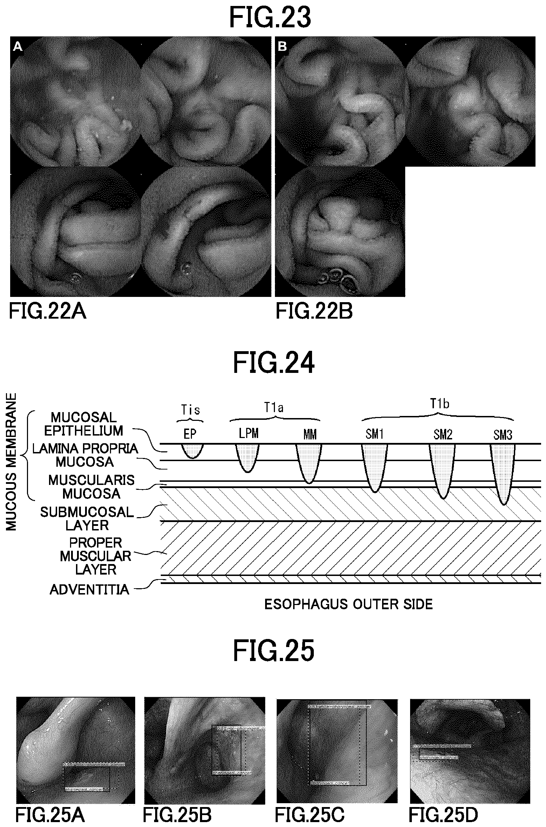

FIG. 23 shows seven false negative images classified as normal mucosa by the CNN according to the fifth embodiment. From among these images, 4 images shown in FIG. 23A were correctly classified as blood contents by the SBI and the other 3 images shown in FIG. 23B were incorrectly classified as normal by the SBI. Note that classification obtained by the CNN system according to the fifth embodiment and the SBI for each of FIGS. 23A and 23B is indicated in Table 20 as below and a relation of the classification between the CNN system and the SBI is indicated in Table 21.

[Table 20]

| CNN classification | | Classification by specialists | | |
|---|---|---|---|---|
| | | Blood content | Normal mucosa | Total |
| | Blood content | 201 | 4 | 205 |
| | Normal mucosa | 7 | 9,996 | 10,003 |
| | Total | 208 | 10,000 | 10,208 |
| | | **Sensitivity 96.63%** | **Degree of specificity 99.96%** | |

| SBI classification | | Classification by specialists | | |
|---|---|---|---|---|
| | | Blood content | No blood | Total |
| | Positive (red bar) | 160 | 18 | 178 |
| | Negative | 48 | 9,982 | 10,030 |
| | Total | 208 | 10,000 | 10,208 |
| | | **Sensitivity 76.92%** | **Degree of specificity 99.82%** | |

[Table 21]

| CNN classification | | SBI Classification | | |
|---|---|---|---|---|
| | | Positive (red bar) | Negative | Total |
| | Blood content | 160 | 45 | 205 |
| | Norma mucosa | 18 | 9,985 | 10,003 |
| | Total | 178 | 10,030 | 10,208 |

As described above, the trained CNN system according to the fifth embodiment was able to distinguish between images of blood contents and images of normal mucosa with a high accuracy of 99.9% (AUC, 0.9998). Further, direct comparison with the SBI revealed that the trained CNN system according to the fifth embodiment was able to classify more accurately than the SBI. Also at the simple cut-off point of 0.5, the trained CNN system according to the fifth embodiment was superior to the SBI in both the sensitivity and the degree of specificity. This result indicates that the trained CNN system according to the fifth embodiment can be used as a highly accurate screening tool for the WCE.

[Sixth Embodiment]

In a sixth embodiment, a diagnostic assistance method, a diagnostic assistance system, a diagnostic assistance program, and a computer-readable recording medium storing therein the diagnostic assistance program for diagnosing the invasion depth of squamous cell carcinoma (SCC) using an ordinary endoscope (a non-magnifying endoscope, a non-ME), an endoscopic ultrasonography (EUS), and a magnifying endoscope (ME) will be explained.

To begin with, a relation between an invasion depth of an esophageal SCC and its classification will be explained with reference to FIG. 24. The esophagus consists of, from the inner surface side of the esophagus, a mucosal epithelium (EP), a lamina propria mucosa (LPM), a muscularis mucosa (MM), a submucosal layer (SM), a proper muscular layer, and an adventitia. When the SCC remains in the mucosal epithelium (EP), the SCC is denoted as "EP", and is classified as "Tis". When the SCC reaches the lamina propria mucosa (LPM) below the mucosal epithelium, the SCC is denoted as "LPM", and, in the same manner, when the SCC reaches the muscularis mucosa (MM), the SCC is denoted as "MM", and both are classified as "T1a".

These mucosal epithelium, lamina propria mucosa, and muscularis mucosa correspond to the portion generally referred to as a "mucous membrane". According to a Japanese guideline and a European guideline, it is preferable to apply ER to the esophageal SCC having reached the epithelium (EP)/lamina propria mucosa (LPM), and the muscularis mucosa (MM) by 200 μm or so.

When the SCC has reached the submucosal layer below the lamina propria mucosae, the SCC is denoted as "SM1", "SM2", or "SM3", depending on the depth thereof, and they are all classified as "T1b". The boundaries between the classifications of "SM1", "SM2", and "SM3" are not clear, but can be classified instinctively into three classes as a near surface of a submucosal layer, an intermediary portion of the submucosal layer, and a submucosal deep layer.

The guidelines mentioned above do not suggest anything about the applicability of the ER to the SCC classified as T1b having reached a level deeper than T1a. However, there is a report that when the invasion depth of the SCC is T1a (MM and SM1), the probability of the SCC metastasis is less than 10%, so that the ER is considered as the most appropriate initial treatment for T1a (MM and SM1), particularly when the patient is old or weak, based on the high mortality of the esophagectomy and the substantial morbidity. The esophagectomy is usually applied to the cases of T1b (the intermediary portion of the submucosal layer (SM2) or the deep submucosal layer (SM3)), because their risk of metastasis is over 25%. Therefore, the most important task for preoperative diagnosis of the SCC invasion depth is to distinguish T1a (EP and SM1) and T1b (SM2 or SM3).

[About Data Set]

The CNN system was trained using endoscopic images captured daily in the clinic to which one of the inventors of the present invention belongs. The endoscope systems used included high-resolution or high-definition upper gastrointestinal endoscopes (GIF-XP290N, GIF-Q260J, GIF-RQ260Z, GIF-FQ260Z, GIF-Q240Z, GIF-H290Z, GIF-H290, GIF-HQ290, and GIF-H260Z; manufactured by Olympus Corporation, Tokyo, Japan) and video processors (CV260; manufactured by Olympus Corporation), high-definition magnification gastrointestinal endoscopes (GIF-H290Z, GIF-H290, GIF-HQ290, GIF-H260Z: manufactured by Olympus Corporation) and video processors (EVIS LUCERA CV-260/CLV-260, and EVIS LUCERA ELITE CV-290/CLV-290SL; manufactured by Olympus Medical Systems Corp.), and high-resolution endoscopes (EG-L590ZW, EG-L600ZW, and EG-L600ZW7; manufactured by FUJIFILM Corporation, Tokyo, Japan) and a video endoscope system (LASEREO: manufactured by FUJIFILM Corporation).

The training images were images captured with standard white light imaging, narrow-band imaging (NBI), and blue-laser imaging (BLI), but the images of the patients who met the following exclusion criteria were excluded. The excluded images were those of patients who have severe esophagitis, those of patients with a history of chemotherapy, those of patients having their esophagus exposed to radiation, or those of a lesion located adjacently to an ulcer or the scar of an ulcer; low quality images filled with an excessively small amount of air; those of bleeding, halation, or blurring, those out of focus, and those with mucus.

After the selection, 8,660 non-magnification endoscopic images and 5,678 magnification endoscopic images were collected from pathologically proven superficial esophageal SCC of 804 patients as the training image data set. These images were stored in Joint Photographic Experts Group (JPEG) format, and were pathologically classified into pEP and pLPM, pMM, pSM1, pSM2 and pSM3 cancers, based on the pathological diagnoses of their resected specimens. Under the medical instructor of the Japan Gastroenterological Endoscopy Society, a rectangular frame-like mark was then manually assigned. All of the cancer regions were marked for pEP-pSM1 cancers, and only pSM2 and pSM3 were marked for SM2 and SM3 cancers in a special fashion.

As to a structure enhancement of the endoscope video processor, the narrow-band imaging (NBI) was set to the B-mode level 8, and the level of the blue-laser imaging (BLI) was set to 5 to 6. A black soft hood was attached to the tip of the endoscope so that an appropriate distance was ensured between the tip of the endoscope zoom lens and the surface of the mucous membrane during the magnified observations. The degrees of protrusions and depressions, and the hardness of the cancers were evaluated by performing initial routine examinations with the non-magnification white light imaging, the NBI, or the BLI.

By magnifying the NBI image, changes in the external appearance of the superficial blood vessel structures, particularly those in the capillary loops in the capillary, were then evaluated. Finally, in order to visualize the spread of the cancer, the lesion was stained with iodine.

[Training/Validation Algorithm]

For the CNN system according to the sixth embodiment, a CNN architecture referred to as Single Shot Multibox Detector (SSD) and the Caffe framework, which were substantially the same as those used in the third embodiment, were used without changing the algorithm.

The training was carried out using stochastic gradient descent at a global learning rate of 0.0001. Each of the images was resized to $300 \times 300$ pixels, and the size of the rectangular frame was also changed so that the optimal CNN analysis was to be performed. These values were set through trial and error to ensure that every piece of data has compatibility with SSD.

[Measurement Results and Statistics]

The evaluations based on the trained CNN system according to the sixth embodiment were carried out using independent validation test data of superficial esophageal SCC. Images were collected from patients who received endoscopic submucosal dissection or esophagectomy within a period between January 2017 and April 2018, in the hospital to which one of the inventors of the present invention belongs. After the patients who met the same exclusion criteria as those for the training data set were excluded, 155 patients were selected. Three to six typical images (non-magnification endoscopy and magnification endoscopy) were selected per patient, and diagnoses were made by the CNN system.

The trained CNN system according to the sixth embodiment generated a diagnose of an EP-SM1 or SM2/SM3 cancer having a continuous number between 0 and 1 which corresponds to the probability of the diagnosis. When it was diagnosed that all regions of the lesion are limited to EP-SM1, the lesion was diagnosed as an EP-SM1 cancer. When it was diagnosed that a part of the lesion has entered the SM2 or SM3 level, the lesion was diagnosed as an SM2/3 cancer. The results of the non-magnification endoscopy, the magnification endoscopy, and the final diagnosis (non-magnification endoscopy and magnification endoscopy) were analyzed.

In order to compare the correctness of the trained CNN system according to the sixth embodiment with that of the physicians, 16 certified endoscopy specialists were invited from the Japan Gastroenterological Endoscopy Society as the endoscopy specialists. These endoscopy specialists had 9 to 23 years of expertise as physicians, and had experiences of 3000 to 20000 endoscopic examinations. They also made preoperative diagnosis and performed endoscopic resections of gastrointestinal cancers, on a daily basis. The same validation test data as that provided to the CNN system was provided to the endoscopy specialists, and the specialists made diagnoses of EP-SM1 or SM2/SM3 cancers.

Main output indices were the diagnosis accuracy, the sensitivity, the degree of specificity, the positive prediction value (PPV), the negative prediction value (NPV), and the diagnosis time. These values were then compared between the trained CNN system according to the sixth embodiment and the endoscopy specialists. In order to evaluate variations among the observers in the diagnoses of the invasion depth of the cancers, κ statistic was used. κ value > 0.8 represents almost complete match, and κ value = 0.8 to 0.6 represents substantial match. κ value = 0.6 to 0.4 represents moderate match, and κ value = 0.4 to 0.2 represents low match. κ value < 0.2 represents slight match. κ value = 0 represents accidental match, and κ value < 0 indicates non-match. All of these calculations were performed using statistical software EZR.

This survey was carried out under the approval of the Osaka International Cancer Institute (No. 2017-1710059178) and the Japan Medical Association (ID JMA-IIA00283).

In order to examine the validity of the diagnoses of the trained CNN system according to the sixth embodiment, 405 non-magnification endoscopic images and 509 magnification endoscopic images from 155 patients were selected in total. Table 22 provides a summary of the demographic statistics of the selected patients.

[Table 22]

| Features of patients (n = 155) | |
|---|---|
| Sex (male/female) | 128/27 |
| Mean average (age (range)) | 69 (44-90) |
| Features of lesion (n = 155) | |
| Median tumor size (mm (range)) | 18 (4-95) |
| Position of tumor (Ce/Ut/Mt/Lt/Ae) | 4/25/64/57 /5 |
| Macroscopic height (0-I, 0-IIa)/ squamous(IIb)/depression (0-IIc) | 32/25/98 |
| Tumor depth (EP-LPM/MM/SM1/SM2-) | 117/10/4/2 4 |
| Ce: cervical esophagus, Ut: upper thoracic esophagus, Mt: middle thoracic esophagus, Lt: lower thoracic esophagus EP: epithelium, LPM:lamina propria mucosa, MM: muscularis mucosa, SM: submucosal layer | |

The time required for making the diagnosis for all of the images was 29 seconds. As indicated in Table 23, in the final diagnoses of the pEP-SM1 cancers (non-magnification endoscopy and magnification endoscopy), the sensitivity of 90.1%, the degree of specificity of 95.8%, the positive prediction value of 99.2%, the negative prediction value of 63.9%, and the accuracy of 91.0% were obtained.

[Table 23]

| Diagnoses by CNN system | | | | | |
|---|---|---|---|---|---|
| | Sensitivity | Degree of specificity | PPV | NPV | Correctness |
| Final diagnoses | 90.1% (95%CI 83.6-94.6) | 95.8% (95%CI 78.9-99.9) | 99.2% (95%CI 95.4-100) | 63.9% (95%CI 46.2-79.2) | 91.0% (95%CI 85.3-95.0) |
| Non-ME diagnoses | 95.4% (95%CI 90.3-98.3) | 79.2% (95%CI 57.8-92.9) | 96.2% (95%CI 91.3-98.7) | 76.0% (95%CI 54.9-90.6) | 92.9% (95%CI 87.7-96.4) |
| ME diagnoses | 91.6% (95%CI 85.5-95.7) | 79.2% (95%CI 57.8-92.9) | 96.0% (95%CI 90.9-98.7) | 63.3% (95%CI 43.9-80.1) | 89.7% (95%CI 83.8-94.0) |
| Diagnoses by endoscopy specialists | | | | | |
| | Sensitivity | Degree of specificity | PPV | NPV | Correctness |
| Comprehensive diagnoses | 89.8% (95%CI 86.2-93.4) | 88.3% (95%CI 80.6-95.9) | 97.9% (95%CI 96.5-99.1) | 65.5% (95%CI 58.1-72.8) | 89.6% (95%CI 87.2-91.9) |
| Non-ME diagnosis | 90.6% (95%CI 87.1-94.1) | 87.2% (95%CI 81.4-93.1) | 97.6% (95%CI 96.6-98.6) | 67.2% (95%CI 59.2-75.3) | 90.1% (95%CI 87.7-92.5) |
| ME diagnosis | 91.5% (95%CI 88.4-94.6) | 77.3% (95%CI 68.4-86.3) | 95.8% (95%CI 94.4-97.3) | 66.5% (95%CI 59.3-73.7) | 89.3% (95%CI 87.3-91.2) |
| PPV: positive predictive value, NPV: negative predictive value, ME: magnification endoscopic examinations | | | | | |

In the non-magnification endoscopic diagnoses of the pEP-SM1 cancers, the sensitivity of 95.4%, the degree of specificity of 79.2%, the positive prediction value of 96.2%, the negative prediction value of 76.0%, and the accuracy of 92.9% were obtained. In the magnification endoscopic diagnoses of the pSM1 cancers, the sensitivity of 91.6%, the degree of specificity of 79.2%, the positive prediction value of 96.0%, the negative prediction value 63.3%, and the accuracy of 89.7% were obtained.

In order to examine the performance of the trained CNN system according to the sixth embodiment in distinguishing the M cancers from the SM cancers, the same validity examination test data, that is, 405 non-magnification endoscopic images and 509 magnification endoscopic images from the 155 patients were selected. The time required for making the diagnoses for all of the images was 29 seconds. In the final diagnoses of the pM cancers, the specificity of 89.0% (95% CI, 82.2% to 93.8%), 92.9% (95% CI, 76.5% to 99.1%), the positive prediction value of 98.3% (95% CI, 48.3% to 79.4%), and the accuracy of 89.7% (95% CI, 83.8% to 94.0%) were obtained.

In the non-magnification endoscopic diagnoses of the pM cancers, the sensitivity of 93.7% (95% CI, 88.0% to 97.2%), the degree of specificity of 75.0% (95% CI, 55.1% to 89.3%), the positive prediction value of 94.4% (95% CI, 88.9% to 97.7%), the negative prediction value of 72.4% (95% CI, 52.8% to 87.3%), and the accuracy of 90.3% (95% CI, 84.5% to 94.5%) were obtained. In the diagnoses of the pM cancers with the magnification endoscopy, the sensitivity of 93.7% (95% CI, 88.0% to 97.2%), the degree of specificity 85.7% (95% CI, 67.3% to 96.0%), the positive prediction value of 96.7% (95% CI, 56.6% to 88.5%), and the accuracy of 92.3% (95% CI, 86.9% to 95.9%) were obtained.

The invasion depths of the SCCs in the same validity test data were diagnosed by the 16 endoscopy specialists (Table 23). As a whole, the sensitivity of 89.8%, the degree of specificity of 88.3%, the positive prediction value of 97.9%, the negative prediction value of 65.5%, and the accuracy of 89.6% were obtained. In subgroup analyses by the endoscopy specialists who have a long-term expertise (16 years or more) and by those who have a short-term expertise (less than 16 years), the diagnosis accuracies were 91.0% and 87.7%, respectively. The degree of match between the observers for the diagnoses was 0.303 (Fleiss' $\kappa$ coefficient, Z = 41.1, p value = 0.000). The time required for evaluating the entire validation test data was 115 minutes (within a range between 70 and 180 minutes).

The diagnosis accuracies of the trained CNN system according to the fifth embodiment based on the lesion characteristics are indicated in Tables 24 and 25. The correctness of the trained CNN system according to the sixth embodiment and the endoscopy specialists include the nature of the lesion, e.g., the depth of cancer infiltration, the form, and the size of the lesion.

[Table 24]

| | Diagnoses by CNN system | | Diagnoses by endoscopy specialists | |
|---|---|---|---|---|
| Invasion depth of cancer | EP-SM1 | SM2- | EP-SM1 | SM2- |
| pEP/LPM | 94.00% | 6.00% | 93.40% | 6.60% |
| pMM/SM1 | 57.10% | 42.90% | 60.30% | 39.70% |
| pSM2 | 4.20% | 95.80% | 11.70% | 88.30% |
| p: Pathology, EP: epithelium, LPM: lamina propria mucosa, MM: muscularis mucosa, SM: submucosal layer | | | | |

[Table 25]

| Features of Cancer | Final diagnosis accuracy by CNN system | Diagnosis accuracy by endoscopy specialists |
|---|---|---|
| Protrusion | 81.30% | 77.30% |
| Squamous | 100.00% | 97.50% |
| Depression | 91.80% | 91.60% |
| -10 mm | 83.30% | 89.20% |
| 11-30 mm | 93.30% | 91.10% |
| 31-50 mm | 92.60% | 88.40% |
| 50 mm- | 87.50% | 78.10% |

The non-magnification endoscopic diagnoses by the trained CNN system according to the sixth embodiment exhibited a high performance. A large portion of the non-magnification endoscopic images was white light images. The non-magnification endoscopy using white light imaging is a conventional endoscopic imaging approach that is the most common approach available worldwide. The diagnoses of cancer invasion depths using the conventional non-magnification endoscopy are subjective and based on the protrusion, the depression, and the hardness of the cancer which may be affected by variations among observers.

Such variations in the diagnoses of the cancer invasion depths with such a conventional non-magnification endoscopy derived from its low objectivity, which damaged the reliability and prevented the application of the non-magnification endoscopy as a tool for diagnosing the invasion depth of cancers. However, because the diagnoses by the trained CNN system according to the sixth embodiment can provide clear diagnoses, objective diagnoses can be provided, and the variability issue can be addressed. By contrast, the diagnoses performance of the magnification endoscopy was disadvantageous in the trained CNN system according to the sixth embodiment. This unfavorable performance is due to the small amount of magnification endoscopic images as the training images. By accumulating a larger training data set for the magnification endoscopy, further improvements can be expected.

As described above, the trained CNN system according to the sixth embodiment exhibited a favorable performance for diagnoses of the cancer invasion depths of superficial esophageal SCCs, and the accuracy of the final diagnoses was 91.0%, and was comparable to the accuracy of the endoscopy specialists with a long-term expertise.

[Seventh Embodiment]

Explained now in a seventh embodiment are a diagnostic assistance method, a diagnostic assistance system, a diagnostic assistance program, and a computer-readable recording medium storing therein the diagnostic assistance program for diagnosing a superficial pharyngeal cancer (SPC) with white light imaging (WLI) and narrow band imaging (NBI) using a typical esophagogastroduodenoscopy (EGD).

[About Data Set]

The CNN system was trained using EGD images captured in EGD examinations carried out as screening or preoperative examinations in the clinic to which one of the inventors of the present invention belongs, in their daily clinical practice. The endoscope systems used included high-resolution endoscopes (GIF-XP290N, GIF-H260Z, GIF-H260; Olympus Medical Systems Corp., Tokyo, Japan) and standard endoscope video systems (EVIS LUCERA CV-260/CLV-260, EVIS LUCERA ELITE CV-290/CLV-290SL; Olympus Medical Systems Corp.).

As the training data set, 5,109 training images of a pharyngeal cancer were retrospectively collected. These images included 2,109 images of white light imaging (WLI) and 3,294 of NBI including 247 pharyngeal cancer lesion cases. The cases of these images were histologically proven to be of squamous cell carcinoma (SCC), including 202 superficial pharyngeal cancers and 45 advanced cancers. From the training images, poor quality images resulting from halation, defocus, mucus, saliva, etc. were excluded and magnified images by magnifying endoscopy were also excluded. Further, images of two or more lesions in the same image were also excluded. All cases of a superficial pharyngeal cancer were confirmed to have no other cancer using iodine staining at the time of endoscopic resection (ER) application and follow-up endoscopy after the treatment. All images of pharyngeal cancer lesions were marked manually by a well-experienced endoscopy specialist with more than 6 years of experience and having performed 6,000 examinations in a large-scale cancer center.

[Training/Validation Algorithm]

For the CNN system according to the sixth embodiment, a CNN architecture referred to as Single Shot MultiBox Detector (SSD) and the Caffe framework, which were substantially the same as those used in the third embodiment, were used without changing the algorithm. The Caffe framework is one of the most widely used frameworks originally developed at Berkeley Vision and Learning Center.

The training was carried out with stochastic gradient descent at a global learning rate of 0.0001. Each of the images was resized to $300\times300$ pixels, and the size of the boundary box was also changed such that the optimal CNN analysis was to be performed. These values were set through trial and error to ensure that every piece of data has compatibility with SSD.

[Measurement Results and Statistics]

To evaluate the trained CNN system according to the seventh embodiment, a validation dataset of patients with a pharyngeal cancer and a validation dataset of patients with non-cancer were independently prepared. These images were collected from 35 patients with 40 pharyngeal cancer cases, including 35 cases of a superficial cancer and 5 cases of advanced cancers (928 images of a pharyngeal cancer and 732 images without cancer) and 40 patients without pharyngeal cancer (252 images without cancer). From among the 35 patients with a pharyngeal cancer, 30 patients each had 1 lesion and 5 patients each had 2 lesions simultaneously. All these patients with a pharyngeal cancer were treated by endoscopic submucosal dissection or pharyngectomy from 2015 to 2018 in the clinic to which one of the inventors of the present invention belongs. Further, for validation purposes for screening examination, sequential screening images of pharynx were selected using the WLI and the NBI images in all cases.

This study was approved by the Institutional Review Board of the Cancer Institute Hospital (no. 2016-1171) and the Japan Medical Association Ethical Review Board (ID JMA-II A00283).

[Measurement Results and Statistics]

After constructing the trained CNN system according to the seventh embodiment using the training data set, performance was evaluated using independent validation images. When the CNN system detected a pharyngeal cancer from the input data of the validation images, a disease name of the pharyngeal cancer was assigned and a rectangular frame with dotted lines was displayed in an endoscopic image in such a manner as to surround the lesion of interest, based on a diagnostic confidence score of 60 for the CNN system. Then, several criteria were chosen to evaluate the diagnostic performance of the CNN system for the detection of a pharyngeal cancer.

When the CNN system according to the sixth embodiment was able to recognize even a part of a cancer, it was considered that the CNN system correctly made a diagnosis. Because it is sometimes difficult to identify the whole boundary of a cancer in one image and the detection of a cancer was the main purpose for this embodiment. However, even when there was a cancer in an image determined by the trained CNN system according to the sixth embodiment, it was considered incorrect if wide noncancerous sites occupying more than 80% of the image was contained. In an image with a cancer, when the CNN system recognized noncancerous sites as cancerous, determination as a false-positive recognition was made.

Macroscopic types of the lesions were determined according to the Japanese Classification of General Rules for Clinical Studies on Head and Neck Cancer edited by the Japan Society for Head and Neck Cancer. Further, T factor from TNM classification of malignant tumors according to the Union for International Cancer Control was used for a better understanding of cases used as validation dataset. T factor for the hypopharynx is briefly explained as follows:

T1: a tumor is limited to one subsite of the hypopharynx and/or 2 cm or less in greatest dimension,
T2: a tumor invades more than one subsite of the hypopharynx, or is more than 2 cm but not more than 4cm in greatest dimension, without fixation of the hemilarynx,
T3: a tumor is more than 4 cm in greatest dimension, or with fixation of the hemilarunx or extension to the esophagus, and
T4a/T4b: tumor invades any of the adjacent organ.

T factor for the oropharynx is explained as follows:

T1: a tumor is 2 cm or less,
T2: a tumor is more than 2 cm but not more than 4cm.

All continuous variables are expressed as the median with a range. Statistical analyses were conducted using Fisher's exact test using the GraphPad Prism (GraphPad Software, Inc, La Jolla, Calif). $P < 0.05$ was considered statistically significant.

The median tumor size was 22.5 mm, and 72.5% of the lesions were located at the pyriform sinus, and 17.5% of the lesions were at the posterior wall in the hypopharynx. For the macroscopic types, 47.5% were O-IIa and 40% were 0-IIb. All the lesions were proved to be a SCC in the histopathology (see Table 26).

[Table 26]

| Patient characteristics (n = 35) | |
| --- | --- |
| Sex (male/female) | 32/3 |
| Age, median, (range), years | 67 (45-85) |
| **Lesion characteristics (n = 40)** | |
| Size, median, (range), mm | 22.5 (8-55) |
| Location (hypopharynx pyriform sinus/posterior wall in hypopharynx/ post-cricoid area/posterior wall in oropharynx/lateral wall) | 29 / 7 / 2 / 1 / 1 |
| Macroscopic type | |

(continued)

| Lesion characteristics (n = 40) | | |
|---|---|---|
| (0-I/0-IIa/0-IIb/0-IIc) | Superficial cancer | 1 / 19 / 16 / 0 |
| (1/2/3/4) | Advanced cancer | 2 / 2 / 0 / 0 |
| T factor (T1/T2/T3/T4a/T4b) | | 17 / 19 / 3 / 1 / 0 |
| Histopathology (SCC/others) | | 40 / 0 |

Examples of a validation image are indicated in FIG. 25. Note that FIGS. 25A to 25D indicate an image in which the CNN system according to the seventh embodiment correctly detected a pharyngeal cancer. The CNN system according to the seventh embodiment indicates a region recognized as a pharyngeal cancer using a rectangular frame with broken lines. Note that a rectangular frame with solid lines is indicated as a cancer region by an endoscopy specialist.

FIG. 25A indicates a whitish superficial elevated lesion at the pyriform sinus. The CNN system according to the seventh embodiment recognized a lesion correctly and substantially surrounded the same by a rectangular frame with broken lines, which matches with a rectangular square drown by an endoscopy specialist. Further, the CNN system according to the seventh embodiment was also able to recognize a pharyngeal cancer in a narrow-band imaging (NBI) indicated as a brownish area (Figure 25B) and a lesion generally considered to be difficult to detect, such as a faint reddish unclear lesion due to a white light imaging (WLI) (FIG. 25C) and a lesion in a tangential direction of the narrow-band imaging NBI (FIG. 25 D).

The CNN system according to the seventh embodiment detected correctly all lesions of pharyngeal cancers (40/40) with the comprehensive diagnoses of the WLI and the NBI. Its detection rate was 92.5% (37/40) in the WLI and 100% (38/38) in the NBI. The CNN system according to the seventh embodiment could detect all three pharyngeal cancers less than 10 mm in size. Moreover, the CNN according to the seventh embodiment was able to analyze 1912 images in 28 seconds.

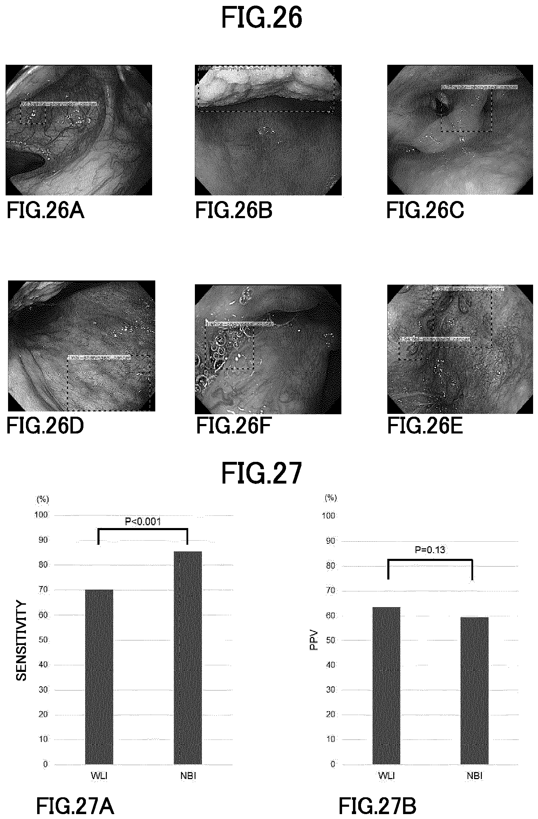

Further, the CNN system according to the seventh embodiment could correctly detect pharyngeal cancers with the sensitivity of 85.6% in the NBI for each image, while it detected with the sensitivity of 70.1% in the WLI. This was significantly lower than the cases of the NBI (see Table 27 and FIG. 27A). The positive prediction value (PPV) in the WLI and the NBI were substantially not different from each other (see Table 27 and FIG. 27B). Further, the degree of specificity, the PPV, and the NPV (negative prediction value) of the CNN system according to the seventh embodiment was 57.1%, 60.7%, and 77.2%, respectively (see Table 24).

[Table 27]

| | Accuracy (95% CI) | Sensitivity (95% CI) | Degree of Specificity (95% CI) | PPV (95% CI) | NPV (95% CI) |
|---|---|---|---|---|---|
| Total images | 67.4% (65.2-69.5) | 79.7% (76.9-82.4) | 57.1% (54.0-60.1) | 60.7% (57.8-63.6) | 77.2% (74.1-80.1) |
| WLI | 61.8% (57.8-65.7) | 70.1% (64.8-75.0) | 52.0% (45.9-58.0) | 63.4% (58.2-68.3) | 59.5% (53.0-65.7) |
| NBI | 69.9% (67.4-72.4) | 85.6% (82.3-88.4) | 58.9% (55.4-62.4) | 59.5% (55.9-62.9) | 85.3% (82.0-88.2) |
| PPV: positive predictive value NPV: negative predictive value 95% CI: 95% confidence interval | | | | | |

The causes of false positives and false negatives in the CNN system according to the seventh embodiment are listed in Tables 28 and 29 in a descending order of frequency. The most frequent causes of false positives were misdiagnoses of normal structures as a cancer, which accounted for 51% (see Table 28). The CNN system according to the seventh embodiment sometimes misdiagnoses normal tongue, arytenoid, epiglottis, and roughness of normal mucosa of the pharynx. Examples of an image in which the CNN system according to the seventh embodiment misdiagnosed as false-positive are indicated in FIG. 26. For example, a case of roughness due to a small cyst (FIG. 26C), an image of a root of tongue (FIG. 26B), and an arytenoid (FIG. 26C) were each misdiagnosed as a cancer. Further, normal mucosa with inflammation misdiagnosed as a cancer accounted for 23% (Table 3) and regions in which normal mucosa had a local reddish area in the WLI, a brownish area in the NBI, or the like, were also misdiagnosed as a cancer (FIG. 26D). Bubbles and blood sometimes remained in the validation images and consequently were sometimes misdiagnosed as a cancer (FIG. 26E), because washing by water in the pharynx is impossible. As for benign lesions, lymphoid follicle was most frequent (FIG. 26F).

[Table 28]

| Causes for false positives | Number of images |
|---|---|
| Normal structure, n (%) | 227 (51) |
| Roughness of mucosa/tongue/arytenoid/ hard palate/vessel/fold/epiglottis/larynx | 100/65/20/18/8/7/7/2 |
| Inflammation, n (%) | 104 (23) |
| Inappropriate conditions, n (%) | 55 (12) |
| bubbles and blood/foggy lens | 53/2 |
| Benign lesion, n (%) | 34 (7.7) |
| lymphoid follicles/dysplasia/melanosis | 32/1/1 |
| Influences of the light, n (%) | 25 (5.7) |
| shadow/halation | 15/10 |
| Others (%) | 2 (0.6) |

[Table 29]

| Causes for false negatives | Number of images |
|---|---|
| Difficult conditions, n (%) | 93 (54) |
| too far/only a part of the lesion/tangential view/ unclear | 44/38/10/1 |
| Obscure lesion in WLI | 53 (31) |
| Lesions in shadow, n (%) | 10 (5.6) |
| Lesions with keratinization, n (%) | 9 (5.2) |
| Foggy lens, n (%) | 2 (0.6) |
| Lesions in background inflammation, n (%) | 1 (0.6) |
| Unknown, n (%) | 5 (3) |

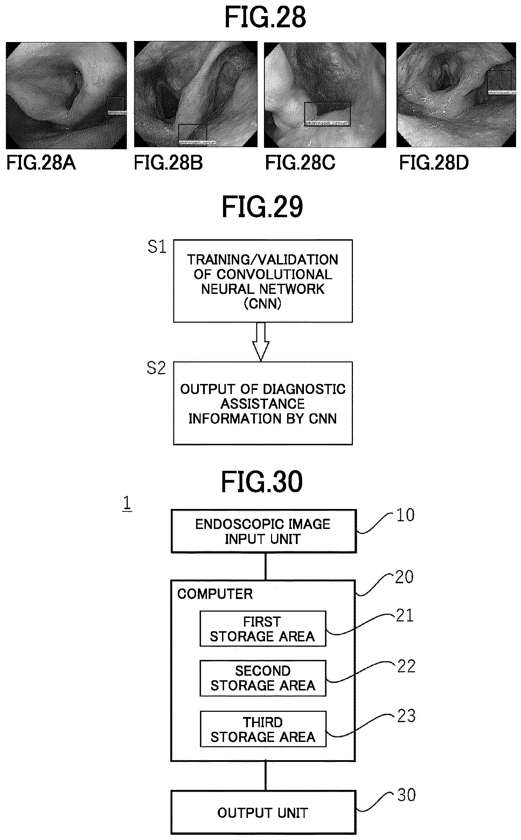

Examples of an image determined as false-positive by the CNN system according to the seventh embodiment were illustrated in FIG. 28. The half of the false negative images were due to difficult conditions (see Table 29) such as the lesions being too distant (FIG. 28A), the presence of only a part of the lesion (FIG. 28B) or the lesions of tangential view (FIG. 28C). The CNN system according to the seventh embodiment also missed some obscure lesions in the WLI (FIG. 28D), which were difficult to diagnose even by endoscopy specialists.

As described above, the CNN system according to the seventh embodiment showed a favorable performance to detect a pharyngeal cancer, which detected all the pharyngeal cancer lesions in each case. The sensitivity of all images was 79.7% and in particular, the sensitivity of NBI images was 85.7%. In other words, in the CNN system according to the seventh embodiment, the NBI was better than the WLI in detecting a pharyngeal cancer. It is consistent with visual detection results through a visual examination by endoscopy specialists, which reported that detection rates were much different in view of conventionally 8% in the WLI and 100% in the NBI (see Non Patent Literature 12). It is because there is only weak contrast between a superficial cancer and a normal mucosa in the WLI. However, the CNN system according to the seventh embodiment detected 69.8% in WLI images. This was much higher than that of endoscopy specialists in the previous report. Therefore, the CNN system according to the seventh embodiment will surely help us to detect pharyngeal cancers even in institutions without the NBI.

[Eighth Embodiment]

A diagnostic assistance method for a disease based on an endoscopic image of a digestive organ with use of a CNN system according to an eighth embodiment will now be explained with reference to FIG. 20. In the eighth embodiment, a diagnostic assistance method for a disease based on an endoscopic image of a digestive organ with use of a CNN system according to any one of the first to the sixth embodiments may be used. At S1, the CNN system is trained/validated with a first endoscopic image of a digestive organ, and with at least one final diagnosis result of the positivity or the negativity for the disease in the digestive organ, a past disease, a severity level, an invasion depth of the disease, and information corresponding to a site where an image is captured, the final diagnosis result being corresponding to the first endoscopic image. When this CNN system is intended for diagnosis of a disease related to *H. pylori* in a gastroscopic image, not only the image data representing *H. pylori positives and H. pylori negatives* but also *H. pylori* eradicated image data are included.

At S2, the CNN trained/validated at S1 outputs at least one of the probability of the positivity and/or the negativity for the disease in the digestive organ, the probability of the past disease, the severity level of the disease, and the probability corresponding to the site where the image is captured, based on a second endoscopic image of the digestive organ. This second endoscopic image represents a newly observed endoscopic image.

At S1, the first endoscopic image may be associated with the site where the first endoscopic image is captured. The site may include at least one of the pharynx, the esophagus, the stomach, the duodenum, the small bowel, and the large bowel, and this site may be sectioned into a plurality of sections in at least one of a plurality of digestive organs.

When the first endoscopic image includes a gastroscopic image, it is also possible to include, at S1, not only the positivity or the negativity for the *H. pylori* infection as the disease, but also the presence/absence of *H. pylori* eradication. At S2, at least one of the probability of the positive *H. pylori* infection, the probability of the negative *H. pylori* infection, and the probability of having the *H. pylori* eradicated may be outputted.

When the first endoscopic image includes a colonoscopic image, the terminal ileum, the cecum, the ascending colon, the transverse colon, the descending colon, the sigmoid colon, the rectum, and the anus may be included as sections at S1. At S2, for the section of the large bowel in the second endoscopic image, for example, a probability corresponding to at least one of the terminal ileum, the cecum, the ascending colon, the transverse colon, the descending colon, the sigmoid colon, the rectum, and the anus may be outputted, or a probability corresponding to at least one of the terminal ileum, the cecum, the ascending colon and transverse colon, the descending colon and sigmoid colon, the rectum, and the anus may be outputted. Furthermore, a probability corresponding to at least one of the terminal ileum, the right colon including the cecum-ascending colon-transverse colon, and the left colon including the descending colon-sigmoid colon-rectum, and the anus may also be outputted.

Furthermore, at S2, the second endoscopic image may be at least one of an image captured by an endoscope, an image transmitted over a communication network, an image provided by a remote control system or a cloud system, an image recorded in a computer-readable recording medium, and a video.

[Ninth Embodiment]

A diagnostic assistance system for a disease based on an endoscopic image of a digestive organ, a diagnostic assistance program using an endoscopic image of a digestive organ, and a computer-readable recording medium according to a ninth embodiment will now be explained with reference to FIG. 21. In the ninth embodiment, the diagnostic assistance system for a disease based on an endoscopic image of a digestive organ explained in the eighth embodiment may be used.

This diagnostic assistance system 1 for a disease based on an endoscopic image of a digestive organ includes an endoscopic image input unit 10, an output unit 30, a computer 20 in which a CNN program is incorporated, and an output unit 30. The computer 20 includes a first storage area 21 that stores therein a first endoscopic image of a digestive organ, a second storage area 22 that stores therein at least one final diagnosis result of the positivity or the negativity for the disease in the digestive organ, a past disease, a severity level, or information corresponding to a site where an image is captured, the final diagnosis result being corresponding to the first endoscopic image, and a third storage area 23 storing therein a CNN program. The CNN program stored in the third storage area 23 is trained/validated based on the first endoscopic image stored in the first storage area 21, and on the final diagnosis result stored in the second storage area 22, and outputs at least one of a probability of the positivity and/or the negativity for the disease in the digestive organ, a probability of the past disease, a severity level of the disease, and a probability corresponding to the site where the image is captured, to the output unit 30, for the second endoscopic image, based on a second endoscopic image of the digestive organ inputted from the endoscopic image input unit 10.

The first endoscopic image stored in the first storage area 21 may be associated with a site where the first endoscopic image is captured. The site may include at least one of the pharynx, the esophagus, the stomach, the duodenum, the small bowel, and the large bowel, and the site may be sectioned into a plurality of sections in at least one of a plurality of digestive organs.

When the first endoscopic image stored in the first storage area 21 includes a gastroscopic image, the final diagnosis result stored in the second storage area 22 may include not only the positivity or the negativity for the *H. pylori* infection, but also the presence/absence of the *H. pylori* eradication. For the second endoscopic image stored in the third storage area, the output unit 30 may output at least one of a probability of the positive *H. pylori* infection, a probability of the negative *H. pylori* infection, and a probability of the eradicated *H. pylori.*

When the first endoscopic image stored in the first storage area 21 includes a colonoscopic image, the sections of the final diagnosis results stored in the second storage area 22 may include the terminal ileum, the cecum, the ascending colon, the transverse colon, the descending colon, the sigmoid colon, the rectum, and the anus. For the sections of the large bowel in the second endoscopic image stored in the third storage area, for example, the output unit 30 may output a probability corresponding to at least one of the terminal ileum, the cecum, the ascending colon, the transverse colon, the descending colon, the sigmoid colon, the rectum, and the anus. The output unit 30 may also output the probability corresponding to at least one of the terminal ileum, the cecum, the ascending colon and transverse colon, the descending colon and sigmoid colon, the rectum, and the anus. Alternatively, the output unit 30 may also output a probability corresponding to at least one of the terminal ileum, the right colon including the cecum-ascending colon-transverse colon, and the left colon including a descending colon-sigmoid colon-rectum, and the anus.

Furthermore, the second endoscopic image stored in the third storage area may be at least one of an image captured by an endoscope, an image transmitted over a communication network, an image provided by a remote control system or a cloud system, an image recorded in a computer-readable recording medium, and a video.

The diagnostic assistance system for a disease based on an endoscopic image of a digestive organ according to the ninth embodiment is provided with a diagnostic assistance program using an endoscopic image of a digestive organ, the diagnostic assistance program being a computer program for causing a computer to operate as the units. Furthermore, the diagnostic assistance program using an endoscopic image of a digestive organ may be stored in a computer-readable recording medium.

Reference Signs List

10    endoscopic image input unit
20    computer
21    first storage area
22    second storage area
23    third storage area
30    output unit

**Claims**

1. A diagnostic assistance system for a disease based on an endoscopic image of a digestive organ with use of a convolutional neural network system, a diagnostic assistance method comprising:

   training the convolutional neural network system using:

      a first endoscopic image of the digestive organ; and
      at least one final diagnosis result of the positivity or the negativity for the disease in the digestive organ, a severity level, or information corresponding to an invasion depth of the disease, the final diagnosis result being corresponding to the first endoscopic image, wherein

      the trained convolutional neural network system outputs at least one of a probability of the positivity or the negativity for the disease in the digestive organ, the severity level, or a probability corresponding to the invasion depth of the disease, based on a second endoscopic image of the digestive organ, wherein
      a site of the digestive organ is the small bowel, the endoscopic image is a wireless capsule endoscopic image, and the trained convolutional neural network program outputs a probability score of a protruding lesion in the wireless capsule endoscopic image inputted from the endoscopic image input unit.

2. The diagnostic assistance system for a disease based on an endoscopic image of a digestive organ with use of a convolutional neural network system according to claim 1, **characterized in that** the trained convolutional neural network program displays a region of the detected protruding lesion in the second endoscopic image and displays the probability score in the second endoscopic image.

3. The diagnostic assistance system for a disease based on an endoscopic image of a digestive organ with use of a convolutional neural network system according to claim 1, **characterized in that** a region of the protruding lesion is displayed in the second endoscopic image, based on a final diagnosis result of the positivity or the negativity for the disease in the small bowel, and
the trained convolutional neural network program determines whether a result diagnosed by the trained convolutional neural network program is correct, based on an overlap between the disease-positive region displayed in the second endoscopic image and the disease-positive region displayed by the trained convolutional neural network program in the second endoscopic image.

4. The diagnostic assistance system for a disease based on an endoscopic image of a digestive organ with use of a convolutional neural network system according to claim 3, **characterized in that**

(1) when the overlap occupies 80% or more of the disease-positive region displayed in the second endoscopic image, as the final diagnosis result of the positivity or the negativity for the disease in the small bowel, or
(2) when a plurality of the disease-positive regions are displayed by the trained convolutional neural network program in the second endoscopic image, and any one of the regions overlaps with the disease-positive region displayed in the second endoscopic image, as the final diagnosis result of the positivity or the negativity for the disease,

the diagnosis made by the trained convolutional neural network program is determined to be correct.

5. The diagnostic assistance system for a disease based on an endoscopic image of a digestive organ with use of a convolutional neural network system according to claim 1, **characterized in that** a diagnosis result of the positivity or the negativity for the disease in the small bowel determines that the protruding lesion is one of a polyp, a nodule, an epithelial tumor, a submucosal tumor, and a venous structure.

6. A diagnostic assistance method for a disease based on an endoscopic image of a digestive organ with use of a convolutional neural network system, comprising:

training the convolutional neural network system using

a first endoscopic image of the digestive organ, and
at least one final diagnosis result of the positivity or the negativity for the disease in the digestive organ, a severity level, or information corresponding to an invasion depth of the disease, the final diagnosis result being corresponding to the first endoscopic image, wherein

the trained convolutional neural network system outputs at least one of a probability of the positivity or the negativity for the disease in the digestive organ, the severity level, or a probability corresponding to the invasion depth of the disease, based on a second endoscopic image of the digestive organ, wherein
the digestive organ is the small bowel, the endoscopic image is a wireless capsule endoscopic image, and the disease is the presence/absence of bleeding.

7. A diagnostic assistance method for a disease based on an endoscopic image of a digestive organ with use of a convolutional neural network system, comprising:

training the convolutional neural network system using:

a first endoscopic image of the digestive organ; and
at least one final diagnosis result of the positivity or the negativity for the disease in the digestive organ, a severity level, or information corresponding to an invasion depth of the disease, the final diagnosis result being corresponding to the first endoscopic image, wherein

the trained convolutional neural network system outputs at least one of a probability of the positivity or the negativity for the disease in the digestive organ, the severity level, or a probability corresponding to the invasion depth of the disease, based on a second endoscopic image of the digestive organ, wherein
the digestive organ is the esophagus, the endoscopic image is a non-magnification endoscopic image or a magnification endoscopic image, and the disease is an invasion depth of a squamous cell carcinoma.

8. The diagnostic assistance method for a disease based on an endoscopic image of a digestive organ with use of a convolutional neural network system according to claim 7, **characterized in that** a diagnosis result of the positivity or the negativity for the disease in the esophagus determines that the invasion depth of the squamous cell carcinoma is one of a mucosal epithelium-lamina propria mucosa, a muscularis mucosa, a section near a surface of a submucosal layer, and a level deeper than an intermediary portion of the submucosal layer.

9. A diagnostic assistance method for a disease based on an endoscopic image of a digestive organ with use of a convolutional neural network system, comprising:

training the convolutional neural network system using:

a first endoscopic image of the digestive organ; and
at least one final diagnosis result of the positivity or the negativity for the disease in the digestive organ, a severity level, or information corresponding to an invasion depth of the disease, the final diagnosis result being corresponding to the first endoscopic image, wherein

the trained convolutional neural network system outputs at least one of a probability of the positivity or the negativity for the disease in the digestive organ, the severity level, or a probability corresponding to the invasion depth of the disease, based on a second endoscopic image of the digestive organ, **characterized in that** a site of the digestive organ is the pharynx, the endoscopic image is an esophagogastroduodenoscopic examination image, and the disease is a pharyngeal cancer.

10. The diagnostic assistance method for a disease based on an endoscopic image of a digestive organ with use of a convolutional neural network system according to claim 9, **characterized in that** the endoscopic image is a white light endoscopic image.

11. The diagnostic assistance method for a disease based on an endoscopic image of a digestive organ with use of a convolutional neural network system according to any one of claims 1 to 10, **characterized in that** the convolutional neural network is further combined with three dimensional information from an X-ray computer tomographic imaging apparatus, an ultrasound computer tomographic imaging apparatus, or a magnetic resonance imaging diagnosis apparatus.

12. The diagnostic assistance method for a disease based on an endoscopic image of a digestive organ with use of a convolutional neural network system according to any one of claims 1 to 10, **characterized in that** the second endoscopic image is at least one of an image captured by an endoscope, an image transmitted via a communication network, an image provided by a remote control system or a cloud system, an image recorded in a computer-readable recording medium, and a video.

13. A diagnostic assistance system for a disease based on an endoscopic image of a digestive organ with use of a convolutional neural network system, comprising: an endoscopic image input unit; an output unit; and a computer, in the computer a convolutional neural network being incorporated, wherein

the computer includes:

a first storage area that stores therein a first endoscopic image of the digestive organ;
a second storage area that stores therein at least one final diagnosis result of the positivity or the negativity for the disease in the digestive organ, a severity level, or information corresponding to an invasion depth of the disease, the final diagnosis result being corresponding to the first endoscopic image; and
a third storage area that stores therein a program of the convolutional neural network program, wherein

the convolutional neural network program

is trained based on the first endoscopic image stored in the first storage unit, and the final diagnosis result stored in the second storage area, and
outputs to the output unit, based on a second endoscopic image of the digestive organ, the second endoscopic image being inputted from the endoscopic image input unit, at least one of a probability of the positivity or the negativity for the disease in the digestive organ, the severity level, or a probability corresponding to the information corresponding to the invasion depth of the disease, for a second endoscopic image, wherein

a site of the digestive organ is the small bowel, the endoscopic image is a wireless capsule endoscopic image, and the trained convolutional neural network program outputs a probability score of a protruding lesion in the wireless capsule endoscopic image inputted from the endoscopic image input unit.

14. The diagnostic assistance system for a disease based on an endoscopic image of a digestive organ with use of a convolutional neural network system according to claim 13, **characterized in that** the trained convolutional neural network program displays a region of the detected protruding lesion in the second endoscopic image and displays the probability score in the second endoscopic image.

15. The diagnostic assistance system for a disease based on an endoscopic image of a digestive organ with use of a convolutional neural network system according to claim 13, **characterized in that** a region of the protruding lesion is displayed in the second endoscopic image, based on the final diagnosis result of the positivity or the negativity for the disease in the small bowel, and
the trained convolutional neural network program determines whether a result diagnosed by the convolutional neural network program is correct, based on an overlap between the disease-positive region displayed in the second endoscopic image and the disease-positive region displayed by the trained convolutional neural network program in the second endoscopic image.

16. The diagnostic assistance system for a disease based on an endoscopic image of a digestive organ with use of a convolutional neural network system according to claim 15, **characterized in that**

(1) when the overlap occupies 80% or more of the disease-positive region displayed in the second endoscopic image, as the final diagnosis result of the positivity or the negativity for the disease in the small bowel, or
(2) when a plurality of the disease-positive regions are displayed by the trained convolutional neural network program in the second endoscopic image, and any one of the regions overlaps with the disease-positive region displayed in the second endoscopic image, as the final diagnosis result of the positivity or the negativity for the disease,

the diagnosis made by the trained convolutional neural network program is determined to be correct.

17. The diagnostic assistance system for a disease based on an endoscopic image of a digestive organ with use of a convolutional neural network system according to claim 13, **characterized in that** the trained convolutional neural network program displays in the second endoscopic image that the protruding lesion is one of a polyp, a nodule, an epithelial tumor, a submucosal tumor, and a venous structure.

18. A diagnostic assistance system for a disease based on an endoscopic image of a digestive organ, comprising an endoscopic image input unit; an output unit; and a computer, in the computer a convolutional neural network being incorporated, wherein

the computer includes:

a first storage area that stores therein a first endoscopic image of the digestive organ;
a second storage area that stores therein at least one final diagnosis result of the positivity or the negativity for the disease in the digestive organ, a severity level, or information corresponding to an invasion depth of the disease, the final diagnosis result being corresponding to the first endoscopic image; and
a third storage area that stores therein the convolutional neural network program, wherein

the convolutional neural network program

is trained based on the first endoscopic image stored in the first storage unit, and the final diagnosis result stored in the second storage area, and
outputs to the output unit, based on a second endoscopic image of the digestive organ, the second endoscopic image being inputted from the endoscopic image input unit, at least one of a probability of the positivity or the negativity for the disease in the digestive organ, the severity level, or a probability corresponding to the information corresponding to the invasion depth of the disease, for a second endoscopic image, wherein

a site of the digestive organ is the small bowel, the endoscopic image is a wireless capsule endoscopic image, and the trained convolutional neural network program displays in the second endoscopic image a probability of the presence/absence of bleeding as the disease.

19. A diagnostic assistance system for a disease based on an endoscopic image of a digestive organ, comprising an endoscopic image input unit; an output unit; and a computer, in the computer a convolutional neural network being incorporated, wherein

the computer includes:

a first storage area that stores therein a first endoscopic image of the digestive organ;
a second storage area that stores therein at least one final diagnosis result of the positivity or the negativity for the disease in the digestive organ, a severity level, or information corresponding to an invasion depth of the disease, the final diagnosis result being corresponding to the first endoscopic image; and
a third storage area that stores therein the convolutional neural network program, wherein

the convolutional neural network program

is trained based on the first endoscopic image stored in the first storage unit, and the final diagnosis result stored in the second storage area, and
outputs to the output unit, based on a second endoscopic image of the digestive organ, the second endoscopic image being inputted from the endoscopic image input unit, at least one of a probability of the positivity or the negativity for the disease in the digestive organ, the severity level, or a probability corresponding to the information corresponding to the invasion depth of the disease, for a second endoscopic image, wherein

a site of the digestive organ is the esophagus, the endoscopic image is a non-magnification endoscopic image or a magnification endoscopic image, and the trained convolutional neural network program displays in the second image an invasion depth of a squamous cell carcinoma as the disease.

20. The diagnostic assistance system for a disease based on an endoscopic image of a digestive organ with use of a convolutional neural network system according to claim 19, **characterized in that** the trained convolutional neural network program displays in the second image that the invasion depth of the squamous cell carcinoma is one of a mucosal epithelium-lamina propria mucosa, a muscularis mucosa, a section near a surface of a submucosal layer, and a level deeper than an intermediary portion of the submucosal layer.

21. A diagnostic assistance system for a disease based on an endoscopic image of a digestive organ with use of a convolutional neural network system, comprising an endoscopic image input unit; an output unit; and a computer, in the computer a convolutional neural network being incorporated, wherein

the computer includes:

a first storage area that stores therein a first endoscopic image of the digestive organ;
a second storage area that stores therein at least one final diagnosis result of the positivity or the negativity for the disease in the digestive organ, a severity level, or information corresponding to an invasion depth of the disease, the final diagnosis result being corresponding to the first endoscopic image; and
a third storage area that stores therein a program of the convolutional neural network program, wherein

the convolutional neural network program

is trained based on the first endoscopic image stored in the first storage unit, and the final diagnosis result stored in the second storage area, and
outputs to the output unit, based on a second endoscopic image of the digestive organ, the second endoscopic image being inputted from the endoscopic image input unit, at least one of a probability of the positivity or the negativity for the disease in the digestive organ, the severity level, or a probability corresponding to the information corresponding to the invasion depth of the disease, for a second endoscopic image, wherein

a site of the digestive organ is the pharynx, the endoscopic image is an esophagogastroduodenoscopic examination image, and the disease is a pharyngeal cancer.

22. The diagnostic assistance system for a disease based on an endoscopic image of a digestive organ with use of a convolutional neural network system according to claim 21, **characterized in that** the endoscopic image is a white light endoscopic image.

23. The diagnostic assistance system for a disease based on an endoscopic image of a digestive organ with use of a convolutional neural network system according to any one of claims 13 to 22, wherein the convolutional neural network program is further combined with three dimensional information from an X-ray computer tomographic imaging apparatus, an ultrasound computer tomographic imaging apparatus, or a magnetic resonance imaging diagnosis apparatus.

24. The diagnostic assistance system for a disease based on an endoscopic image of a digestive organ with use of a convolutional neural network system according to any one of claims 13 to 22, wherein the second endoscopic image is at least one of an image captured by an endoscope, an image transmitted via a communication network, an image provided by a remote control system or a cloud system, an image recorded in a computer-readable recording medium, and a video.

25. A diagnostic assistance program based on an endoscopic image of a digestive organ with use of a convolutional neural network system, **characterized by** being configured to cause a computer to operate as units included in the diagnostic assistance system for a disease based on an endoscopic image of a digestive organ according to any one of claims 13 to 24.

26. A computer-readable recording medium storing therein the diagnostic assistance program based on an endoscopic image of a digestive organ with use of a convolutional neural network system according to claim 25.

# FIG.1

FIG.1A          FIG.1B          FIG.1C

# FIG.2

# FIG.3

# FIG.4

| ENDOSCOPIC EXAMINATION CASES n = 871 |
|---|

| CASES EXCLUDED n = 24 | •H. PYLORI INFECTION STATUS UNCLEAR n = 22 •AFTER GASTRECTOMY n = 2 |
|---|---|

| CASES EVALUATED n = 847 |
|---|

EP 3 884 838 A1

# FIG.5

ASCENDING COLON

TRANSVERSE COLON

DESCENDING COLON

CECUM

SIGMOID COLON

TERMINAL ILEUM

ANUS

RECTUM

54

# FIG.6

COLONOSCOPIC IMAGE

| TRAINING | 9,995 IMAGES/409 PEOPLE |
| | ⇩ |
| | ANATOMICAL ANNOTATIONS APPENDED |
| | ⇩ |
| | CNN |

COLONOSCOPIC IMAGE

VALIDATION

5,121 IMAGES/118 PEOPLE

⇩

ANATOMICAL CLASSIFICATION

- TERMINAL ILEUM    - CECUM
- ASCENDING COLON-
  TRANSVERSE COLON
- DESCENDING COLON-
  SIGMOID COLON
- RECTUM    - ANUS

# FIG.7

PROBABILITY SCORE (PS)

| | |
|---|---|
| TERMINAL ILEUM | 0 |
| CECUM | 0 |
| ASCENDING COLON-TRANSVERSE COLON | |
| DESCENDING COLON-SIGMOID COLON | 5 |
| RECTUM | 0 |
| ANUS | 0 |

OUTPUT
95

# FIG.8

TERMINAL ILEUM

SENSITIVITY
DEGREE OF SPECIFICITY
AUC=0.979

## FIG.8A

CECUM

SENSITIVITY
DEGREE OF SPECIFICITY
AUC=0.940

## FIG.8B

ASCENDING COLON-TRANSVERSE COLON

SENSITIVITY
DEGREE OF SPECIFICITY
AUC=0.850

## FIG.8C

DESCENDING COLON-SIGMOID COLON

SENSITIVITY
DEGREE OF SPECIFICITY
AUC=0.846

## FIG.8D

RECTUM

SENSITIVITY
DEGREE OF SPECIFICITY
AUC=0.835

## FIG.8E

ANUS

SENSITIVITY
DEGREE OF SPECIFICITY
AUC=0.992

## FIG.8F

# FIG.9

CORRECT CLASSIFICATION

ERRONEOUS CLASSIFICATION

PROBABILITY
SCORE (PS)

| TERMINAL ILEUM | 0 |
| CECUM | 0 |
| ASCENDING COLON-TRANSVERSE COLON | 0 |
| DESCENDING COLON-SIGMOID COLON | 0 |
| RECTUM | 0 |
| ANUS | 100 |

FIG.9A

PROBABILITY
SCORE (PS)

| TERMINAL ILEUM | 0 |
| CECUM | 0 |
| ASCENDING COLON-TRANSVERSE COLON | 0 |
| DESCENDING COLON-SIGMOID COLON | 0 |
| RECTUM | 0 |
| ANUS | 99 |

FIG.9B

# FIG.10

CORRECT CLASSIFICATION

ERRONEOUS CLASSIFICATION

PROBABILITY
SCORE (PS)

| | |
|---|---|
| TERMINAL ILEUM | 0 |
| CECUM | 99 |
| ASCENDING COLON-<br>TRANSVERSE COLON | 0 |
| DESCENDING COLON-<br>SIGMOID COLON | 0 |
| RECTUM | 0 |
| ANUS | 0 |

## FIG.10A

PROBABILITY
SCORE (PS)

| | |
|---|---|
| TERMINAL ILEUM | 91.6 |
| CECUM | 1.3 |
| ASCENDING COLON-<br>TRANSVERSE COLON | 1.4 |
| DESCENDING COLON-<br>SIGMOID COLON | 2.3 |
| RECTUM | 1.1 |
| ANUS | 2.3 |

## FIG.10B

# FIG.11

TRAINING

```
┌─────────────────────────────────────┐
│   5,360 IMAGES OF EROSION/ULCER      │
│   (NUMBER OF PATIENTS = 115)         │
└─────────────────────────────────────┘
              │
        ╭───────────────╮
        │   ANNOTATION   │
        ╰───────────────╯
              │
              ▼
┌─────────────────────────────────────┐
│              C N N                   │
└─────────────────────────────────────┘
```

VALIDATION

```
┌─────────────────────────────────────┐
│  440 IMAGES OF EROSION/ULCER AND     │
│       10,000 NORMAL IMAGES           │
│   (NUMBER OF PATIENTS =65)           │
└─────────────────────────────────────┘
              │
      ╭─────────────────────╮
      │ AUTOMATIC DETECTION OF │
      │   EROSION/ULCER BY     │
      │    TRAINED CNN         │
      ╰─────────────────────╯
              │
              ▼
          ┌─────────┐
          │ OUTPUT  │
          └─────────┘
```

PROBABILITY SCORE
0.89

# FIG.12

AUC 0.960 (95% CI, 0.950-0.969)

1 - DEGREE OF SPECIFICITY

# FIG.13

FIG.13A

FIG.13B

FIG.13C

FIG.13D

# FIG.14

FIG.14A

FIG.14B

FIG.14C

FIG.14D

FIG.14E

FIG.14F

FIG.14G

FIG.14H

# FIG. 15

| TRAINING | 30,5841 IMAGES OF EROSION/ULCER (NUMBER OF PATIENTS = 292) |
|---|---|
| | ANNOTATION BY ENDOSCOPY SPECIALISTS |
| | CNN |
| VALIDATION | 7,507 IMAGES OF PROTRUDING LESION IN SMALL BOWEL AND 10,000 NORMAL IMAGES (NUMBER OF PATIENTS = 93) |
| | AUTOMATIC DETECTION OF PROTRUDING LESION BY TRAINED CNN |
| | CLASSIFICATION (POLYPS) AND PROBABILITY SCORE (PS = 0.998) |

# FIG.16

Area under ROC curve = 0.9112

# FIG.17

| POLYP | NODULE | EPITHELIAL TUMOR | SUBMUCOSAL TUMOR | VENOUS STRUCTURE |
|-------|--------|------------------|------------------|------------------|

FIG.17A     FIG.17B     FIG.17C     FIG.17D     FIG.17D

# FIG.18

FIG.18A      FIG.18B      FIG.18C

# FIG.19

FIG.19A

FIG.19B

# FIG.20

TRAINING

6,503 IMAGES CONTAINING BLOOD OF
SMALL BOWEL
21,344 NORMAL IMAGES OF SMALL BOWEL

ANNOTATION OF "BLOOD
CONTAINED" OR "NORMAL"

C N N

VALIDATION

208 IMAGES CONTAINING BLOOD OF
SMALL BOWEL (NUMBER OF PATIENTS = 5)
10,000 NORMAL IMAGES OF SMALL BOWEL
(NUMBER OF PATIENTS = 20)

AUTOMATIC
CLASSIFICATION OF BLOOD
QUANTITY BY TRAINED CNN

OUTPUT

PROBABILITY SCORE (PS)
0.99

## FIG.21

AUC 0.9998 (95% CI, 0.9996-1.0000)

## FIG.22

FIG.22A

FIG.22B

# FIG.23

FIG.22A                    FIG.22B

# FIG.24

MUCOUS MEMBRANE

MUCOSAL EPITHELIUM
LAMINA PROPRIA MUCOSA
MUSCULARIS MUCOSA
SUBMUCOSAL LAYER
PROPER MUSCULAR LAYER
ADVENTITIA

Tis — EP
T1a — LPM, MM
T1b — SM1, SM2, SM3

ESOPHAGUS OUTER SIDE

# FIG.25

FIG.25A        FIG.25B        FIG.25C        FIG.25D

# FIG.26

FIG.26A

FIG.26B

FIG.26C

FIG.26D

FIG.26F

FIG.26E

# FIG.27

FIG.27A

FIG.27B

# FIG.28

FIG.28A    FIG.28B    FIG.28C    FIG.28D

# FIG.29

S1
TRAINING/VALIDATION
OF CONVOLUTIONAL
NEURAL NETWORK
(CNN)

S2
OUTPUT OF DIAGNOSTIC
ASSISTANCE
INFORMATION BY CNN

# FIG.30

1

ENDOSCOPIC IMAGE
INPUT UNIT — 10

COMPUTER — 20

FIRST
STORAGE AREA — 21

SECOND
STORAGE AREA — 22

THIRD
STORAGE AREA — 23

OUTPUT UNIT — 30

**INTERNATIONAL SEARCH REPORT**

| | |
|---|---|
| International application No. | |
| | PCT/JP2019/045580 |

**A. CLASSIFICATION OF SUBJECT MATTER**
A61B 1/00(2006.01)i; A61B 1/045(2006.01)i; A61B 1/267(2006.01)i; A61B 1/273(2006.01)i; G06T7/00(2017.01)i
FI: A61B1/045 614; A61B1/00 C; A61B1/045 622; A61B1/045 620; G06T7/00 350C; G06T7/00 612; A61B1/267; A61B1/273

According to International Patent Classification (IPC) or to both national classification and IPC

**B. FIELDS SEARCHED**

Minimum documentation searched (classification system followed by classification symbols)
A61B1/00-1/32

Documentation searched other than minimum documentation to the extent that such documents are included in the fields searched
| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | SHICHIJO, Satoki et al., "Application of Convolutional Neural Networks in the Diagnosis of Helicobacter pylori Infection Based on Endoscopic Images", EBioMedicine, 2017, vol. 25, pp. 106-111, ISSN 2352-3964, pp. 106-111 | 1-26 |
| Y | 宮崎 祐太，畳み込みニューラルネットワークを用いたカプセル内視鏡画像における小腸病変の位置検出，情報処理学会 研究報告 コンピュータビジョンとイメージメディア(CVIM)[online], 2016, 2016-CVIM-202, vol. 23, pp. 1-8, ISSN 2188-8701, pp. 1-8, non-official translation (MIYAZAKI, Yuta, "Detection of location of small intestine lesion in capsule endoscopy image using convolutional neural network", IPSJ SIG Technical Reports, Computer | 1-6,11-18,23-26 |

☒ Further documents are listed in the continuation of Box C.     ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 06 February 2020 (06.02.2020) | 18 February 2020 (18.02.2020) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2019/045580

C (Continuation). DOCUMENTS CONSIDERED TO BE RELEVANT

| Category* | Citation of document, with indication, where appropriate, of the relevant passages | Relevant to claim No. |
|---|---|---|
| Y | JIA, Xiao et al., "A Deep Convolutional Neural Network for Bleeding Detection in Wireless Capsule Endoscopy Images", 38th Annual International Conference of the IEEE Engineering in Medicine and Biology Society, 2016, pp. 639-642, ISSN 1558-4615, pp. 639-642 | 1-6,11-18,23-26 |
| Y | HIRASAWA, Toshiaki et al., "Application of artificial intelligence using a convolutional neural network for detecting gastric cancer in endoscopic images", Gastric Cancer, 15 January 2018, vol. 21, issue 4, pp. 653-660, ISSN 1436-3291, pp. 653-660 | 1-26 |
| Y | 工藤進英，森悠一，Endocytoscopy は virtual biopsy となりうるか，消化器内視鏡 6 月号，第２７巻第６号，株式会社東京医学社，25 June 2015, pp. 996-998, ISSN 0915-3217, pp. 996-998, non-official translation (KUDO, Shin-ei, MORI, Yuichi, "Can Endocytoscopy be a virtual biopsy?", ENDOSCOPIA DIGESTIVA June issue, vol. 27, no. 6, TOKYO-IGAKUSHA KK) | 1-26 |
| Y | CN 107705852 A (BEIJING HOTWIRE MEDICAL TECH DEV CO., LTD.) 16.02.2018 (2018-02-16) paragraphs [0009]-[0015] | 7-12,19-26 |
| Y | US 2017/0084036 A1 (SIEMENS AKTIENGESELLSCHAFT) 23.03.2017 (2017-03-23) paragraphs [0018], [0021], [0027] | 11,23,25-26 |
| A | 山田真善，上條憲一，斎藤豊，形態情報定量化を基盤とする人工知能システムを活用した大腸がんおよび前がん病変発見のためのリアルタイム内視鏡画像自動解析システムの開発，日本消化器病学会雑誌 第 114 巻臨時増刊号（大会），15 September 2017, A498, ISSN 0446-6586, A489, non-official translation (YAMADA, Masayoshi, KAMIJO, Kenichi, SAITO, Yutaka, "Development of real-time endoscopic image automatic analysis system for finding colorectal cancer and pre-cancerous lesions using an artificial intelligence system based on quantification of shape information", Nippon Shokakibyo Gakkai Zasshi, vol. 114 special extra edition(conference)) | 1-26 |

Form PCT/ISA/210 (continuation of second sheet) (January 2015)

**INTERNATIONAL SEARCH REPORT**
Information on patent family members

International application No.

PCT/JP2019/045580

| Patent Documents referred in the Report | Publication Date | Patent Family | Publication Date |
|---|---|---|---|
| CN 107705852 A | 16 Feb. 2018 | (Family: none) | |
| US 2017/0084036 A1 | 23 Mar. 2017 | WO 2017/053056 A1 paragraphs [0018], [0021], [0027] CN 108140242 A paragraphs [0018], [0021], [0027] | |

Form PCT/ISA/210 (patent family annex) (January 2015)

## REFERENCES CITED IN THE DESCRIPTION

### Patent documents cited in the description

- JP 2002301019 A
- JP 2006095304 A
- JP 2017045341 A
- JP 2017067489 A

### Non-patent literature cited in the description

- **BIBAULT JE ; GIRAUD P ; BURGUN A.** Big Data and machine learning in radiation oncology: State of the art and future prospects. *Cancer Lett.,* 2016, vol. 382 (1), 110-117
- **ESTEVA A ; KUPREL B ; NOVOA RA et al.** Dermatologist-level classification of skin cancer with deep neural networks. *Nature,* 2017, vol. 542 (7639), 115-118
- **GULSHAN V ; PENG L ; CORAM M et al.** Development and Validation of a Deep Learning Algorithm for Detection of Diabetic Retinopathy in Retinal Fundus Photographs. *JAMA,* 2016, vol. 316 (22), 2402-2410
- **BYRNE MF ; CHAPADOS N ; SOUDAN F et al.** Real-time differentiation of adenomatous and hyperplastic diminutive colorectal polyps during analysis of unaltered videos of standard colonoscopy using a deep learning model. *Gut,* 2017
- **CHEN PJ ; LIN MC ; LAI MJ ; LIN JC ; LU HH ; TSENG VS.** Accurate Classification of Diminutive Colorectal Polyps Using Computer-Aided Analysis. *Gastroenterology,* 2018, vol. 154 (3), 568-575
- **MISAWA M ; KUDO SE ; MORI Y et al.** Artificial Intelligence-Assisted Polyp Detection for Colonoscopy: Initial Experience. *Gastroenterology,* 2018
- **TAKIYAMA H ; OZAWA T ; ISHIHARA S et al.** Automatic anatomical classification of esophagogastroduodenoscopy images using deep convolutional neural networks. *Sci Rep.,* 2018, vol. 8 (1), 7497
- **HIRASAWA T ; AOYAMA K ; TANIMOTO T et al.** Application of artificial intelligence using a convolutional neural network for detecting gastric cancer in endoscopic images. *Gastric Cancer,* 2018
- **SHICHIJO S ; NOMURA S ; AOYAMA K et al.** Application of Convolutional Neural Networks in the Diagnosis of Helicobacter pylori Infection Based on Endoscopic Images. *EBioMedicine,* 2017, vol. 25, 106-111
- **IAKOVIDIS DK ; KOULAOUZIDIS A.** Automatic lesion detection in capsule endoscopy based on color saliency: closer to an essential adjunct for reviewing software. *Gastrointestinal endoscopy,* November 2014, vol. 80 (5), 877-83
- **BOAL CARVALHO P ; MAGALHAES J ; DIAS DE CASTRO F et al.** Suspected blood indicator in capsule endoscopy: a valuable tool for gastrointestinal bleeding diagnosis. *Arq Gastroenterol.,* 2017, vol. 54 (1), 16-20
- **MUTO M ; MINASHI K ; YANO T et al.** Early detection of superficial squamous cell carcinoma in the head and neck region and esophagus by narrow band imaging: a multicenter randomized controlled trial. *J Clin Oncol.,* 20 March 2010, vol. 28 (9), 1566-1572
- **KORMAN LY ; DELVAUX M ; HAGENMULLER F ; KEUCHEL M ; FRIEDMAN S ; WEINSTEIN M ; SHETZLINE M ; CAVE D ; DE FRANCHIS R.** Capsule endoscopy structured terminology (CEST): proposal of a standardized and structured terminology for reporting capsule endoscopy procedures. *Endoscopy,* 2005, vol. 37 (10), 951-959
- Japanese Classification of General Rules for Clinical Studies on Head and Neck Cancer